# EUROPEAN PATENT SPECIFICATION

(11) **EP 2 917 365 B1**
(45) Date of publication and mention of the grant of the patent: **11.03.2020**
(21) Application number: 13789320.2
(22) Date of filing: 12.11.2013
(51) Int. Cl.: C12Q 1/68

(54) **METHODS AND KITS FOR THE PROGNOSIS OF COLORECTAL CANCER**
VERFAHREN UND KITS ZUR PROGNOSE VON KOLOREKTALEM KARZINOM
PROCÉDÉS ET KITS POUR LE DIAGNOSTIC DU CANCER COLORECTAL

(30) Priority: 12.11.2012 EP 12192291; 24.12.2012 EP 12382530
(43) Date of publication of application: 16.09.2015
(73) Proprietor: Institució Catalana de Recerca i Estudis Avançats, 08010 Barcelona (ES); Fundació Institut de Recerca Biomèdica (IRB Barcelona), 08028 Barcelona (ES)
(72) Inventor: BATLLE GÓMEZ, Eduard, E-08028 Barcelona (ES); SANCHO SUILS, Elena, E-08028 Barcelona (ES); ROSSELL RIBERA, David, E-08360 Canet de Mar (ES); CALON, Alexandre, F-67205 Oberhausbergen (FR); ESPINET HERNÁNDEZ, Elisa, E-47014 Valladolid (ES); PALOMO PONCE, Sergio, E-08950 Esplugues de Llobregat (ES)
(74) Representative: ABG Intellectual Property Law, S.L.
(86) International application number: PCT/EP2013/073552
(87) International publication number: WO 2014/072517

(56) References cited:
- ESCHRICH S ET AL: "Molecular staging for survival prediction of colorectal cancer patients", JOURNAL OF CLINICAL ONCOLOGY, AMERICAN SOCIETY OF CLINICAL ONCOLOGY, US, vol. 23, no. 15, 20 May 2005 (2005-05-20), pages 3526-3535, XP002567037, ISSN: 0732-183X, DOI: 10.1200/JCO.2005.00.695
- FREDERIKSEN CASPER MOLLER ET AL: "Classification of Dukes' B and C colorectal cancers using expression arrays", JOURNAL OF CANCER RESEARCH AND CLINICAL ONCOLOGY, SPRINGER INTERNATIONAL, BERLIN, DE, vol. 129, no. 5, 15 May 2003 (2003-05-15), pages 263-271, XP009088733, ISSN: 0171-5216
- ZHANG B ET AL: "Targeting transforming growth factor-beta signaling in liver metastasis of colon cancer", CANCER LETTERS, NEW YORK, NY, US, vol. 277, no. 1, 8 May 2009 (2009-05-08), pages 114-120, XP025981419, ISSN: 0304-3835, DOI: 10.1016/J.CANLET.2008.11.035 [retrieved on 2009-01-14]
- KORPAL M ET AL: "Targeting the transforming growth factor-beta signalling pathway in metastatic cancer", EUROPEAN JOURNAL OF CANCER, PERGAMON PRESS, OXFORD, GB, vol. 46, no. 7, 1 May 2010 (2010-05-01), pages 1232-1240, XP027000707, ISSN: 0959-8049 [retrieved on 2010-03-20]
- GULLOTTI LUCIA ET AL: "FHL2 expression in peritumoural fibroblasts correlates with lymphatic metastasis in sporadic but not in HNPCC-associated colon cancer", LABORATORY INVESTIGATION, vol. 91, no. 12, December 2011 (2011-12), pages 1695-1705, XP002692058,

## Description

### FIELD OF THE INVENTION

The invention relates to the field of diagnosis and, more in particular, to methods for predicting the risk of relapse of cancer patients as well as methods for providing personalized medicine to said patients. The invention relates as well to kits for carrying out the diagnostic and predictive medicine methods.

### BACKGROUND OF THE INVENTION

Colorectal cancer (CRC) is one of the most frequent neoplasias in the western world, it is the third cause of death in men, after lung cancer and prostate cancer and it is the second in frequency among women, after breast cancer. Colorectal cancer is the third most common cancer in men (663 000 cases, 10.0% of the total) and the second in women (571 000 cases, 9.4% of the total) worldwide. About 608 000 deaths from colorectal cancer are estimated worldwide, accounting for 8% of all cancer deaths, making it the fourth most common cause of death from cancer. (GLOBOCAN.iarc.fr)

The main treatment option for colorectal cancer is surgery, with or without adjuvant chemotherapy and/or radiotherapy, depending on the individual patient's staging and other medical factors.

The selection of an appropriate treatment is crucial both for the patient and for economical reasons. For patient survival, it is essential to know when to use immediately a heavy and aggressive treatment protocol in order to prevent extension of a malignant colorectal cancer. Otherwise, survival of the patient may be compromised. In contrast, performing a heavy and aggressive treatment when it is not necessitated is highly disadvantageous for the patient. Such treatments subject patients to a degree of discomfort and inconvenience derived from adverse toxicities that may significantly affect the patient's quality of life. Of note, each patient incurs a one in 400 chance that the therapy will result in fatal toxicity. In addition, heavy and aggressive treatments are usually very costly, and thus they should be performed only when necessary.

Currently, treatment selection is based on tumor staging, which is usually performed using the Tumor/Node/Metastasis (TNM) test from the American Joint Committee on Cancer (AJCC). The TNM system assigns a number based on three categories. "T" denotes the degree of invasion of the intestinal wall, "N" the degree of lymphatic node involvement, and "M" the degree of metastasis. The broader stage of a cancer is usually quoted as a number I, II, III, IV derived from the TNM value grouped by prognosis; a higher number indicates a more advanced cancer and likely a worse outcome.

Although the AJCC classification provides some valuable information concerning the stage at which colorectal cancer has been diagnosed, it does not give information on the tumor aggressiveness and its usefulness for prognosis is limited. Whereas it is clear that patients at stage IV have bad prognosis, diagnosis of colorectal cancer at an early stage does not preclude the possibility that the tumor may further develop very rapidly. In particular, it is totally unknown why 20 to 40 percent of patients with stage II colorectal cancer (i.e., early cancer with neither metastasis nor lymph node invasion at diagnosis) will rapidly worsen and die. Some studies suggest that a subset of patients with high-risk stage II colon cancer may benefit from adjuvant therapy (Quasar collaborative group et al., Lancet 2007; 370:2020-2029). Yet, histopathological variables, such as high-risk features in stage II disease, are only directive when stratifying therapy. When lymph nodes are invaded by tumor cells, the TNM test scores as bad prognosis and the patient is usually subjected to surgery followed by heavy chemotherapy. Clinical studies show that for every 25 patients identified as high-risk stage II CRC, 20 will cure regardless of whether they receive treatment or not (Quasar collaborative group et al., Lancet 2007; 370:2020-2029). Likewise, a subset of patients with stage III colon cancer treated only by surgery did not recur in 5 years even without adjuvant treatment (Ranghammar et al., Acta Oncologica 2001; 40: 282-308). Adjuvant chemotherapy is standard recommendation for stage III CRC, yet prospective identification of this subgroup of patients with stage III colon cancer could spare therapy. Thus, an accurate and reliable method that identifies patients at greatest and least risk (eg, "high-risk" stage II and "low-risk" stage III colon cancer) could improve the selection of individualized therapy within these groups.

For this reason, several methods for predicting the outcome of patients suffering colorectal cancer based on the expression levels of molecular markers have been described.

Nevertheless, despite the research carried out on this topic, today there are very few tumor markers which are useful from the clinical point of view both for the diagnosis of CRC and for determining the stage of a CRC carcinoma. A test capable of quantifying likelihood of patient benefit from chemotherapy to identify more accurately Stage III patients for treatment would be extremely useful. A patient having a low recurrence risk resembling that of a Stage II patient and a low likelihood of benefit from chemotherapy might elect to forego chemotherapy. A patient with a high recurrence risk and a low likelihood of benefit from 5-FU based chemotherapy might elect an alternative treatment.

Therefore, there is a need in the art for markers or panels of markers which allow the diagnosis of CRC and the classification of the stage of colorectal carcinomas with a high reliability.

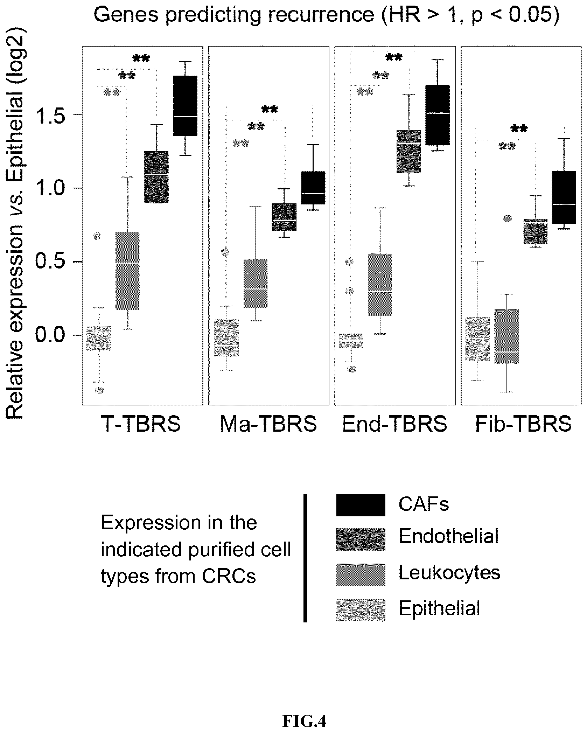

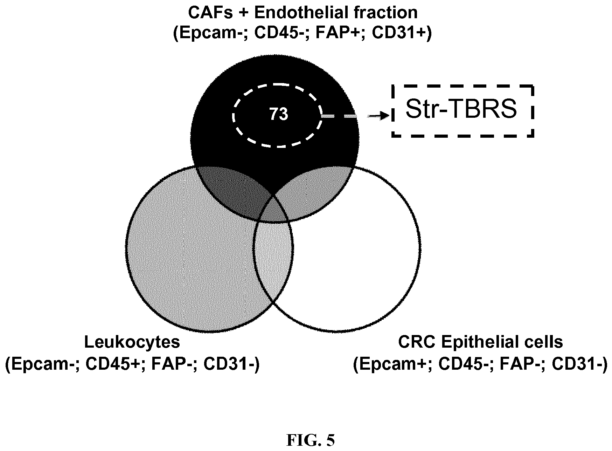

Eschrich, S. et al. (Journal of Clinical Oncology, 2005, vol. 23 (15): 3526-3535) and Frederiksen, C. M. et al. (Journal of Cancer Research, 2003, vol. 129 (5): 263-271) provide microarrays for genome wide expression analysis of genes for the prediction of survival in colorectal cancer patients and for classifying Dukes' B and C colorectal cancers, respectively. In addition, Zhang, B. et al. (Cancer Letters, 2009, vol. 277 (1): 114-120) disclose that a novel dual kinase inhibitor of TGF-beta type I and type II receptors, LY2109761, inhibits TGF-beta mediated activation of Smad and non-Smad pathways in CT26 colon adenocarcinoma cells having K-Ras mutation (see document, figures 4, 5). The inhibitor attenuates the oncogenic effects of TGF-beta on cell migration, invasion and tumorigenicity of CT26 cells. Furthermore, LY2109761 decreases liver metastases and prolongs survival in an experimental metastasis model. On the other hand, Korpal, M. et al. (European Journal of Cancer, 2010, vol. 46 (7): 1232-1240) teach that advanced-stage tumors produce excessive levels of TGF-beta with pro-metastatic effects. Table 1 in this document summarizes strategies to inhibit the TGF-beta pathway as a treatment for metastatic cancers using small molecule inhibitors, antibodies, soluble receptor ligand traps.

Thus, an accurate and reliable method that identifies patients at greatest and least risk (e.g., "high-risk" stage II and "low-risk" stage III colon cancer) could improve the selection of individualized therapy within these groups.

### SUMMARY OF THE INVENTION

In a first aspect, the invention relates to a method for predicting the outcome of a patient suffering colorectal cancer, for selecting a suitable treatment for patient suffering colorectal cancer or for selecting a patient which is likely to benefit from adjuvant therapy after surgical resection of colorectal cancer comprising the determination of the expression levels of the FAM46A, FHL2, FOXC2 and COL4A1 genes in a sample from said patient, wherein an increased expression level of said genes with respect to a reference value for said genes is indicative of an increased likelihood of a negative outcome of the patient, that the patient is candidate for receiving therapy after surgical treatment or that the patient is likely to benefit from therapy after surgical treatment or wherein a decreased expression level of said genes with respect to reference values for said genes is indicative of an increased likelihood of a positive outcome of the patient, that the patient is not candidate for receiving therapy after surgical treatment or that the patient is unlikely to benefit from therapy after surgical treatment.

In another aspect, the invention relates to a kit comprising reagents adequate for determining the expression levels of the FAM46A, FHL2, FOXC2 and COL4A1 genes and, optionally, reagents for the determination of the expression levels of one or more housekeeping genes.

In yet another aspect, the invention relates to the use of a kit according to the invention for predicting the outcome of a patient suffering colorectal cancer or for determining whether a patient suffering colorectal cancer is candidate to chemotherapy or radiotherapy after surgery.

In another aspect, the invention relates to a TGF-beta inhibitor for use in the treatment or prevention of a colorectal cancer metastasis.

### BRIEF DESCRIPTION OF THE FIGURES

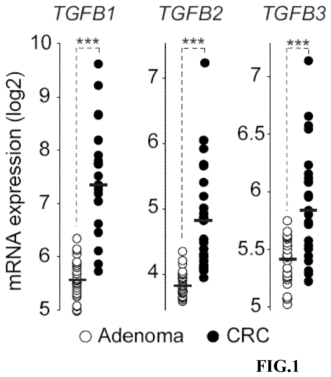

**Figure 1**: shows that there is an increase in TGF-β signaling at the Adenoma-Carcinoma transition during CRC progression. *TGFB1, 2* and *3* mRNA levels are increased in CRC samples (●) compared to adenomas (○). Each dot represents a patient sample in a log2scale (***: p<0.001, Student's test).

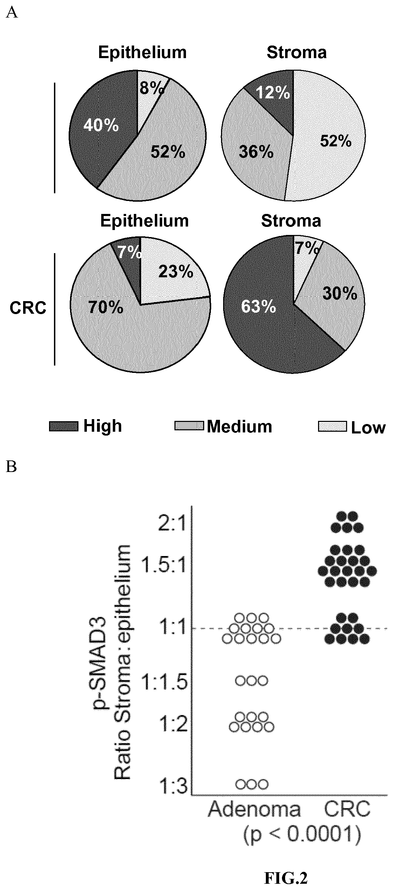

**Figure 2**: shows that TGF-beta signalling acts preferentially over the stromal component of CRC. A. Classification of Adenoma (n = 25) and CRC samples (n = 30) analyzed according to the distribution and intensity of nuclear p-SMAD3 reactivity in epithelial and stromal cells. Whereas the stroma of most adenomas contained few p-SMAD3 highly positive cells and stained weakly overall, a large proportion of CRCs (63%) were characterized by an abundance of stromal cells with strong nuclear p-SMAD3 staining, indicative of active TGF-beta signalling in these cells. B Relative p-SMAD3 positivity in stromal vs epithelial cells from Figure 2. Dots represent samples. P-value calculated using Fisher's exact test.

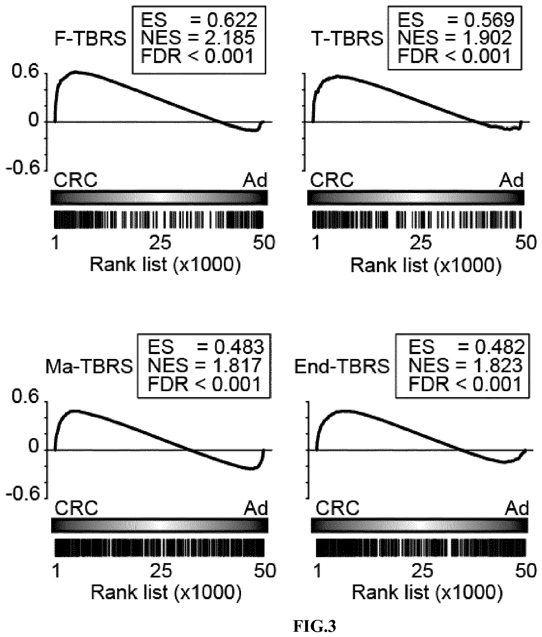

**Figure 3**: Shows gene set enrichment analyses (GSEA) of TGF-beta response signatures (TBRS) of Fibroblasts (F-TBRS), T-Cells (T-TBRS), Macrophages (Ma-TBRS) or Endothelial cells (End-TBRS) in carcinoma versus adenoma samples. ES: enrichment score, NES: normalized enrichment score, FDR: false discovery rate. All stromal TBRSs are highly enriched in CRC samples compared to adenomas.
**Figure 4****:** Genes predicting recurrence amongst the Fibroblast (F-TBRS), T-Cell (T-TBRS), Macrophage (Ma-TBRS) or Endothelial cell (End-TBRS) TGF-beta response signatures are highly expressed in stromal cancer associated fibroblasts, and in endothelial cells purified from CRC patient samples. Whiskers represent minimum and maximum values, truncated at 1.5 times the inter-quartile range. (log2; *: p<0.05, **: p<0.01, Student's t test).
**Figure 5**: TGF-beta induced genes were obtained by microarray analysis of CCD-co-18 normal human colon fibroblasts, adenoma associated fibroblasts (AAFs) and colorectal carcinoma associated fibroblasts (CAFs) treated or not with TGF-beta. Individual TGF-beta response signatures were identified for each population by selecting genes with limma p-value < 0.05 and at least two fold up-regulation in TGF-beta treated fibroblasts. We further refined the classifier by analysing their differential expression in cell populations purified by FACS from CRC patients (GEO datasets GSE39396 and GSE39395). Genes specifically expressed in Cancer associated fibroblasts (FAP+) were defined as those up-regulated with limma p-value < 0.05 (Smyth et al., 2004, Linear Models for Microarray, User's Guide) in the three comparisons FAP+ vs CD31+ (endothelial), FAP+ *vs* CD45+ (Leukocytes) and FAP+ *vs* EPCAM+ (epithelial) cell populations. We defined endothelial (CD31+) specific genes analogously. In addition, we substracted from the classifier any gene appearing in the publication Calon et al., Cancer Cell 2012, Nov 13; 22(5):571-84). This analysis yielded a signature of 73 annotated genes (134 probes; TBRS of this invention) from which we derived specific predictors.

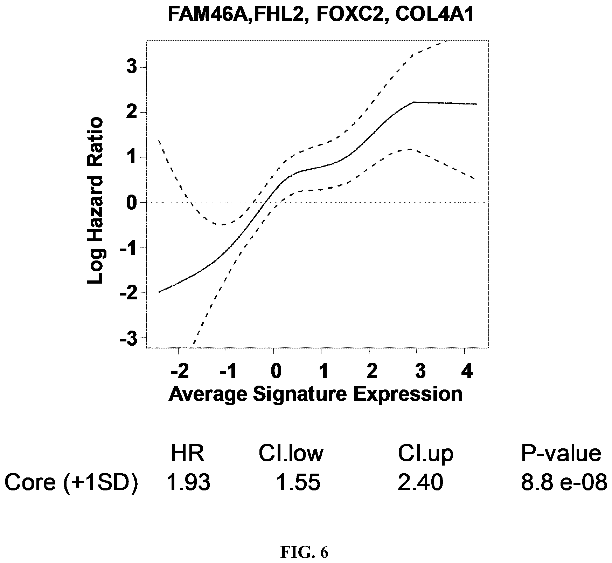

**Figure 6**: shows that the expression of the signature comprised by FAM46A, FHL2, FOXC2, and COL4A1 displays an incremental effect on the risk of recurrence. For every increase in overall expression (+1 Standard Deviation) of the four genes the risk of cancer recurrence augmented by 93% (p<0.0001, HR per +1 Standard Deviation = 1.93).

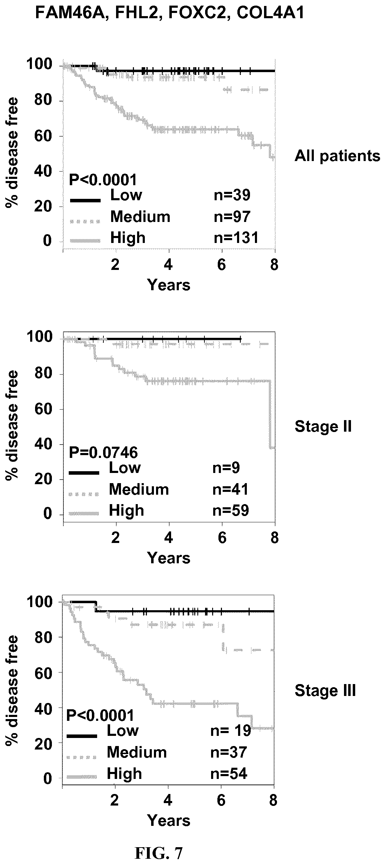

**Figure 7**: Kaplan Meier curves show survival depending on the average expression of the 4 predictors, FAM46A, FHL2, FOXC2, and COL4A1 for all patients, or for stage II or stage III patients.

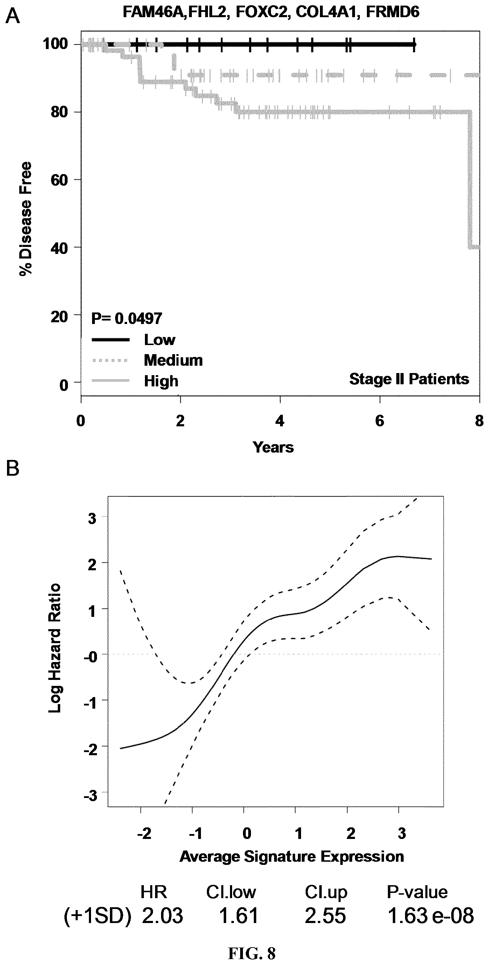

**Figure 8****: A.** Kaplan Meier curves show survival depending on the average expression of the 5 predictors FAM46A, FHL2, FOXC2, COL4A1 and FRMD6 for Stage II patients. **B.** Incremental and approximately linear correlation between expression of the 5 predictors FAM46A, FHL2, FOXC2, COL4A1 and FRMD6 and the risk of recurrence in all patients. For every increase in overall expression (+1 Standard Deviation) of the five genes, the risk of cancer recurrence augmented by 103% (p<0.0001, HR per +1 Standard Deviation = 2.03).

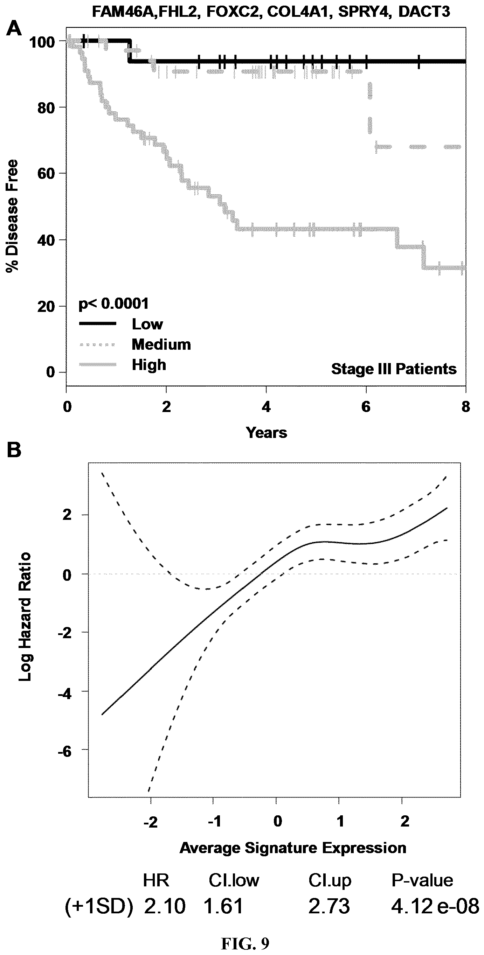

**Figure 9****: A.** Kaplan Meier curves show survival depending on the average expression of the 6 predictors FAM46A, FHL2, FOXC2, COL4A, SPRY4 and DACT3 for Stage III patients. **B.** Incremental and approximately linear correlation between expression of the 6 predictors FAM46A, FHL2, FOXC2, COL4A1 SPRY4 and DACT3 and the risk of recurrence in all patients. For every increase in overall expression (+1 Standard Deviation) of the five genes, the risk of cancer recurrence augmented by 110% (p<0.0001, HR per +1 Standard Deviation = 2.03).

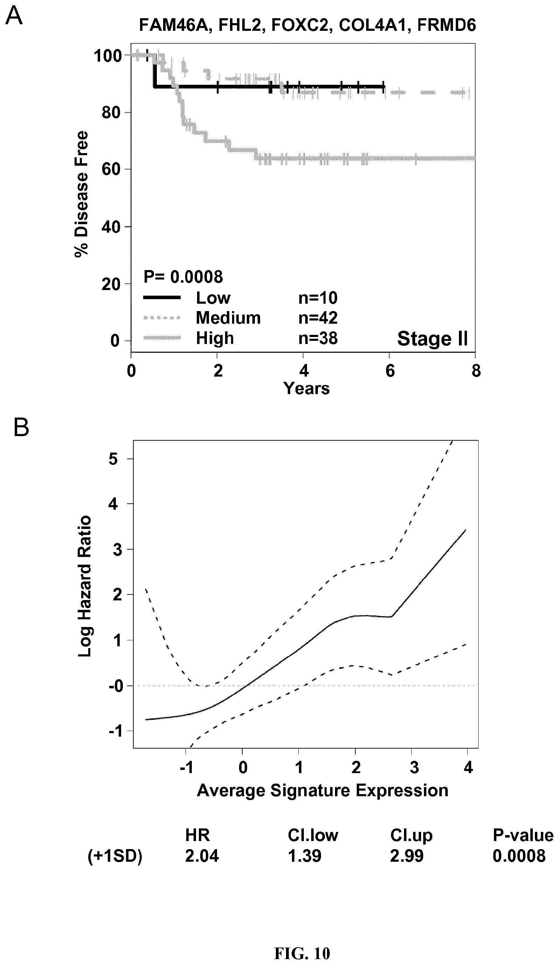

**Figure 10****:** In silico validation. A.- Kaplan Meier curves show probability of remaining disease-free upon therapy depending on the average expression of the 5 predictors FAM46A, FHL2, FOXC2, COL4A1 and FRMD6 for Stage II patients from a completely independent cohort of stage II CRC patients (GSE33113). B. shows that the expression of the 5 predictors FAM46A, FHL2, FOXC2, COL4A1 and FRMD6 displays an incremental effect on the risk of recurrence in this cohort. For every increase in overall expression (+1 Standard Deviation) of the five genes, the risk of cancer recurrence augmented by 104 % (p=0.0008, HR per +1 Standard Deviation = 2.04).

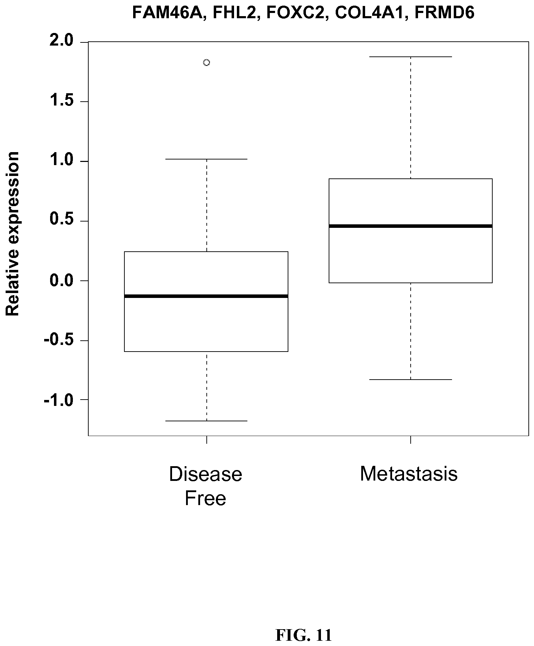

**Figure 11****:** In silico validation in a third independent cohort. A.- Box Plots show higher average signature expression of FAM46A, FHL2, FOXC2, COL4A1 and FRMD6 genes in patients experiencing metastasis after curative treatment in stage II CRC patients from dataset GSE26906. Whiskers represent minimum and maximum values (log2; p<0.05), truncated at 1.5 times the inter-quartile range.

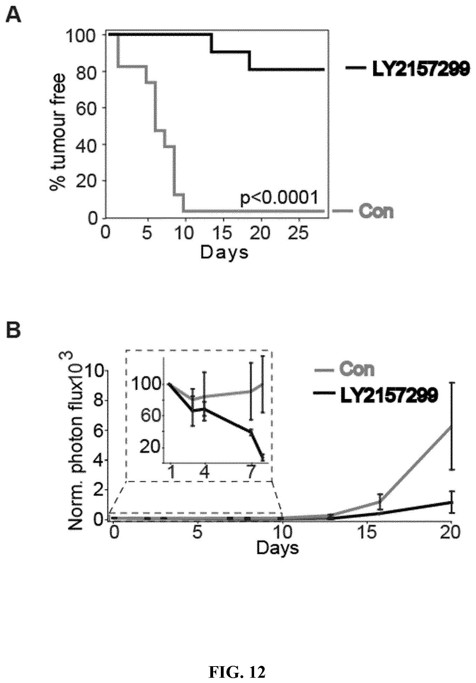

**Figure 12****:** shows that pharmacological inhibition of stromal TGF-beta signalling blocks metastasis initiation. A. Kaplan Meier curves show tumour free survival over time for mice injected subcutaneously with colon cancer stem cells derived from a stage IV patient, treated or not with a TGF beta inhibitor (LY2157299). Mice received LY2157299 or vehicle from three days prior to inoculation until sacrifice (n=12) B. Bioluminescence over time after intrasplenic inoculation of 5 x 10⁵ colon cancer stem cells derived from a stage IV patient in NSG mice treated as in A. Intensities were normalized to day 0 and arbitrarily set to 100. Values are mean ±SEM (*: p<0.05).

### DETAILED DESCRIPTION OF THE INVENTION

### Prognostic methods of the invention

The authors of the present invention have identified a set of genes which provide a reliable method for the identification of CRC patients at greatest and least risk (e.g., "high-risk" stage II and "low-risk" stage III colon cancer) of suffering relapse. For instance, as shown in example 2 of the application, a set of genes which are differentially expressed in response to the administration of TGF-beta in either normal colon fibroblasts (CCDs), adenoma associated fibroblasts (AAFs) and/or carcinoma associated fibroblasts (CAFs) and which are specifically expressed in cancer associated fibroblasts (FAP+) and in tumour endothelial cells was derived in order to find subsets of predictors. By further refining the above signature, the authors of the present invention have selected a small subset of genes from the initial gene signature that allows predicting the risk of recurrence, including both the risk of recurrence of the primary tumors as well as the risk of suffering metastasis. Thus, in a first aspect, the invention relates to a method (hereinafter "prognostic method of the invention") for predicting the outcome of a patient suffering colorectal cancer comprising the determination of the expression levels of the FAM46A, FHL2, FOXC2, and COL4A1 genes in a sample from said patient wherein an increased expression level of said genes with respect to a reference value for said genes is indicative of an increased likelihood of a negative outcome of the patient or wherein a decreased expression level of said genes with respect to a reference values for said genes is indicative of an increased likelihood of a positive outcome of the patient.

The term "predicting the outcome", is used herein to refer to the likelihood that a patient will have a particular clinical outcome, whether positive or negative. The predictive methods of the present invention can be used clinically to make treatment decisions by choosing the most appropriate treatment modalities for any particular patient. The predictive methods of the present invention are valuable tools in predicting if a patient is likely to respond favorably to a treatment regimen, such as chemotherapy. The prediction may include prognostic factors.

As it will be understood by those skilled in the art, the prediction, although preferred to be, need not be correct for 100% of the subjects to be diagnosed or evaluated. The term, however, requires that a statistically significant portion of subjects can be identified as having an increased probability of having a given outcome. Whether a subject is statistically significant can be determined without further ado by the person skilled in the art using various well-known statistic evaluation tools, e.g., determination of confidence intervals, p-value determination, cross-validated classification rates and the like etc. Details are found in Dowdy and Wearden, Statistics for Research, John Wiley & Sons, New York 1983. Preferred confidence intervals are at least 50%, at least 60%, at least 70%, at least 80%, at least 90% or at least 95%. The p-values are, preferably, 0.01, 0,005 or lower.

The term "patient", as used herein, refers to all animals classified as mammals and includes, but is not restricted to, domestic and farm animals, primates and humans, e.g., human beings, non-human primates, cows, horses, pigs, sheep, goats, dogs, cats, or rodents. Preferably, the patient is a male or female human of any age or race.

The term "colorectal cancer" is used in the broadest sense and refers to (1) all stages and all forms of cancer arising from epithelial cells of the large intestine and/or rectum and/or (2) all stages and all forms of cancer affecting the lining of the large intestine and/or rectum. In the staging systems used for classification of colorectal cancer, the colon and rectum are treated as one organ.

In a preferred embodiment, the patient has a stage I, a stage II, a stage III or a stage IV tumor, wherein Stage I is defined as either T1 N0 M0 or T2 N0 M0; Stage II is defined as T3 N0 M0 or T4 N0 M0; Stage III is defined as any T, N1-2; M0 and Stage IV correspond to any T, any N, M1. According to the tumor, node, metastasis (TNM) staging system of the American Joint Committee on Cancer (AJCC) (Greene et al. (eds.), AJCC Cancer Staging Manual. 6th Ed. New York, N.Y.: Springer; 2002), the various stages of colorectal cancer are defined as follows:
- Tumor: T1: tumor invades submucosa; T2: tumor invades muscularis propria; T3: tumor invades through the muscularis propria into the subserose, or into the pericolic or perirectal tissues; T4: tumor directly invades other organs or structures, and/or perforates.
- Node: N0: no regional lymph node metastasis; N1: metastasis in 1 to 3 regional lymph nodes; N2: metastasis in 4 or more regional lymph nodes.
- Metastasis: M0: mp distant metastasis; M1: distant metastasis present.

In a preferred embodiment, the patient whose outcome is to be predicted is a patient which has been diagnosed with colorectal cancer and which has had surgical resection of the cancer. In a preferred embodiment, the patient has had a surgical resection of a stage I tumor, of a stage II tumor, of a stage III tumor or of a stage IV tumor.

In the present invention, the term "sample" or "biological sample" means biological material isolated from a subject. The biological sample can contain any biological material suitable for detecting the desired biomarker and can comprise cell and/or non-cell material of the subject. The sample can be isolated from any suitable tissue or biological fluid such as for example, prostate tissue, blood, blood plasma, serum, urine, cerebrospinal liquid (CSF) or feces. The samples used for the determination of the marker genes are preferably colorectal tissue samples obtained by biopsy.

Alternatively, the samples are biofluid samples. The terms "biological fluid" and "biofluid" are used interchangeably herein and refer to aqueous fluids of biological origin.

The biofluid may be obtained from any location (such as blood, plasma, serum, urine, bile, cerebrospinal fluid, aqueous or vitreous humor, or any bodily secretion), an exudate (such as fluid obtained from an abscess or any other site of infection or inflammation), or fluid obtained from a joint (such as a normal joint or a joint affected by disease such as rheumatoid arthritis).

In a first step, the first method of the invention comprises the determination of the expression levels of the FAM46A, FHL2, FOXC2, and COL4A1 genes in a sample from said patient.

The term "FAM46A", as used herein, refers to family with sequence similarity 46, member A, also known as open reading frame 37 found in chromosome 6 (C6orf37), also known as XTP11, HBV X-transactivated gene 11 protein or FLJ20037. The human FAM46A mRNA is depicted under accession number NM_017633.2 in the GenBank database.

The term "FHL2", as used herein, refers to four and a half LIM domains protein 2, also known as AAG11, DRAL, FHL-2, SLIM-3 or SLIM3. The different transcript variants of the human FHL2 gene are depicted in the GenBank database under accession numbers NM_001450.3, NM_201555.1, NM_001252612.1, NM_201557.3, NM_001039492.2.

The term "FOXC2", as used herein, refers to forkhead box protein C2, also known as forkhead-related protein FKHL14 (FKHL14), transcription factor FKH-14, or mesenchyme forkhead protein 1 (MFH1). The human FOXC2 mRNA is depicted under accession number NM_005251.2 in the GenBank database.

The term "COL4A1", as used herein, refers to collagen type IV alpha-1 chain, also known as arresten, HANAC or POREN1. The human COL4A1 mRNA is depicted under accession number NM_001845.4 in the GenBank database.

In a preferred embodiment, the first method of the invention additionally comprises the determination of the expression levels of the FRMD6 gene, wherein the patient is a patient suffering from stage II colorectal cancer, and wherein increased expression levels of FRMD6 with respect to a reference value for said gene is indicative of an increased likelihood of negative outcome or wherein decreased expression levels of FRMD6 with respect to a reference value for said gene is indicative of an increased likelihood of positive outcome.

The term "FRMD6", as used herein, refers to the FERM domain containing 6, also known as EX1, Willin, C14orf31, MGC17921, c14_5320. The human FRMD6 mRNA is depicted in the GenBank database under accession number AL079307.7.

In another preferred embodiment, the first method of the invention additionally comprises the determination of the expression levels of the SPRY4 and DACT3 genes and wherein the patient is a patient suffering from stage III colorectal cancer, and wherein increased expression levels of SPRY4 and DACT3 with respect to a reference value for said genes is indicative of an increased likelihood of negative outcome or wherein decreased expression levels of SPRY4 and DACT3 with respect to a reference value for said genes is indicative of an increased likelihood of positive outcome.

The term "SPRY4", as used herein, refers to Protein sprouty homolog 4. The different transcript variants of the human SPRY4 gene are depicted in the GenBank database under accession number NM_030964.3 and NM_001127496.1.

The term "DACT3", as used herein, refers to dapper, antagonist of beta-catenin, homolog 3, also known as RRR1 or arginine rich region 1. The human MEX3B mRNA is depicted in the GenBank database under accession number NM_145056.2.

Moreover, in addition to the determination of the markers mentioned above, the method according to the invention may further comprise the determination of one or more markers selected from the group consisting of ACTC1, BOC, CNN1, COMP, CRISPLD2, CRYAB, FAM101B, FILIP1L, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2, and wherein increased expression levels of said genes with respect to a reference value for said genes is indicative of an increased likelihood of negative outcome or wherein decreased expression levels of said genes with respect to a reference value for said genes is indicative of an increased likelihood of positive outcome.

The term "ACTC1", as used herein, refers to the Actin, alpha cardiac muscle 1, also known as ACTC, ASD5, CMD1R, CMH11 or LVNC4. The human ACTC1 mRNA is depicted in the GenBank database under accession number NM_005159.4.

The term "BOC", as used herein, refers to the Brother of CDO protein homolog. The human BOC mRNA is depicted in the GenBank database under accession number NM_033254.2.

The term "CNN1", as used herein, refers to the calponin 1, basic, smooth muscle, also known as Sm-Calp or SMCC. The human CNN1 mRNA is depicted in the GenBank database under accession number NM_001299.4.

The term "COMP", as used herein, refers to Cartilage oligomeric matrix protein, also known as EDM1, EPD1, MED, PSACH or THBS5. The human COMP mRNA is depicted in the GenBank database under accession number NM_000095.2.

The term "CRISPLD2", as used herein, refers to Cysteine-rich secretory protein LCCL domain-containing 2, also known as CRISP11 or LCRISP2. The human CRISPLD2 mRNA is depicted in the GenBank database under accession number NM_031476.3.

The term "CRYAB", as used herein, refers to Alpha-crystallin B chain, also known as KIR or MGC26294. The human CRYAB mRNA is depicted in the GenBank database under accession number NM_001885.1.

The term "FAM101B", as used herein, refers to the family with sequence similarity 101, member B, also known as IRP or INT1L1. The human WNT2 mRNA is depicted in the GenBank database under accession number NC_000007.13 (complement of positions 116916685 to 116963343).

The term "FILIP1L", as used herein, refers to the filamin A interacting protein 1-like gene, also known as DOC-1, DOC1 or GIP90. The different transcript variants of the human FILIP1LM gene are depicted in the GenBank database under accession number NM_182909.2, NM_014890.2 and NM_001042459.1.

The term "GAS7", as used herein, refers to Growth arrest-specific protein 7, also known as MLL/GAS7. The different transcript variants of the human GAS7 gene are depicted in the GenBank database under accession number NM_003644.2, NM_201432.1, NM_201433.1 and NM_001130831.1.

The term "GFPT2", as used herein, refers to glutamine-fructose-6-phosphate transaminase 2, also known as glutamine fructose-6-phosphate aminotransferase 2, hexosephosphate aminotransferase 2, D-fructose-6-phosphate amidotransferase 2 or glutamine:fructose 6 phosphate amidotransferase 2. The human GFPT2 mRNA is depicted in the GenBank database under accession number NM_005110.2.

The term "GLIS3", as used herein, refers to GLIS family zinc finger 3, also known as ZNF515. The different transcript variants of the human GLIS3 gene are depicted in the GenBank database under accession number NM_001042413.1 and NM_152629.3.

The term "HS3ST3A1", as used herein, refers to Heparan sulfate glucosamine 3-O-sulfotransferase 3A1, also known as 30ST3A1, 3OST3A1, heparin-glucosamine 3-O-sulfotransferase, 3-OST-3A, h3-OST-3A; heparan sulfate 3-O-sulfotransferase 3A1 or heparan sulfate D-glucosaminyl 3-O-sulfotransferase 3A1. The human HS3ST3A1 mRNA is depicted in the GenBank database under accession number NM_006042.1.

The term "KIRREL", as used herein, refers to Kin of IRRE-like protein 1, also known as NEPH1, nephrin-like protein 1 or kin of irregular chiasm-like protein 1. The human KIRREL mRNA is depicted in the GenBank database under accession number NM_018240.5.

The term "LRRC15", as used herein, refers to leucine rich repeat containing 15, also known as LIB, isoform b precursor is encoded by transcript variant 2, leucine-rich repeat protein induced by beta amyloid, leucine-rich repeat-containing protein 15, hLib and leucine-rich repeat protein induced by beta-amyloid homolog. The different transcript variants of the human LRRC15 mRNA are depicted in the GenBank database under accession number NM_001135057.2 and NM_130830.4.

The term "LTBP2", as used herein, refers to Latent-transforming growth factor beta-binding protein 2, also known as C14orf141, GLC3D, LTBP3, MSPKA, MSTP031 and WMS3. The human NGF mRNA is depicted in the GenBank database under accession number NM_000428.2.

The term "MFAP2", as used herein, refers to Microfibrillar-associated protein 2, also known as MAGP, MAGP-1 and MAGP1. The different transcript variants of the human MFAP2 gene are depicted in the GenBank database under accession number NM_017459.2, NM_002403.3, NM_001135247.1 and NM_001135248.1.

The term "NNMT", as used herein, refers to Nicotinamide N-methyltransferase. The human NNMT mRNA is depicted in the GenBank database under accession number NM_006169.2.

The term "P4HA3", as used herein, refers to prolyl 4-hydroxylase, alpha polypeptide III, also known as collagen prolyl 4-hydroxylase alpha(III), procollagen-proline, 2-oxoglutarate 4-dioxygenase (proline 4-hydroxylase) alpha polypeptide III, prolyl 4-hydroxylase subunit alpha-3, C-P4H alpha III, 4-PH alpha-3 or procollagen-proline 2-oxoglutarate-4-dioxygenase subunit alpha-3. The human P4HA3 mRNA is depicted in the GenBank database under accession number NM_182904.3.

The term "PPAPDC1A", as used herein, refers to phosphatidic acid phosphatase type 2 domain containing 1A, also known as DPPL2 or PPAPDC1. The human PPAPDC1A mRNA is depicted in the GenBank database under accession number NM_001030059.1.

The term "PPP1R3C", as used herein, refers to Protein phosphatase 1 regulatory subunit 3C, also known as PTG or PPP1R5. The human PPP1R3C mRNA is depicted in the GenBank database under accession number NM_005398.5.

The term "S1PR1", as used herein, refers to sphingosine-1-phosphate receptor 1, also known as CD363, CHEDG1, D1S3362, ECGF1, EDG-1, S1P1 or EDG1. The human S1PR1 mRNA is depicted in the GenBank database under accession number NM_001400.4.

The term "SYNPO2", as used herein, refers to Synaptopodin-2, also known as DKFZp686G051. The different transcript variants of the human SYNPO2 gene are depicted in the GenBank database under accession number NM_133477.2, NM_001128933.1 and NM_001128934.1.

It will be understood that the method according to the present invention may comprise the determination of any naturally occurring polymorphic variant of one or more of the above genes.

In one embodiment, the method of the invention comprises the determination of the expression levels of the genes ACTC1, BOC, CNN1, COL4A1, COMP, CRISPLD2, CRYAB, FAM101B, FAM46A, FHL2, FILIP1L, FOXC2, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2 wherein increased expression levels of said genes with respect to a reference value for said genes is indicative of an increased likelihood of negative outcome or wherein decreased expression levels of said genes with respect to a reference value for said genes is indicative of an increased likelihood of positive outcome. The patient may be a stage II or stage III cancer patient.

In one embodiment, the method of the invention comprises the determination of the expression levels of the genes ACTC1, BOC, CNN1, COL4A1, COMP, CRISPLD2, CRYAB, FAM101B, FAM46A, FHL2, FILIP1L, FOXC2, FRMD6, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2 wherein increased expression levels of said genes with respect to a reference value for said genes is indicative of an increased likelihood of negative outcome or wherein decreased expression levels of said genes with respect to a reference value for said genes is indicative of an increased likelihood of positive outcome. The patient may be a stage II or stage III cancer patient.

In another embodiment, the method of the invention comprises the determination of the expression levels of the genes ACTC1, BOC, CNN1, COL4A1, COMP, CRISPLD2, CRYAB, DACT3, FAM101B, FAM46A, FHL2, FILIP1L, FOXC2, GAS7, GFPT2, GLIS3, HS3ST3A1, KIRREL, LRRC15, LTBP2, MFAP2, NNMT, P4HA3, PPAPDC1A, PPP1R3C, S1PR1 SPRY4 and SYNPO2 and wherein increased expression levels of said genes with respect to a reference value for said genes is indicative of an increased likelihood of negative outcome or wherein decreased expression levels of said genes with respect to a reference value for said genes is indicative of an increased likelihood of positive outcome.

The method according to the present invention may further comprise the determination of the expression levels of one or more additional genes which are up-regulated by TGF beta at least two fold in either AAF, CAFs or normal mucosa fibroblasts and which are specifically expressed in cancer associated fibroblasts (FAP+; EPCAM- CD45- CD31-) or endothelial cells (CD31+; FAP- EpCAM- CD45-) versus epithelial cells (EPCAM+; CD45- FAP- CD31-) and Leukocytes (CD45+; EPCAM-FAP- CD31-). Thus, in another embodiment, the method according to the invention further comprises determination of the expression levels of one or more genes selected from the group consisting of ADAMTS6, BMP6, CCDC71L, CH25H, CNNM2, CPNE2, CREB3L2, DCBLD1, EFR3B, EGR1, ELN, ENDOD1, FHOD3, FOXC1, FZD8, GBP1, GXYLT2, IGF1, IL4R, ITGA11, JPH2, KIAA1211, KIF26B, LIMK1, LINC00340, LPCAT2, LRRC8A, METTL7A, OLFM2, PID1, PPP1R12B, PRSS23, RASD2, RNF152, SCUBE3, SEC14L2, SERPINE2, SGK1, SH3PXD2A, SHISA2, SMTN, SRGAP1, TRPC6 and UCK2 and of the genes which hybridize specifically with the probes having the sequences SEQ ID NO:1 to 26 wherein increased expression levels of said genes with respect to a reference value for said genes is indicative of an increased likelihood of negative outcome or wherein decreased expression levels of said genes with respect to a reference value for said genes is indicative of an increased likelihood of positive outcome.

The gene signature comprising all the genes which are determined according to the different methods of the invention is known as Str-TBRS and are shown in Table 1. In one embodiment, the invention comprises the determination of the expression levels of the genes ACTC1, ADAMTS6, BMP6, BOC, CCDC71L, CH25H, CNN1, CNNM2, COL4A1, COMP, CPNE2, CREB3L2, CRISPLD2, CRYAB, DACT3, DCBLD1, EFR3B, EGR1, ELN, ENDOD1, FAM101B, FAM46A, FHL2, FHOD3, FILIP1L, FOXC1, FOXC2, FRMD6, FZD8, GAS7, GBP1, GFPT2, GLIS3, GXYLT2, HS3ST3A1, IGF1, IL4R, ITGA11, JPH2, KIAA1211, KIF26B, KIRREL, LIMK1, LINC00340, LPCAT2, LRRC15, LRRC8A, LTBP2, METTL7A, MFAP2, NNMT, OLFM2, P4HA3, PID1, PPAPDC1A, PPP1R12B, PPP1R3C, PRSS23, RASD2, RNF152, S1PR1, SCUBE3, SEC14L2, SERPINE2, SGK1, SH3PXD2A, SHISA2, SMTN, SPRY4, SRGAP1, SYNPO2, TRPC6, and UCK2 genes and of the genes which hybridize specifically with the probes having the sequences SEQ ID NO:1 to 26 wherein increased expression levels of said genes with respect to reference values for said genes is indicative of an increased likelihood of a negative outcome of the patient or wherein decreased expression levels of said genes with respect to reference values for said genes is indicative of an increased likelihood of a positive outcome of the patient.

The term "specifically hybridizing", as used herein, refers to conditions which allow the hybridization of two polynucleotide sequences under high stringent conditions or moderately stringent conditions. The expressions "high stringent conditions" and "moderately stringent conditions" are defined below in respect to the kit of the invention and are equally applicable in the context of the present method.

Virtually any conventional method can be used within the frame of the invention to detect and quantify the levels of said marker genes. By way of a non-limiting illustration, the expression levels are determined by means of the quantification of the levels of mRNA encoded by said genes or by means of the quantification of the protein levels.

Methods for determining the quantity of mRNA are well-known in the art. For example the nucleic acid contained in the sample (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e.g., Northern blot analysis or by oligonucleotide microarrays after converting the mRNA into a labeled cDNA) and/or amplification (e.g., RT-PCR). Preferably quantitative or semi-quantitative RT-PCR is preferred. Real-time quantitative or semi-quantitative RT-PCR is particularly advantageous. Preferably, primer pairs were designed in order to overlap an intron, so as to distinguish cDNA amplification from putative genomic contamination. Suitable primers may be easily designed by the skilled person. Other methods of amplification include ligase chain reaction (LCR), transcription-mediated amplification (TMA), strand displacement amplification (SDA) and nucleic acid sequence based amplification (NASBA). Preferably, the quantity of mRNA is measured by quantitative or semi-quantitative RT-PCR or by real-time quantitative or semi-quantitative RT-PCR.

Alternatively, it is also possible to determine the expression levels of the marker genes by means of the determination of the expression levels of the proteins encoded by said genes, since if the expression of genes is increased, an increase of the amount of corresponding protein should occur. The determination of the expression levels of the different proteins can be carried out using any conventional method. By way of a non-limiting example, said determination can be carried out using antibodies with the capacity for binding specifically to the protein to be determined (or to fragments thereof containing the antigenic determinants) and subsequent quantification of the resulting antigen-antibody complexes. The antibodies that are going to be used in this type of assay can be, for example polyclonal sera, hybridoma supernatants or monoclonal antibodies, antibody fragments, Fv, Fab, Fab' and F(ab')2, scFv, diabodies, triabodies, tetrabodies and humanized antibodies. At the same time, the antibodies may or may not be labeled. Illustrative, but non-exclusive, examples of markers that can be used include radioactive isotopes, enzymes, fluorophores, chemoluminescent reagents, enzyme cofactors or substrates, enzyme inhibitors, particles, dyes, etc. There is a wide variety of well-known assays that can be used in the present invention, using non-labeled antibodies (primary antibody) and labeled antibodies (secondary antibodies); these techniques include Western-blot or immunoblot, ELISA (enzyme-linked immunosorbent assay), RIA (radioimmunoassay), competitive EIA (enzyme immunoassay), DAS-ELISA (double antibody sandwich ELISA), immunocytochemical and immunohistochemical techniques, techniques based on the use of biochips or protein microarrays including specific antibodies or assays based on the colloidal precipitation in formats such as reagent strips. Other forms of detecting and quantifying the protein include affinity chromatography techniques, ligand-binding assays, etc.

Once the expression levels of the above genes in a sample from a patient have been determined, the levels are then compared with reference values for each of said gene. Typically, reference values are the expression level of the genes being compared in a reference sample.

A "reference sample", as used herein, means a sample obtained from a pool of healthy subjects which does not have a disease state or particular phenotype. For example, the reference sample may comprise samples from colon mucosa from patients which do not suffer colon cancer or which do not have a history of colon cancer. Alternatively, the reference sample could be a sample or a pool of samples of colon cancer with a low risk of recurrence. This sample or pool of samples can be obtained from patients which have had surgical resection of the tumor and which have not suffered relapse, preferably in the absence of adjuvant chemotherapy. In another embodiment, the reference sample is a sample from a type I CRC or a pool of type I CRCs.

The suitable reference expression levels of genes can be determined by measuring the expression levels of said genes in several suitable subjects, and such reference levels can be adjusted to specific subject populations (for example, a reference level can be linked to the age so that comparisons can be made between expression levels in samples of subjects of a certain age and reference levels for a particular disease state, phenotype, or lack thereof in a certain age group). In a preferred embodiment, the reference sample is obtained from several healthy subjects or from subjects without prior history of colorectal cancer. Alternatively, the reference sample is a sample or a pool of samples of colon cancer from patients which have had surgical resection of the tumor and which have not suffered relapse, preferably in the absence of adjuvant chemotherapy. The person skilled in the art will appreciate that the type of reference sample can vary depending on the specific method to be performed. Thus, in the case that a diagnosis or prognosis of the disease is to be carried out, the references sample may be a pool of non-tumor colorectal tissue samples, either from individuals that do not have a history of colorectal cancer or from a pool of distal non-tumor tissues with respect to the respective tumor tissues, or a sample or a pool of samples of colon cancer from patients which have had surgical resection of the tumor and which have not suffered relapse, preferably in the absence of adjuvant chemotherapy. In the event that the method of the invention is aimed at determining the effect of a therapy in a patient, the reference sample is preferably a sample obtained from said patient before starting the treatment.

The expression profile of the genes in the reference sample can preferably, be generated from a population of two or more individuals. The population, for example, can comprise 3, 4, 5, 10, 15, 20, 30, 40, 50 or more individuals. Furthermore, the expression profile of the genes in the reference sample and in the sample of the individual that is going to be diagnosed according to the methods of the present invention can be generated from the same individual, provided that the profiles to be assayed and the reference profile are generated from biological samples taken at different times and are compared to one another. For example, a sample of an individual can be obtained at the beginning of a study period. A reference biomarker profile from this sample can then be compared with the biomarker profiles generated from subsequent samples of the same individual. In a preferred embodiment, the reference sample is a pool of samples from several individuals and corresponds to portions of colorectal tissue that are far from the tumor area and which have preferably been obtained in the same biopsy but which do not have any anatomopathologic characteristic of tumor tissue.

Once the expression levels of the marker genes in relation to reference values for said genes have been determined, it is necessary to identify if there are alterations in the expression of said genes (increase or decrease of the expression). The expression of a gene is considered increased in a sample of the subject under study when the levels increase with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more. Similarly, the expression of a gene is considered decreased when its levels decrease with respect to the reference sample by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100% (i.e., absent).

Lastly, the patient is then classified as having a high risk of negative outcome if the marker genes show increased expression levels with respect to a reference sample and as having a low risk of negative outcome if the marker genes show decreased expression levels with respect to a reference sample. In a preferred embodiment, a patient is then classified as having a high risk of negative outcome if the expression levels of the gene is higher than the expression level of the same gene in a sample or in a pool of samples of colon cancer from patients which have had surgical resection of the tumor and which have not suffered relapse, preferably in the absence of adjuvant chemotherapy.

The term "positive outcome" in relation to CRC means an improvement in any measure of patient status, including those measures ordinarily used in the art, such as an increase in the duration of Recurrence-Free interval (RFI), an increase in the time of Overall Survival (OS), an increase in the time of Disease-Free Survival (DFS), an increase in the duration of Distant Recurrence-Free Interval (DRFI), and the like. An increase in the likelihood of positive clinical outcome corresponds to a decrease in the likelihood of cancer recurrence.

The term "negative outcome" in relation to CRC means the worsening in any measure of patient status, including those measures ordinarily used in the art, such as a decrease in the duration of Recurrence-Free interval (RFI), a decrease in the time of Overall Survival (OS), a decrease in the time of Disease-Free Survival (DFS), a decrease in the duration of Distant Recurrence-Free Interval (DRFI), and the like. An increase in the likelihood of negative clinical outcome corresponds to an increase in the likelihood of cancer recurrence.

In a preferred embodiment, the outcome in a given patient is measured as the risk of metastasis or as the risk of recurrence.

The term "risk of metastasis", as used herein, refers to a likelihood or probability assessment regarding the chances or the probability that a subject or individual may develop a similar or the same neoplastic disease at an anatomically distant location within a defined time interval, comparable to the one that the subject or individual has been treated for or diagnosed for.

The term "risk of recurrence", as used herein, refers to a likelihood or probability assessment regarding the chances or the probability that a subject or individual may be afflicted with or may be developing a similar or the same neoplastic disease (either at the same anatomical location or an event at an anatomically distant location), within a defined time interval, comparable to the one that the subject or individual has been treated for or diagnosed for.

The method according to the invention further contemplates the possibility of predicting the outcome of a patient combining the expression levels of the different marker genes mentioned above with one or more clinical prognostic factors.

Prognostic factors are those variables related to the natural history of colorectal cancer, which influence the recurrence rates and outcome of patients once they have developed colorectal cancer. Clinical parameters that have been associated with a worse prognosis include, for example, lymph node involvement, and high grade tumors. Prognostic factors are frequently used to categorize patients into subgroups with different baseline relapse risks. In a preferred embodiment, the clinical prognostic factor used in the method of the invention is tumor stage, wherein increased tumor stage is indicative of an increased risk of recurrence or wherein decreased tumor stage is indicative that the patient shows low risk of recurrence.

In a preferred embodiment, the clinical prognostic factor is the tumor stage according to the AJCC classification (See for example AJCC Cancer Staging Manual, Seventh Edition (2010) published by Springer- Verlag New York, herein incorporated by reference) and as defined above. The term "tumor stage", as mentioned above, is a value that is determined on the basis of the TNM value for the tumor. Thus, the stage I corresponds to T1 N0 M0 or T2 N0 M0; Stage II correspond to T3 N0 M0 or T4 N0 M0; Stage III corresponds to any T, N1-2; M0 and Stage IV corresponds to any T, any N and M1.

Thus, in a preferred embodiment, the invention further comprises the determination of the tumor stage in the patient wherein a high tumor stage is indicative of an increased likelihood of a negative outcome of the patient or wherein a low tumor stage is indicative of an increased likelihood of a positive outcome.

The term "low tumor stage", as used herein, refers to an AJCC stage of I or II.

The term "high tumor stage", as used herein, refers to an AJCC stage of III or IV.

Patients analysed according to the present invention may or may not have been treated with one or more therapies aimed at decreasing tumor size. Thus, in a preferred embodiment, the patients have not been treated prior to the determination of the expression levels of the different genes according to the invention. In another embodiment, the patients are treated prior to the determination of the expression levels of the different genes according to the invention with a therapy selected from the group consisting of chemotherapy, radiotherapy or surgery.

The terms "chemotherapy", "radiotherapy" and "surgery" are defined in detailed below and are used with the same meaning in the context of the present invention.

### Method for selecting a suitable treatment for patient suffering colorectal cancer

The prognostic method defined above also allows providing personalized therapies to patients suffering colorectal cancer. In particular, patients which are considered as having a high risk of relapse or of negative outcome will most likely benefit from an additional therapy after surgery. Conversely, patients showing low risk of relapse may forego additional therapeutic treatment following surgery.

Thus, in another aspect, the invention relates to a method (hereinafter "first personalized therapeutic method of the invention") for selecting a suitable treatment for colorectal cancer in a patient comprising the determination of the expression levels of the FAM46A, FHL2, FOXC2 and COL4A1 genes in a sample from said patient, wherein an increased expression level of said genes with respect to a reference value for said genes is indicative that the patient is candidate for receiving therapy after surgical treatment or wherein a decreased expression level of said genes with respect to reference values for said genes is indicative that the patient is not candidate for receiving therapy after surgical treatment.

As used herein, the terms "treatment" or "therapy" can be used indistinctly and refer to clinical intervention in an attempt to prevent, cure, delay, reduce the severity of, or ameliorate one or more symptoms of the disease or disorder or recurring disease or disorder, or in order to prolong the survival of a patient beyond that expected in the absence of such treatment.

The term "colorectal cancer" has been described in detail in the context of the prognostic methods of the invention and is used with the same meaning in the context of the personalized methods according to the invention.

In a first step, the first personalized therapeutic method according to the invention comprises the determination of the expression level of the FAM46A, FHL2, FOXC2 and COL4A1 genes in a sample from said patient.

The terms "colorectal cancer", "patient", "FAM46A gene", "FHL2 gene", "FOXC2 gene", "COL4A1 gene", "expression levels", and "sample" have been described in detail above and are equally applied to the methods according to the present method.

In a preferred embodiment, the first personalized therapeutic method of the invention additionally comprises the determination of the expression levels of the FRMD6 gene, and wherein the patient is a patient suffering from stage II colorectal cancer, wherein increased expression levels said gene with respect to a reference value for said genes is indicative that the patient is candidate for receiving therapy after surgical treatment, wherein decreased expression levels of said genes with respect to a reference value for said gene is indicative that the patient is not a candidate for receiving therapy after surgical treatment.

In another preferred embodiment, the first personalized therapeutic method of the invention additionally comprises the determination of the expression levels of the SPRY4 and DACT3 genes and wherein the patient is a patient suffering from stage III, wherein increased expression levels of SPRY4 and DACT3 with respect to a reference value for said genes is indicative that the patient is candidate for receiving therapy after surgical treatment, wherein decreased expression levels of SPRY4 and DACT3 with respect to a reference value for said genes is indicative that the patient is not a candidate for receiving therapy after surgical treatment.

In yet another preferred embodiment, the first personalized therapeutic method according to the invention further comprises the determination of the expression levels of one or more genes selected from the group consisting of ACTC1, BOC, CNN1, COMP, CRISPLD2, CRYAB, FAM101B, FILIP1L, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2 wherein increased expression levels of one or more of said genes with respect to a reference value for one or more of said genes is indicative that the patient is candidate for receiving therapy after surgical treatment, wherein decreased expression levels of said genes with respect to a reference value for one or more of said genes is indicative that the patient is not a candidate for receiving therapy after surgical treatment.

The terms referring to each of these genes have been described in detail above and are equally applied to the methods according to the present method.

In one embodiment, the method of the invention comprises the determination of the expression levels of the genes ACTC1, BOC, CNN1, COL4A1, COMP, CRISPLD2, CRYAB, FAM101B, FAM46A, FHL2, FILIP1L, FOXC2, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2 wherein increased expression levels of one or more of said genes with respect to a reference value for one or more of said genes is indicative that the patient is candidate for receiving therapy after surgical treatment, wherein decreased expression levels of said genes with respect to a reference value for one or more of said genes is indicative that the patient is not a candidate for receiving therapy after surgical treatment. The patient may be a stage II or stage III patient.

In one embodiment, the method of the invention comprises the determination of the expression levels of the genes ACTC1, BOC, CNN1, COL4A1, COMP, CRISPLD2, CRYAB, FAM101B, FAM46A, FHL2, FILIP1L, FOXC2, FRMD6, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2 wherein increased expression levels of one or more of said genes with respect to a reference value for one or more of said genes is indicative that the patient is candidate for receiving therapy after surgical treatment, wherein decreased expression levels of said genes with respect to a reference value for one or more of said genes is indicative that the patient is not a candidate for receiving therapy after surgical treatment. The patient may be a stage II or stage III patient.

In another embodiment, the method of the invention comprises the determination of the expression levels of the genes ACTC1, BOC, CNN1, COL4A1, COMP, CRISPLD2, CRYAB, DACT3, FAM101B, FAM46A, FHL2, FILIP1L, FOXC2, GAS7, GFPT2, GLIS3, HS3ST3A1, KIRREL, LRRC15, LTBP2, MFAP2, NNMT, P4HA3, PPAPDC1A, PPP1R3C, S1PR1 SPRY4 and SYNPO2 wherein increased expression levels of one or more of said genes with respect to a reference value for one or more of said genes is indicative that the patient is candidate for receiving therapy after surgical treatment, wherein decreased expression levels of said genes with respect to a reference value for one or more of said genes is indicative that the patient is not a candidate for receiving therapy after surgical treatment. The patient may be a stage II or stage III patient.

In yet another embodiment, the first personalized therapeutic method according to the invention further comprises the determination of the expression levels of one or more genes which are up-regulated by TGF beta at least two fold in either AAF, CAFs or normal mucosa fibroblasts and which are specifically expressed in cancer associated fibroblasts (FAP+; EPCAM- CD45- CD31-) or endothelial cells (CD31+; FAP-EpCAM- cd45-) versus epithelial cells (EPCAM+; FAP- CD45- CD31-) and Leukocytes (CD45 +; EPCAM- FAP- CD31-). Thus, in another embodiment, the method according to the invention further comprises the determination of the expression levels of one or more genes selected from the group consisting of ADAMTS6, BMP6, CCDC71L, CH25H, CNNM2, CPNE2, CREB3L2, DCBLD1, EFR3B, EGR1, ELN, ENDOD1, FHOD3, FOXC1, FZD8, GBP1, GXYLT2, IGF1, IL4R, ITGA11, JPH2, KIAA1211, KIF26B, LIMK1, LINC00340, LPCAT2, LRRC8A, METTL7A, OLFM2, PID1, PPP1R12B, PRSS23, RASD2, RNF152, SCUBE3, SEC14L2, SERPINE2, SGK1, SH3PXD2A, SHISA2, SMTN, SRGAP1, TRPC6 and UCK2 and of the genes which hybridize specifically with the probes having the sequences SEQ ID NO:1 to 26 wherein increased expression levels of one or more of said genes with respect to a reference value for one or more of said genes is indicative that the patient is candidate for receiving therapy after surgical treatment, wherein decreased expression levels of said genes with respect to a reference value for one or more of said genes is indicative that the patient is not a candidate for receiving therapy after surgical treatment.

In one embodiment, the first personalized therapeutic method according to the invention comprises the determination of the expression levels of the genes ACTC1, ADAMTS6, BMP6, BOC, CCDC71L, CH25H, CNN1, CNNM2, COL4A1, COMP, CPNE2, CREB3L2, CRISPLD2, CRYAB, DACT3, DCBLD1, EFR3B, EGR1, ELN, ENDOD1, FAM101B, FAM46A, FHL2, FHOD3, FILIP1L, FOXC1, FOXC2, FRMD6, FZD8, GAS7, GBP1, GFPT2, GLIS3, GXYLT2, HS3ST3A1, IGF1, IL4R, ITGA11, JPH2, KIAA1211, KIF26B, KIRREL, LIMK1, LINC00340, LPCAT2, LRRC15, LRRC8A, LTBP2, METTL7A, MFAP2, NNMT, OLFM2, P4HA3, PID1, PPAPDC1A, PPP1R12B, PPP1R3C, PRSS23, RASD2, RNF152, S1PR1, SCUBE3, SEC14L2, SERPINE2, SGK1, SH3PXD2A, SHISA2, SMTN, SPRY4, SRGAP1, SYNPO2, TRPC6, and UCK2 genes and of the genes which hybridize specifically with the probes having the sequences SEQ ID NO:1 to 26 wherein increased expression levels of one or more of said genes with respect to a reference value for one or more of said genes is indicative that the patient is candidate for receiving therapy after surgical treatment, wherein decreased expression levels of said genes with respect to a reference value for one or more of said genes is indicative that the patient is not a candidate for receiving therapy after surgical treatment. The patient may be a stage II or stage III patient.

The term "specifically hybridizing", as used herein, refers to conditions which allow the hybridization of two polynucleotide sequences under high stringent conditions or moderately stringent conditions. The expressions "high stringent conditions" and "moderately stringent conditions" are defined below in respect to the kit of the invention and are equally applicable in the context of the present method.

The expression levels of the different genes used in the first personalized therapeutic method of the invention can be determined by determining the levels of the mRNA encoded by said genes or by determining the levels of the polypeptide encoded by said genes.

In a second step, the personalized therapeutic method according to the invention comprises the identification of those patients showing increased expression levels of said genes with respect to a reference value for said genes as candidates for receiving therapy after surgical treatment or of those patients showing decreased expression levels of the gene with respect to a reference value as a patient which is not a candidate for receiving therapy after surgery.

In a particular embodiment, the therapy after surgical treatment for which the patient is or is not candidate is a therapy selected from the group consisting of chemotherapy, radiotherapy and/or a therapy comprising a TGF-beta inhibitor.

The term "surgery" or "surgical treatment", as used herein, means any therapeutic procedure that involves methodical action of the hand or of the hand with an instrument, on the body of a human or other mammal, to produce a curative or remedial.

As used herein the term "chemotherapy", "chemotherapeutic drug" refers broadly to the use of a chemical drug or a combination thereof for the treatment of cancer, tumors or malign neoplasia, including both cytotoxic and cytostatic drugs. Examples of chemotherapy agents which may be in accordance to the present invention include:
- alkylating agents (for example mechlorethamine, chlorambucil, cyclophosphamide, ifosfamide, streptozocin, carmustine, lomustine, melphalan, busulfan, dacarbazine, temozolomide, thiotepa or altretamine);
- platinum drugs (for example cisplatin, carboplatin or oxaliplatin);
- antimetabolite drugs (for example 5-fluorouracil, capecitabine, 6-mercaptopurine, methotrexate, gemcitabine, cytarabine, fludarabine or pemetrexed);
- anti-tumor antibiotics (for example daunorubicin, doxorubicin, epirubicin, idarubicin, actinomycin-D, bleomycin, mitomycin-C or mitoxantrone);
- mitotic inhibitors (for example paclitaxel, docetaxel, ixabepilone, vinblastine, vincristine, vinorelbine, vindesine or estramustine); and
- topoisomerase inhibitors (for example etoposide, teniposide, topotecan, irinotecan, diflomotecan or elomotecan).

The term "radiotherapy" is a term commonly used in the art to refer to multiple types of radiation therapy including internal and external radiation therapies or radio immunotherapy, and the use of various types of radiations including X-rays, gamma rays, alpha particles, beta particles, photons, electrons, neutrons, radioisotopes, and other forms of ionizing radiations.

The expression "therapy comprising a TGF-beta inhibitor" is a therapy that comprises the use of one or more TGF-beta inhibitors. In the present invention "a TGFβ inhibitor" is understood as any compound capable of preventing signal transmission caused by the interaction between TGFβ and its receptor. TGFβ1 inhibitors that can be used according to the present invention include compounds preventing the competitive or allosteric binding of TGFβ to its receptor, compounds binding to TGFβ and compounds inhibiting the intracellular signalling of TGFβ. Proper assays to determine the inhibitory capacity of a TGFβ inhibitor include the *in vitro* inhibition of TGFβ biological activity by using the inhibitor in Mv-1-Lu cell proliferation assays as well as the *in vivo* inhibition of TGFβ biological activity by the inhibitor using a model of acute liver damage induced by CCl4 (disclosed in WO200519244). For more details about TGF-beta antagonists see also Wojtowicz-Praga (2003).

Suitable TGFβ inhibitors for use in the present invention include, without limitation, any of the inhibitors mentioned in Table 1.

| | | |
|---|---|---|
| 1 | LY2157299 (inhibitor of TGF-beta receptor type I; Lilly Research) (2-(6-methyl-pyridin-2-yl)-3-[6-amido-quinolin-4-yl)-5,6-dihydro-4H-pyrrolo[l,2-b]pyrazole) having the structure | |
| | 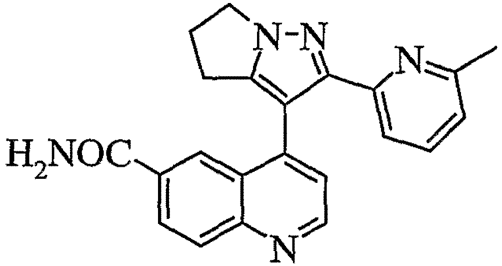 | |
| | in a crystalline form or any polymorph, solvate or hydrate thereof | |
| 2 | Ki 26894, as described by Ehata et al., Cancer Sci. 2007;98:127-33 or any polymorph, solvate or hydrate thereof | |
| 3 | IN-1130 (3-((5-(6-methylpyridin-2-yl)-4-(quinoxalin-6-yl)-1H-imidazol-2-yl)methyl)benzamide) as described by Lee et al., J. Urol. 2008; 180(6): 2660-7, or any polymorph, solvate or hydrate thereof | |
| 4 | Tranilast (N-[3,4-dimethoxycinnamoyl]- anthranilic acid) (Wilkenson, K. A. 2000) or any polymorph, solvate or hydrate thereof | |
| 5 | SB-431542 (inhibitor of TGF-beta receptor II) as described by Halder et al, Neoplasia 2005; 7(5): 509-21 or any polymorph, solvate or hydrate thereof | |
| 6 | 4-(5-Benzol[l,3]dioxol-5-yl-4-pyrldin-2-yl-1H-imidazol-2-yl)-benzamide hydrate or any polymorph, solvate or hydrate thereof, | |
| 7 | 4-[4-(3,4-Methylenedioxyphenyl)-5-(2-pyridyl)-1H-imidazol-2-yl]-benzamide hydrate or any polymorph, solvate or hydrate thereof, | |
| 8 | 4-[4-(1,3-Benzodioxol-5-yl)-5-(2-pyridinyl)-1H-imidazol-2-yl]- benzamide hydrate) or any polymorph, solvate or hydrate thereof; | |
| 9 | NPC-30345 (inhibitor of TGF-beta receptor I) or any polymorph, solvate or hydrate thereof | |
| 10 | LY364947 (4-(3-pyridin-2-yl-1H-Pyrazol-4-yl)quinoline) (inhibitor of TGF-beta receptor I) or any polymorph, solvate or hydrate thereof | |
| 11 | SD-093 (inhibitor of TGF-beta receptor type I; Scios Inc) or any polymorph, solvate or hydrate thereof | |
| 12 | SD-208 (2-(5-Chloro-2-fluorophenyl)pteridin-4-yl]pyridin-4-yl-amine) (inhibitor of TGF-beta receptor type I), or any polymorph, solvate or hydrate thereof | |
| 13 | A-83-01 (3-(6-Methylpyridin-2-yl)-l-phenylthiocarbamoyl-4-quinolin-4-yl pyrazole) (inhibitor of TGF-beta receptor type I) or any polymorph, solvate or hydrate thereof; | |
| 14 | The compound LY2109761 having the structure | |
| | 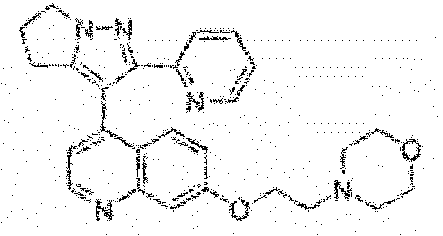 | |
| | or any polymorph, solvate or hydrate thereof | |
| 15 | LY550410 (inhibitor of TGF-beta receptor type I; Lilly Research) as described by Sawyer et al., Bioorg. Med. Chem. Lett. 2004; 14(13): 3581-4 or any polymorph, solvate or hydrate thereof | |
| 16 | LY580276 (inhibitor of TGF-beta receptor type I; Lilly Research) as described by Sawyer et al., Bioorg. Med. Chem. Lett. 2004; 14(13): 3581-4; or any polymorph, solvate or hydrate thereof | |
| 17 | LY566578 (inhibitor of TGF- beta receptor type I; Lilly Research) or any polymorph, solvate or hydrate thereof | |
| 18 | SB-505124 (2-(5- benzo[l,3]dioxol-5-yl-2-tert-butyl-3H-imidazol-4-yl)-6-methylpyridine hydrochloride) (selective inhibitor of TGF-beta receptor type I) or any polymorph, solvate or hydrate thereof | |
| 19 | SB-525334 (6-(2-tert-butyl-5-(6-methylpyridin-2-yl)-1H-imidazol-4-yl)quinoxaline) and polymorphs, solvates, and hydrates thereof | |
| 20 | LDN 193189 (4-(6-(4-(piperazin-1-yl)phenyl)pyrazolo[1,5-a]pyrimidin-3-yl)quinoline) and polymorphs, solvates, and hydrates thereof. | |
| 21 | Disitertide (P144) as described by Santiago et al. (J. Invest. Dermatol. 125, 450-455 (2005) | |
| 22 | SM16 having the structure | |
| | 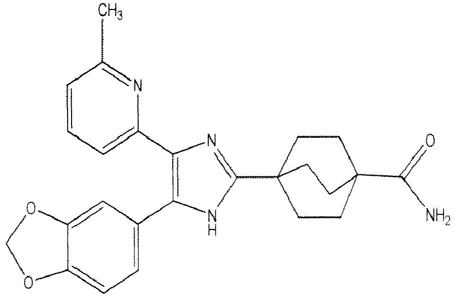 | |
| | or any polymorph, solvate or hydrate thereof | |
| 23 | GW788388 (4-(4-(3-(pyridin-2-yl)-1H-pyrazol-4-yl)pyridin-2-yl)-N-(tetrahydro-2H-pyran-4-yl)benzamide) or any polymorph, solvate, and hydrates thereof | |
| 24 | GB1201 as described by Yao, E. H. et al. Cardiovasc. Res. 81, 797-804 (2009) and polymorphs, solvates, and hydrates thereof | |
| 25 | GB1203 as described by Yao, E. H. et al. Cardiovasc. Res. 81, 797-804 (2009) and polymorphs, solvates, and hydrates thereof. | |
| 26 | Compounds having the structure | |
| | 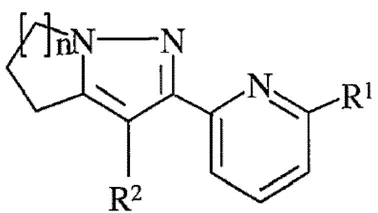 | |
| | wherein n is 1-2 | |
| | R1 is hydrogen or C1-C4 alkyl | |
| | R2 is selected from the group consisting of 1-*H*-pyrrolo[2,3-b], 1-*H-*pyrrolo[2,3c]pyridine, 1-*H*-pyrazolol[3,4-b]pyridine and 7-*H*-pyrrolo[2,3-d]pyrimidine all of which may be optionally substituted with C1-C4 alkyl or phenyl | |
| | and polymorphs, solvates, and hydrates thereof | |
| | as well as the compounds defined by the following structural formulae: | |
| | 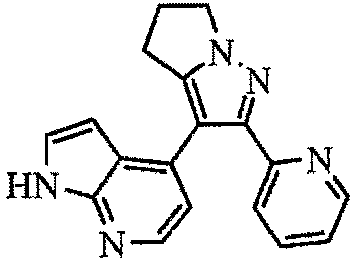 | 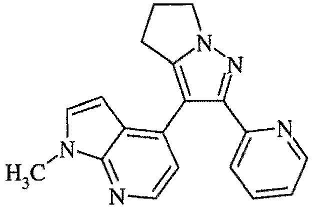 |
| | 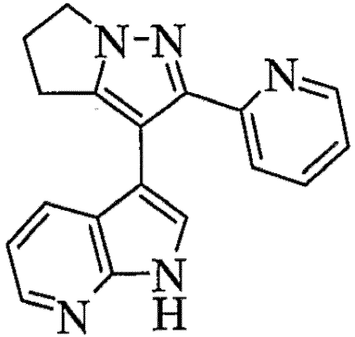 | 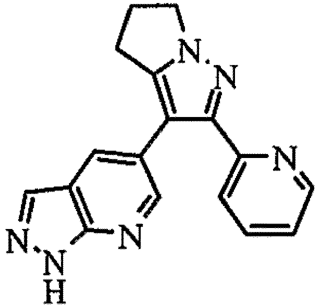 |
| | 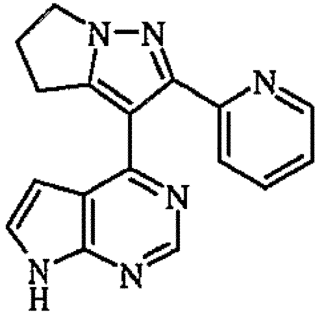 | 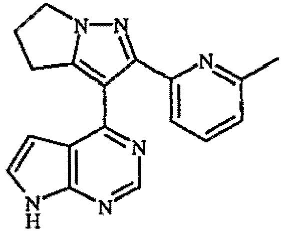 |
| | 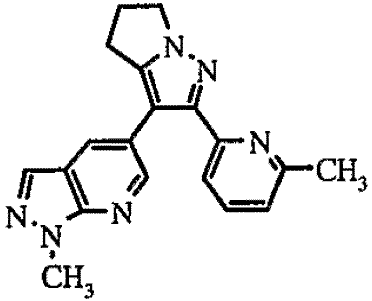 | 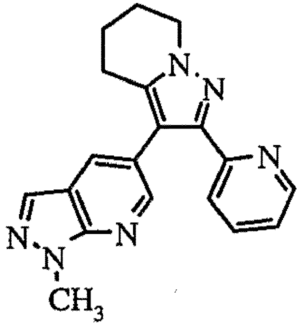 |
| | 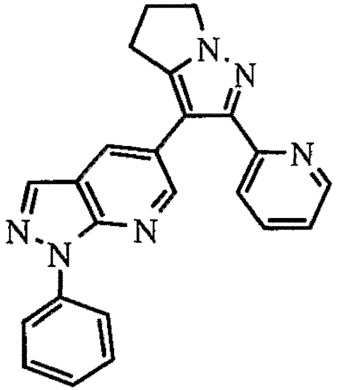 | 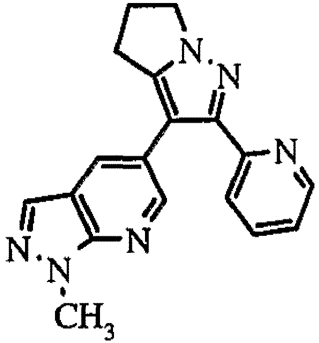 |
| | and polymorphs, solvates, and hydrates thereof. | |
| 27 | Compounds having the general structure | |
| | 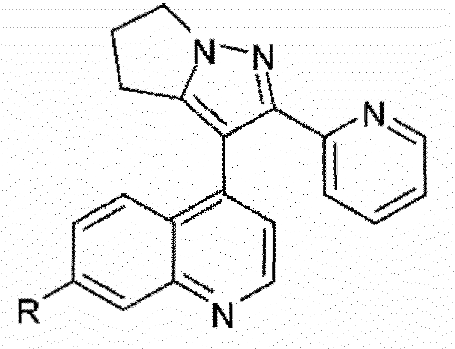 | |
| | **1:** R=H | |
| | **1a**: R=NHCOCH₂N(CH₃)₂ | |
| | **1b**: R=NH₂ | |
| | Wherein H is R, NHCOCH₂N(CH₃)₂ or NH₂ and polymorphs, solvates, and hydrates thereof. | |
| 28 | Compounds having the general structure | |
| | 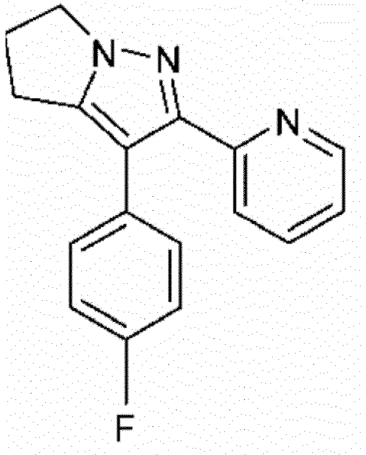 | |
| | and polymorphs, solvates, and hydrates thereof. | |
| 29 | Compounds having the general structure | |
| | 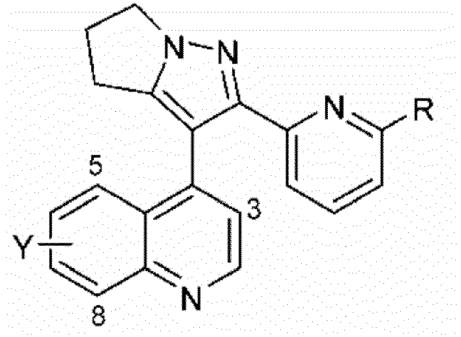 | |
| | wherein Y is 7-OMe and R is H or | |
| | Y is 2-Cl and R 9 is H or | |
| | Y is 6,8-(OMe)₂ and R is Me or | |
| | Y is 8-F and R is Me or | |
| | Y is 6-Br and R is Me or | |
| | Y is 6-OCF₃ and R is Me | |
| | and polymorphs, solvates, and hydrates thereof. | |
| 30 | Compounds having the general structure | |
| | 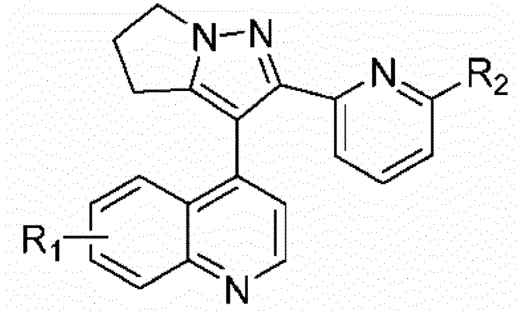 | |
| | Wherein R1 and R2 are | |

| | R1 | R2 |
|---|---|---|
| | | |
| | 2-Cl | H |
| | 6,8-OMe | Me |
| | 8-F | Me |
| | 6-Br | Me |
| | 6-OCF₃ | Me |
| | 6-COOMe | Me |
| | 6-CONH(CH₂)₂N(CH₃)₃ | Me |
| | 2-OMe | H |
| | 2-SEt | H |
| | 2-N(CH₂)₄ | H |
| | 7-OH | H |
| | 7-O(CH₂)₃N(CH₂CH₂)₂NCH₃ | H |
| | 7-O(CH₂)₂Cl | H |
| | 7-O(CH₂)₂N(CH₃)₂ | H |
| | 7-O(CH₂)₂N(CH₂CH₃)(CH₃) | H |
| | 7-O(CH₂)₂N(CH₂CH₂)₂NCH₃ | H |
| | 7-O(CH₂)₂N(CH₂CH₂)₂O | H |
| | -(CH₂)₅N(CH₃)₂ | H |
| | 7-OCH₂CON(CH₂CH₂)₂NCH₃ | H |
| | | |
| | and polymorphs, solvates, and hydrates thereof | |
| 31 | TGF-β receptor type I kinase inhibitors as described in, e.g., DaCosta Bayfield, (Mol. Pharmacol., 2004, 65:744-52), Laping, (Curr. Opin. Pharmacol., 2003, 3:204-8) and Laping (Mol. Pharmacol., 2002, 62:58-64) or any polymorph, solvate or hydrate thereof | |
| 32 | Dominant-negative TGF-β receptor as described by Bollard et al. (Blood, 2002, 99:3179-87) | |
| 33 | Avotermin as described by Occleston, N. L. et al.. Wound Repair Regen.19 (Suppl. 1), S38-S48 (2011). | |
| 34 | Pirfenidone as described by Sheppard, D. Proc. Am. Thorac. Soc. 3, 413-417 (2006) and polymorphs, solvates, and hydrates thereof. | |
| 35 | Losartan as described by Holm, T. M. et al. Science 332, 358-361 (2011) and polymorphs, solvates, and hydrates thereof. | |
| 36 | IMC-TR1 as described by Zhong, Z. et al. Clin. Cancer Res. 16, 1191-1205 (2010) or any antigen-binding fragment thereof. | |
| 37 | P17 as described by Llopiz, D. et al. Int. J. Cancer 125, 2614-2623, (2009). | |
| 38 | LSKL as described by Lu, A., et al., Am. J. Pathol. 178, 2573-2586 (2011) and polymorphs, solvates, and hydrates thereof. | |
| 39 | SR2F as described by J. Clin. Invest. 109, 1607-1615, (2002). | |
| 40 | Soluble proteins which naturally bind to and inhibit TGFβ (LAP, decorin, fibromodulin, lumican, endoglin, α2-macroglobulin) | |
| 41 | Receptors competing with the TGFβ endogenous receptor for binding to the ligand, such as BAMBI as described by Onichtchouk et al. (Nature, 1999, 401:480-5.) | |
| 42 | STX-100 (a humanized anti Anti-αvβ6 antibody) as described by Allison, M. Nature Biotech. 30, 375-376 (2012) or any antigen-binding fragment thereof. | |
| 43 | Inhibitory anti-TGFβ antibodies including, without limitation: | |
| | | - multispecific antibodies, |
| | | - polyclonal antobodies, |
| | | - monoclonal antibodies, |
| | | - F(ab')₂, and Fab fragments of antibodies, such as those described in EP117544, Ling et al., (J. Amer. Soc. Nephrol. 14: 377-388 (2003)), McCormick et al (J. Immunol., 1999, 163:5693-5699) and Cordeiro, (Curr. Opin. Mol. Ther., 2003, 5:199-203); |
| | | - the 2G7 antibody described by Arteaga et al. (J Clin Invest. 1993 Dec;92(6):2569-76) or any antigen-binding fragment thereof, |
| | | - the 1D11 antibody described by Dasch et al. (J Immunol. 1989 Mar 1;142(5):1536-41) or any antigen-binding fragment thereof, |
| | | - the GC1008 antibody described by Morris et al. (J Clin Oncol 26: 2008, May 20 suppl; abstr 9028) or any antigen-binding fragment thereof, |
| | | - Lerdelimumab as described by Mead, A. L., et al., Invest. Ophthalmol. Vis. Sci. 44, 3394-3401(2003) or any antigen-binding fragment thereof, |
| | | - Metelimumab as described by Denton, C. P. Arthritis Rheum. 56, 323-333 (2007) or any antigen-binding fragment thereof, |
| | | - Fresolimumab as described by Trachtman, H. et al. Kidney Int. 79, 1236-1243 (2011) or any antigen-binding fragment thereof. |
| | | - LY2382770 or any antigen-binding fragment thereof |
| 44 | Monoclonal and polyclonal antibodies specific to TGFβ receptor and TGFβ receptor soluble forms. | |
| 45 | Soluble forms TGFβ receptor, i.e. the extracellular domain of TGFβ receptor, which can be obtained physiologically by proteolytic processing of endoglin or betaglycan (type III receptors), or by recombinant technology by expressing only the extracellular domain of type I and type II TGFβ receptors. | |
| 46 | Antisense oligonucleotides specific for TGF-β, such as the AP11014 described by Schlingensiepen, K. H. et al. (J. Clin. Oncol. 22, Abstract 3132 (2004)) or the AP12009 described by Hau et al. (Expert Rev. Anticancer Ther., 2009, 9:1663-74) | |
| 47 | siRNA and shRNAs specific for TGF-β | |
| 48 | Aptamers specific for TGF-beta or for Smad2-4, such as the Trx-xFoxH1b aptamer described by Cui et al. (Oncogene, 2005, 24:3864-74) and the Trx-SARA aptamer described by Zhao and Hoffman (Mol. Biol. Cell., 2006, 17:3819-31) | |
| 49 | Peptides comprising the 112 amino acids counted from the end of the TGF-beta 1, TGF-beta 2 or TGF-beta 3 peptide. The start of those peptides is after the RXXR motif, ending 113 amino acids before the end of the TGF-β 1, TGF-β 2 or TGF-β 3 peptide, in which R is the amino acid Arginin and XX represents any amino acid or is even no amino acid. | |
| 50 | Fusion proteins comprising the TGFβR2 and Fc such as the TGFβRII:Fc described by Won et al. (Cancer Res. 1999, 59:1273-7) | |
| 51 | Fusion proteins comprising the TGFβR2 and beta-glycan such as the fusion protein described by Bandyopadhyay et al. (Oncogene, 2002, 21:3541-51) | |
| 52 | Combined vaccine-antisense Belagenpumatucel-L (allogeneic tumor cell vaccine comprising cells expressing an anti-TGFβ2 antisense) as described by Nemunaitis et al. (Cancer Gene Ther., 2009, 16:620-4) | |

The term "polymorph", as used herein, refers to a particular crystalline state of a substance, having particular physical properties described by X-ray diffraction patterns, IR spectra, phase transition point, and the like. The different polymorphs may result from differences in crystal packing (packing polymorphism) or differences in packing between different conformers of the same molecule (conformational polymorphism).

In a preferred embodiment, the TGFβ inhibitor is LY2157299 ((2-(6-methyl-pyridin-2-yl)-3-[6-amido-quinolin-4-yl)-5,6-dihydro-4H-pyrrolo[1,2-b]pyrazole) inhibitor of TGF-beta receptor type I; Lilly Research), as described in the international patent application published as WO2004048382-A1, having the structure 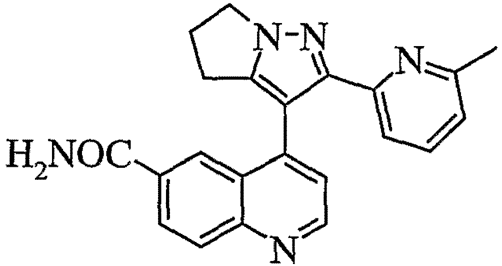 in a crystalline form or any polymorph, solvate or hydrate thereof.

In a preferred embodiment, the TGFβ inhibitor is a crystalline LY2157299 monohydrate characterized by the X-ray powder diffraction pattern (Cu radiation, λ= 1.54056 Å) comprising a peak at 9.05 and one or more peaks selected from the group comprising 11.02, 11.95, and 14.84 (2θ +/· 0.1°).

In a preferred embodiment, the TGFβ inhibitor is a crystalline LY2157299 monohydrate further characterized by the X-ray powder diffraction pattern (Cu radiation, λ= 1.54056 Å) comprising a peak at 9.05 (2 θ +/· 0.1°).

In another preferred embodiment, the TGFβ inhibitor is a crystalline LY2157299 monohydrate further characterized by the solid state 13C nuclear magnetic resonance having a chemical shift (ppm) of 108.8, 115.6, 122.6, and 171.0 (+/- 0.2) ppm.

As used herein, the term "solvate" means a compound which further includes a stoichiometric or non-stoichiometric amount of solvent such as water, acetone, ethanol, methanol, dichloromethane, 2-propanol, or the like, bound by non-covalent intermolecular forces. When the solvent is water, the term "hydrate" is used instead of solvate.

The present invention further provides antibodies and antibody fragments that specifically bind with such polypeptides. Exemplary antibodies include neutralizing antibodies, polyclonal antibodies, murine monoclonal antibodies, humanized antibodies derived from murine monoclonal antibodies, and human monoclonal antibodies. Illustrative antibody fragments include F(ab')2, F(ab)2, Fab', Fab, Fv, scFv, and minimal recognition units.

In a preferred embodiment, the therapy is neoadjuvant or adjuvant chemotherapy.

The term "neoadjuvant therapy", as used herein, refers to any type of treatment of cancer given prior to surgical resection of the primary tumor, in a patient affected with a cancer. The most common reason for neoadjuvant therapy is to reduce the size of the tumor so as to facilitate a more effective surgery. Neoadjuvant therapies comprise radiotherapy and therapy, preferably systemic therapy, such as hormone therapy, chemotherapy, immunotherapy and monoclonal antibody therapy.

The term "adjuvant therapy", as used herein, refers to any type of treatment of cancer (e.g., chemotherapy or radiotherapy) given as additional treatment, usually after surgical resection of the primary tumor, in a patient affected with a cancer that is at risk of metastasizing and/or likely to recur. The aim of such an adjuvant treatment is to improve the prognosis. Adjuvant therapies comprise radiotherapy and therapy, preferably systemic therapy, such as hormone therapy, chemotherapy, immunotherapy and monoclonal antibody therapy.

### Method for selecting a patient which is likely to benefit from adjuvant therapy after surgical resection of colorectal cancer

In another aspect, the invention relates to a method (hereinafter "second personalized therapeutic method of the invention") for selecting a patient which is likely to benefit from adjuvant therapy after surgical resection of colorectal cancer comprising the determination of the expression levels of the FAM46A, FHL2, FOXC2 and COL4A1 genes in a sample from said patient, wherein an increased expression level of said genes with respect to a reference value for said genes is indicative that the patient is likely to benefit from therapy after surgical treatment or wherein a decreased expression level of said genes with respect to a reference value for said genes is indicative that the patient is unlikely to benefit from therapy after surgical treatment.

As used herein, the terms "treatment" or "therapy" can be used indistinctly and refer to clinical intervention in an attempt to prevent, cure, delay, reduce the severity of, or ameliorate one or more symptoms of the disease or disorder or recurring disease or disorder, or in order to prolong the survival of a patient beyond that expected in the absence of such treatment.

The term "colorectal cancer" has been described in detail in the context of the prognostic methods of the invention and is used with the same meaning in the context of the personalized methods according to the invention.

In a first step, the second personalized therapeutic method according to the invention comprises the determination of the expression level of the FAM46A, FHL2, FOXC2 and COL4A1 genes in a sample from said patient.

The terms "colorectal cancer", "patient", "FAM46A gene", "FHL2gene", "FOXC2 gene", "COL4A1 gene", "expression levels", "sample", and "therapy" have been described in detail above and are equally applied to the methods according to the present method.

In a preferred embodiment, the second personalized therapeutic method according to the invention additionally comprises the determination of the expression levels of the FRMD6 gene and wherein the patient is a patient suffering from stage II colorectal cancer, wherein an increased expression level of said genes with respect to a reference value for said genes is indicative that the patient is likely to benefit from therapy after surgical treatment or wherein a decreased expression level of said genes with respect to a reference value for said genes is indicative that the patient is unlikely to benefit from therapy after surgical treatment.

In another preferred embodiment, the second personalized therapeutic method according to the invention additionally comprises the determination of the expression levels of the SPRY4 and DACT3 genes and wherein the patient is a patient suffering from stage III colorectal cancer and wherein increased expression of said genes is indicative that the patient is likely to benefit from therapy after surgical treatment or wherein a decreased expression level of said genes with respect to a reference value for said genes is indicative that the patient is unlikely to benefit from therapy after surgical treatment.

Moreover, in addition to the determination of the markers mentioned above, the method according to the invention may further comprise the determination of one or more markers selected from the group consisting of ACTC1, BOC, CNN1, COMP, CRISPLD2, CRYAB, FAM101B, FILIP1L, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2 wherein increased expression of said genes is indicative that the patient is likely to benefit from therapy after surgical treatment or wherein a decreased expression level of said genes with respect to a reference value for said genes is indicative that the patient is unlikely to benefit from therapy after surgical treatment. The patient may be a stage II or stage III patient.

Thus, in one embodiment, the method of the invention comprises the determination of the expression levels of the genes ACTC1, BOC, CNN1, COL4A1, COMP, CRISPLD2, CRYAB, FAM101B, FAM46A, FHL2, FILIP1L, FOXC2, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2 wherein increased expression of said genes is indicative that the patient is likely to benefit from therapy after surgical treatment or wherein a decreased expression level of said genes with respect to a reference value for said genes is indicative that the patient is unlikely to benefit from therapy after surgical treatment. The patient may be a stage II or stage III patient.

Thus, in one embodiment, the method of the invention comprises the determination of the expression levels of the genes ACTC1, BOC, CNN1, COL4A1, COMP, CRISPLD2, CRYAB, FAM101B, FAM46A, FHL2, FILIP1L, FOXC2, FRMD6, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2 wherein increased expression of said genes is indicative that the patient is likely to benefit from therapy after surgical treatment or wherein a decreased expression level of said genes with respect to a reference value for said genes is indicative that the patient is unlikely to benefit from therapy after surgical treatment. The patient may be a stage II or stage III patient.

In another embodiment, the method of the invention comprises the determination of the expression levels of the genes ACTC1, BOC, CNN1, COL4A1, COMP, CRISPLD2, CRYAB, DACT3, FAM101B, FAM46A, FHL2, FILIP1L, FOXC2, GAS7, GFPT2, GLIS3, HS3ST3A1, KIRREL, LRRC15, LTBP2, MFAP2, NNMT, P4HA3, PPAPDC1A, PPP1R3C, S1PR1 SPRY4 and SYNPO2 wherein increased expression of said genes is indicative that the patient is likely to benefit from therapy after surgical treatment or wherein a decreased expression level of said genes with respect to a reference value for said genes is indicative that the patient is unlikely to benefit from therapy after surgical treatment. The patient may be a stage II or stage III patient.

The terms referring to each of these genes have been described in detail above and are equally applied to the methods according to the present method.

In yet another embodiment, the second personalized therapeutic method according to the invention comprises the determination of the expression levels of the genes shown in Table 1 having a fold change value higher than 2 in either AAFs, CAFs and CCDs in response to TGF-beta and which are specifically expressed in cancer associated fibroblasts (FAP+; EPCAM- CD45- CD31-) or endothelial cells (CD31+; FAP- EpCAM- cd45-) versus epithelial cells (EPCAM+) and Leukocytes (cd45+) wherein an increased expression level of said genes with respect to a reference value for said genes is indicative that the patient is likely to benefit from therapy after surgical treatment or wherein a decreased expression level of said genes with respect to a reference value for said genes is indicative that the patient is unlikely to benefit from therapy after surgical treatment. Thus, in another embodiment, the second personalized therapeutic method according to the invention further comprise determination of the expression levels of one or more genes selected from the group consisting of ADAMTS6, BMP6, CCDC71L, CH25H, CNNM2, CPNE2, CREB3L2, DCBLD1, EFR3B, EGR1, ELN, ENDOD1, FHOD3, FOXC1, FZD8, GBP1, GXYLT2, IGF1, IL4R, ITGA11, JPH2, KIAA1211, KIF26B, LIMK1, LINC00340, LPCAT2, LRRC8A, METTL7A, OLFM2, PID1, PPP1R12B, PRSS23, RASD2, RNF152, SCUBE3, SEC14L2, SERPINE2, SGK1, SH3PXD2A, SHISA2, SMTN, SRGAP1, TRPC6 and UCK2 and of the genes which hybridize specifically with the probes having the sequences SEQ ID NO:1 to 26 wherein an increased expression level of said genes with respect to a reference value for said genes is indicative that the patient is likely to benefit from therapy after surgical treatment or wherein a decreased expression level of said genes with respect to a reference value for said genes is indicative that the patient is unlikely to benefit from therapy after surgical treatment.

The gene signature comprising all the genes which are determined according to the different methods of the invention is known as Str-TBRS and is shown in Table 1. In one embodiment, the invention comprises the determination of the expression levels of the genes ACTC1, ADAMTS6, BMP6, BOC, CCDC71L, CH25H, CNN1, CNNM2, COL4A1, COMP, CPNE2, CREB3L2, CRISPLD2, CRYAB, DACT3, DCBLD1, EFR3B, EGR1, ELN, ENDOD1, FAM101B, FAM46A, FHL2, FHOD3, FILIP1L, FOXC1, FOXC2, FRMD6, FZD8, GAS7, GBP1, GFPT2, GLIS3, GXYLT2, HS3ST3A1, IGF1, IL4R, ITGA11, JPH2, KIAA1211, KIF26B, KIRREL, LIMK1, LINC00340, LPCAT2, LRRC15, LRRC8A, LTBP2, METTL7A, MFAP2, NNMT, OLFM2, P4HA3, PID1, PPAPDC1A, PPP1R12B, PPP1R3C, PRSS23, RASD2, RNF152, S1PR1, SCUBE3, SEC14L2, SERPINE2, SGK1, SH3PXD2A, SHISA2, SMTN, SPRY4, SRGAP1, SYNPO2, TRPC6, and UCK2 genes and of the genes which hybridize specifically with the probes having the sequences SEQ ID NO:1 to 26 wherein an increased expression level of said genes with respect to a reference value for said genes is indicative that the patient is likely to benefit from therapy after surgical treatment or wherein a decreased expression level of said genes with respect to a reference value for said genes is indicative that the patient is unlikely to benefit from therapy after surgical treatment.

The term "specifically hybridizing", as used herein, refers to conditions which allow the hybridization of two polynucleotide sequences under high stringent conditions or moderately stringent conditions. The expressions "high stringent conditions" and "moderately stringent conditions" are defined below in respect to the kit of the invention and are equally applicable in the context of the present method.

The expression levels of the different genes used in the second personalized therapeutic method of the invention can be determined by determining the levels of the mRNA encoded by said genes or by determining the levels of the polypeptide encoded by said genes.

In a second step, the personalized therapeutic method according to the invention comprises the identification of those patients showing increased expression levels of said genes with respect to a reference value for said genes as patients who are likely to benefit from therapy after surgical treatment or of those patients showing decreased expression levels of the said genes with respect to a reference value for said genes as patients who are unlikely to benefit from therapy after surgical treatment.

The term "benefit" relates to improving the disease state of the patient. Beneficial or desired clinical results include, but not limiting, release of symptoms, reduction of the length of the disease, stabilized pathological state (specifically not deteriorated), retard in the disease's progression, improve of the pathological state, prolongation of survival compared to the expected survival if the treatment is not applied, and remission (both partial and total), both detectable and not detectable.

In a particular embodiment, the therapy after surgical treatment for which the patient is or is not candidate is a therapy selected from the group consisting of chemotherapy, radiotherapy and/or a therapy comprising a TGF-beta inhibitor.

The terms "surgery" or "surgical treatment", "chemotherapy", "chemotherapeutic drug", "radiotherapy", and "therapy comprising a TGF-beta inhibitor" have been described in detail above and are equally applied to the methods according to the present method.

### Personalized therapies of the invention

The prognostic method and the personalized therapeutic methods defined above also allow providing personalized therapies to patients suffering colorectal cancer. In particular, patients which are considered as having a high risk of relapse will most likely benefit from an additional therapy after surgery. Conversely, patients showing low risk of relapse may do without additional therapeutic treatment following surgery.

Thus, in another aspect, the invention relates to a chemotherapeutic compound and/or a TGF-beta inhibitor for use in the treatment of colorectal cancer in a patient after surgical removal of the cancer, wherein the patient has been selected by the prognostic method of the invention or the personalized therapeutic methods of the invention.

The term "TGF-beta inhibitor" has been described above in the context of the personalized method according to the invention and includes specific inhibitors of TGF-β1, TGF-β2 or TGF-β3 as well as broad spectrum inhibitors which inhibit two or more of the above isofoms.

In a preferred embodiment, the TGFβ inhibitor is LY2157299 ((2-(6-methyl-pyridin-2-yl)-3-[6-amido-quinolin-4-yl)-5,6-dihydro-4H-pyrrolo[1,2-b]pyrazole) inhibitor of TGF-beta receptor type I; Lilly Research) in a crystalline form or any polymorph, solvate or hydrate thereof.

In a preferred embodiment, the TGF-beta inhibitor is selected from the group consisting of any compound identified in Table 1 or any combination thereof. In a more preferred embodiment, the TFG-beta inhibitor is a compound having the structure 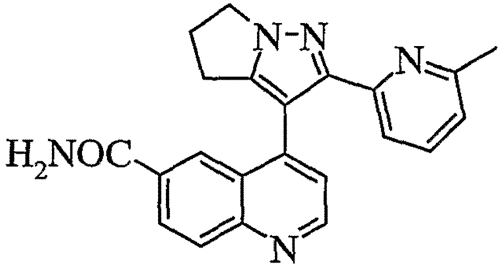 or any polymorph, solvate or hydrate thereof.

The terms "therapy", "treatment", "colorectal cancer", "patient", "radiotherapy", "surgery" or "surgical treatment", "chemotherapy", "chemotherapeutic drug", "radiotherapy", and "therapy comprising a TGF-beta inhibitor" have been described in detail above and are equally applied to the personalized therapies of the invention.

### Kits of the invention

In another embodiment, the invention relates to a kit which is useful for the determination of the expression levels of the genes forming the Str-TBRS signature of the invention or the minisignatures derived from the Str-TBRS of the invention. Thus, in a preferred embodiment, the kit of the invention comprises reagents adequate for the determination of the expression level of the FAM46A, FHL2, FOXC2 and COL4A1 genes, wherein the reagents adequate for the determination of the expression levels of the FAM46A, FHL2, FOXC2 and COL4A1 genes comprise at least 10% of the total amount of reagents adequate for the determination of the expression levels of genes forming the kit, wherein the reagents are selected from the group consisting of primers, probes, aptamers and antibodies. In another embodiment, the kit of the invention further comprises reagents adequate for the determination of the expression levels of the FRMD6 gene or for the SPRY4 and DACT3 genes, wherein the reagents are selected from the group consisting of primers, probes, aptamers and antibodies.

In another embodiment, the kit according to the invention further comprises reagents adequate for the determination of the expression levels of one or more genes selected from the group consisting of ACTC1, BOC, CNN1, COMP, CRISPLD2, CRYAB, FAM101B, FILIP1L, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2 genes, wherein the reagents are selected from the group consisting of primers, probes, aptamers and antibodies.

In one embodiment, the kit according to the invention comprises reagents adequate for the determination of the expression levels of the ACTC1, BOC, CNN1, COL4A1, COMP, CRISPLD2, CRYAB, FAM101B, FAM46A, FHL2, FILIP1L, FOXC2, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2 genes, wherein the reagents are selected from the group consisting of primers, probes, aptamers and antibodies.

In one embodiment, the kit according to the invention comprises reagents adequate for the determination of the expression levels of the ACTC1, BOC, CNN1, COL4A1, COMP, CRISPLD2, CRYAB, FAM101B, FAM46A, FHL2, FILIP1L, FOXC2, FRMD6, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2 genes, wherein the reagents are selected from the group consisting of primers, probes, aptamers and antibodies.

In another embodiment, the kit according to the invention comprises reagents adequate for the determination of the expression levels of the ACTC1, BOC, CNN1, COL4A1, COMP, CRISPLD2, CRYAB, DACT3, FAM101B, FAM46A, FHL2, FILIP1L, FOXC2, GAS7, GFPT2, GLIS3, HS3ST3A1, KIRREL, LRRC15, LTBP2, MFAP2, NNMT, P4HA3, PPAPDC1A, PPP1R3C, S1PR1 SPRY4 and SYNPO2 genes, wherein the reagents are selected from the group consisting of primers, probes, aptamers and antibodies.

In another embodiment, the kit according to the present invention further comprises reagents adequate for the determination of the expression levels of the genes which are specifically expressed in the cell populations enriched in cancer associated fibroblasts (FAP+) or endothelial cells (CD31+) and having at least a 2-fold increase in response to TGF-beta in either AAFs, CAFs or normal mucosa fibroblasts (CCD-co-18), wherein the reagents are selected from the group consisting of primers, probes, aptamers and antibodies. Thus, in another embodiment, the kit according to the present invention further comprises reagents adequate for the determination of the expression levels of the one or more genes selected from the group consisting of ADAMTS6, BMP6, CCDC71L, CH25H, CNNM2, CPNE2, CREB3L2, DCBLD1, EFR3B, EGR1, ELN, ENDOD1, FHOD3, FOXC1, FZD8, GBP1, GXYLT2, IGF1, IL4R, ITGA11, JPH2, KIAA1211, KIF26B, LIMK1, LINC00340, LPCAT2, LRRC8A, METTL7A, OLFM2, PID1, PPP1R12B, PRSS23, RASD2, RNF152, SCUBE3, SEC14L2, SERPINE2, SGK1, SH3PXD2A, SHISA2, SMTN, SRGAP1, TRPC6 and UCK2 and of the genes which hybridize specifically with the probes having the sequences SEQ ID NO:1 to 26, wherein the reagents are selected from the group consisting of primers, probes, aptamers and antibodies.

In another embodiment, the kit according to the present invention further comprises reagents adequate for the determination of the expression levels of the genes ACTC1, ADAMTS6, BMP6, BOC, CCDC71L, CH25H, CNN1, CNNM2, COMP, COL4A, CPNE2, CRISPLD2, CRYAB, DACT3, CREB3L2, DCBLD1, EFR3B, EGR1, ELN, ENDOD1, FAM101B, FAM46A, FHL2, FHOD3, FILIP1L, FOXC1, FOXC2, FRMD6, FZD8, GAS7, GBP1, GFPT2, GLIS3, GXYLT2, HS3ST3A1, IGF1, IL4R, ITGA11, JPH2, KIAA1211, KIF26B, KIRREL, LIMK1, LINC00340, LPCAT2, LRRC15, LRRC8A, LTBP2, METTL7A, MFAP2, NNMT, OLFM2, P4HA3, PID1, PPAPDC1A, PPP1R12B, PPP1R3C, PRSS23, RASD2, RNF152, S1PR1, SCUBE3, SEC14L2, SERPINE2, SGK1, SH3PXD2A, SHISA2, SMTN, SPRY4, SRGAP1, SYNPO2, TRPC6, and UCK2 genes and of the genes which hybridize specifically with the probes having the sequences SEQ ID NO:1 to 26, wherein the reagents are selected from the group consisting of primers, probes, aptamers and antibodies.

In a preferred embodiment, the reagents adequate for the determination of the expression levels of one or more genes comprise at least 20%, at least 30%, at least 40%, at least 50%, at least 60%, at least 70%, at least 80%, at least 90% or at least 100% of the total amount of reagents adequate for the determination of the expression levels of genes forming the kit. Thus, in the particular case of kits comprising reagents for the determination of the expression levels of the FAM46A, FHL2, FOXC2 and COL4A1 genes, the reagents specific for said genes (e.g. probes which are capable of hybridizing under stringent conditions to the FAM46A, FHL2, FOXC2 and COL4A1 genes) comprise at least 20%, at least 30%, at least 40%, at least 50%, at least 60%, at least 70%, at least 80%, at least 90% or at least 100% of the probes present in the kit.

In further embodiments, the reagents adequate for the determination of the expression levels of one or more genes comprise at least 55% at least 60%, at least 65%, at least 70%, at least 75%, at least 80%, at least 90%, at least 95%, at least 96%, at least 97%, at least 98% or at least 99% of the total amount of reagents forming the kit.

In the context of the present invention, "kit" is understood as a product containing the different reagents necessary for carrying out the methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

The expression "reagent which allows determining the expression level of a gene" means a compound or set of compounds that allows determining the expression level of a gene both by means of the determination of the level of mRNA or by means of the determination of the level of protein. Thus, reagents of the first type include probes capable of specifically hybridizing with the mRNAs encoded by said genes. Reagents of the second type include compounds that bind specifically with the proteins encoded by the marker genes and preferably include antibodies, although they can be specific aptamers.

In a particular embodiment of the kit of the invention, the reagents of the kit are nucleic acids which are capable of specifically detecting the mRNA level of the genes mentioned above and/or the level of proteins encoded by one or more of the genes mentioned above. Nucleic acids capable of specifically hybridizing with the genes mentioned above can be one or more pairs of primer oligonucleotides for the specific amplification of fragments of the mRNAs (or of their corresponding cDNAs) of said genes.

In a preferred embodiment, the first component of the kit of the invention comprises a probe which can specifically hybridize to the genes mentioned above.

The term "specifically hybridizing", as used herein, refers to conditions which allow hybridizing of two polynucleotides under high stringent conditions or moderately stringent conditions.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50° C.; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C.; or (3) employ 50% formamide, 5xSSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42°C., with washes at 42°C in 0.2xSSC (sodium chloride/sodium citrate) and 50% formamide, followed by a high-stringency wash consisting of 0.1xSSC containing EDTA at 55 °C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37°C. in a solution comprising: 20% formamide, 5xSSC (150 mM NaCl, 15 mM trisodium citrate), 50 mM sodium phosphate (pH 7.6), 5x Denhardt's solution, 10% dextran sulfate, and 20 mg/ml denatured sheared salmon sperm DNA, followed by washing the filters in 1xSSC at about 37-50°C. The skilled artisan will recognize how to adjust the temperature, ionic strength, etc. as necessary to accommodate factors such as probe length and the like.

In the event that the expression levels of several of the genes identified in the present invention are to be simultaneously determined, it is useful to include probes for all the genes the expression of which is to be determined in a microarray hybridization.

The microarrays comprise a plurality of nucleic acids that are spatially distributed and stably associated to a support (for example, a biochip). The nucleic acids have a sequence complementary to particular subsequences of genes the expression of which is to be detected, therefore are capable of hybridizing with said nucleic acids. In the methods of the invention, a microarray comprising an array of nucleic acids is put into contact with a preparation of nucleic acids isolated from the patient object of the study. The incubation of the microarray with the preparation of nucleic acids is carried out in conditions suitable for the hybridization. Subsequently, after the elimination of the nucleic acids which have not been retained in the support, the hybridization pattern is detected, which provides information on the genetic profile of the sample analyzed. Although the microarrays are capable of providing both qualitative and quantitative information of the nucleic acids present in a sample, the invention requires the use of arrays and methodologies capable of providing quantitative information.

The invention contemplates a variety of arrays with regard to the type of probes and with regard to the type of support used. The probes included in the arrays that are capable of hybridizing with the nucleic acids can be nucleic acids or analogs thereof which maintain the hybridization capacity such as for example, nucleic acids in which the phosphodiester bond has been substituted with a phosphorothioate, methylimine, methylphosphonate, phosphoramidate, guanidine bond and the like, nucleic acids in which the ribose of the nucleotides is substituted with another hexose, peptide nucleic acids (PNA). The length of the probes can of 5 to 50 nucleotides and, preferably, of 7, 10, 15, 20, 25, 30, 35, 40, 45, 50, 55, 60, 65, 70, 75, 100 nucleotides and vary in the range of 10 to 1000 nucleotides, preferably in the range of 15 to 150 nucleotides, more preferably in the range of 15 to 100 nucleotides and can be single-stranded or doublestranded nucleic acids. The array can contain all the specific probes of a certain mRNA of a certain length or can contain probes selected from different regions of an mRNA. Each probe is assayed in parallel with a probe with a changed base, preferably in a central position of the probe. The array is put into contact with a sample containing nucleic acids with sequences complementary to the probes of the array and the signal of hybridization with each of the probes and with the corresponding hybridization controls is determined. Those probes in which a higher difference is observed between the signal of hybridization with the probe and its hybridization control are selected. The optimization process can include a second round of optimization in which the hybridization array is hybridized with a sample that does not contain sequences complementary to the probes of the array. After the second round of selection, those probes having signals of hybridization lower than a threshold level will be selected. Thus, probes which pass both controls, i.e., which show a minimum level of unspecific hybridization and a maximum level of specific hybridization with the target nucleic acid are selected.

The selection of the specific probes for the different target genes is carried out such that they bind specifically to the target nucleic acid with a minimum hybridization to non-related genes. However, there are probes of 20 nucleotides which are not unique for a certain mRNA. Therefore, probes directed to said sequences will show a cross-hybridization with identical sequences that appear in mRNA of non-related genes. In addition, there are probes that do not specifically hybridize with the target genes in the conditions used (because of secondary structures or of interactions with the substrate of the array). This type of probe must not be included in the array. Therefore, the person skilled in the art will observe that the probes that are going to be incorporated in a certain array must be optimized before their incorporation to the array. The optimization of the probes is generally carried out by generating an array containing a plurality of probes directed to the different regions of a certain target polynucleotide. This array is put into contact firstly with a sample containing the target nucleic acid in an isolated form and, secondly, with a complex mixture of nucleic acids. Probes which show a highly specific hybridization with the target nucleic acid but low or no hybridization with the complex sample are thus selected for their incorporation to the arrays of the invention. Additionally, it is possible to include in the array hybridization controls for each of the probes that is going to be studied. In a preferred embodiment, the hybridization controls contain an altered position in the central region of the probe. In the event that high levels of hybridization are observed between the studied probe and its hybridization control, the probe is not included in the array.

The microarrays of the invention contain not only specific probes for the polynucleotides indicating a determined pathophysiological situation, but also containing a series of control probes, which can be of three types: normalization controls, expression level controls and hybridization controls.

Normalization controls are oligonucleotides that are perfectly complementary to labeled reference sequences which are added to the preparation of nucleic acids to be analyzed. The signals derived from the normalization controls after the hybridization provide an indication of the variations in the hybridization conditions, intensity of the marker, efficiency of the detection and another series of factors that can result in a variation of the signal of hybridization between different microarrays. The signals detected from the rest of probes of the array are preferably divided by the signal emitted by the control probes, thus normalizing the measurements. Virtually any probe can be used as normalization control. However, it is known that the efficiency of the hybridization varies according to the composition of nucleotides and the length of the probe. Therefore, preferred normalization probes are those which represent the mean length of the probes present in the array, although they can be selected such that they include a range of lengths that reflect the rest of probes present in the array. The normalization probes can be designed such that they reflect the mean composition of nucleotides of the rest of probes present in the array. A limited number of normalization probes is preferably selected such that they hybridize suitably, i.e., they do not have a secondary structure and do not show sequence similarity with any of the probes of the array is used. The normalization probes can be located in any position in the array or in multiple positions in the array to efficiently control variations in hybridization efficiency related to the structure of the array. The normalization controls are preferably located in the corners of the array and/or in the center thereof.

The expression controls levels are probes which hybridize specifically with genes which are expressed constitutively in the sample which is analyzed. The expression level controls are designed to control the physiological state and the metabolic activity of the cell. The examination of the covariance of the expression level control with the expression level of the target nucleic acid indicates if the variations in the expression levels are due to changes in the expression levels or are due to changes in the overall transcriptional rate in the cell or in its general metabolic activity. Thus, in the case of cells which have deficiencies in a certain metabolite essential for cell viability, the observation of a decrease both in the expression levels of the target gene as in the expression levels of the control is expected. On the other hand, if an increase in the expression of the expression of the target gene and of the control gene is observed, it probably due to an increase of the metabolic activity of the cell and not to a differential increase in the expression of the target gene. Probes suitable for use as expression controls correspond to genes expressed constitutively, such as genes encoding proteins which exert essential cell functions such as β-2-microglobulin, ubiquitin, ribosomal protein 18S, cyclophilin A, transferrin receptor, actin, GAPDH, tyrosine 3-monooxygenase/tryptophan 5-monooxygenase activation protein (YWHAZ) and beta-actin.

Hybridization controls can be included both for the probes directed to target genes and for the probes directed to the expression level or to the normalization controls. Error controls are probes of oligonucleotides identical to the probes directed to target genes but which contain mutations in one or several nucleotides, i.e., which contain nucleotides in certain positions which do not hybridize with the corresponding nucleotide in the target gene. The hybridization controls are selected such that, applying the suitable hybridization conditions, the target gene should hybridize with the specific probe but not with the hybridization control or with a reduced efficiency. The hybridization controls preferably contain one or several modified positions in the center of the probe. The hybridization controls therefore provide an indication of the degree of unspecific hybridization or of cross-hybridization to a nucleic acid in the sample to a probe different from that containing the exactly complementary sequence.

The arrays of the invention can also contain amplification and sample preparation controls which are probes complementary to subsequences of selected control genes because they normally do not appear in the biological sample object of the study, such as probes for bacterial genes. The RNA sample is supplemented with a known amount of a nucleic acid which hybridizes with the selected control probe. The determination of the hybridization to said probe indicates the degree of recovery of the nucleic acids during their preparation as well as an estimation of the alteration caused in the nucleic acids during the processing of the sample.

Once a set of probes showing the suitable specificity and a set of control probes are provided, the latter are arranged in the array in a known position such that, after the steps of hybridization and of detection, it is possible to establish a correlation between a positive signal of hybridization and the particular gene from the coordinates of the array in which the positive signal of hybridization is detected.

The microarrays can be high density arrays with thousands of oligonucleotides by means of photolithographic in situ synthesis methods (Fodor et al., 1991, Science, 767-773). This type of probe is usually redundant, i.e., they include several probes for each mRNA which is to be detected. In a preferred embodiment, the arrays are low density arrays or LDA containing less than 10000 probes per square centimeter. In said low density arrays, the different probes are manually applied with the aid of a pipette in different locations of a solid support (for example, a crystal surface, a membrane). The supports used to fix the probes can be obtained from a large variety of materials, including plastic, ceramics, metals, gels, membranes, crystals and the like. The microarrays can be obtained using any methodology known for the person skilled in the art.

After the hybridization, in the cases in which the non-hybridized nucleic acid is capable of emitting a signal in step of detection, a step of washing is necessary to eliminate said non-hybridized nucleic acid. The step of washing is carried out using methods and solutions known by the person skilled in the art.

In the event that the labeling in the nucleic acid is not directly detectable, it is possible to connect the microarray comprising the target nucleic acids bound to the array with the other components of the system necessary to cause the reaction giving rise to a detectable signal. For example, if the target nucleic acids are labeled with biotin, the array is put into contact with conjugated streptavidin with a fluorescent reagent in suitable conditions so that the binding between biotin and streptavidin occurs. After the incubation of the microarray with the system generating the detectable signal, it is necessary to carry out a step of washing to eliminate all the molecules which have bound non-specifically to the array. The washing conditions will be determined by the person skilled in the art using suitable conditions according to the system generating the detectable signal and which are well-known for the person skilled in the art.

The resulting hybridization pattern can be viewed or detected in several different ways, said detection being determined by the type of system used in the microarray. Thus, the detection of the hybridization pattern can be carried out by means of scintillation counting, autoradiography, determination of a fluorescent signal, calorimetric determinations, detection of a light signal and the like.

After the hybridization and the possible subsequent washing and treatment processes, the hybridization pattern is detected and quantified, for which the signal corresponding to each point of hybridization in the array is compared to a reference value corresponding to the signal emitted by a known number of terminally labeled nucleic acids in order to thus obtain an absolute value of the number of copies of each nucleic acid which is hybridized in a certain point of the microarray.

In the event that the expression levels of the genes according to the present invention is determined by measuring the levels of the polypeptide or polypeptides encoded by said gene or genes, the kits according to the present invention comprise reagents which are capable of specifically binding to said polypeptides.

For this purpose, the arrays of antibodies such as those described by De Wildt et al. (2000) Nat. Biotechnol. 18:989-994; Lueking et al. (1999) Anal. Biochem. 270:103-111; Ge et al. (2000) Nucleic Acids Res. 28, e3, I-VII; MacBeath and Schreiber (2000) Science 289:1760-1763; WO 01/40803 and WO 99/51773A1 are useful. The antibodies of the array include any immunological agent capable of binding to a ligand with high affinity, including IgG, IgM, IgA, IgD and IgE, as well as molecules similar to antibodies which have an antigen binding site, such as Fab', Fab, F(ab')2, single domain antibodies or DABS, Fv, scFv and the like. The techniques for preparing said antibodies are very well-known for the person skilled in the art and include the methods described by Ausubel et al. (Current Protocols in Molecular Biology, eds. Ausubel et al, John Wiley & Sons (1992)).

The antibodies of the array can be applied at high speed, for example, using commercially available robotic systems (for example, those produced by Genetic Microsystems or Biorobotics). The substrate of the array can be nitrocellulose, plastic, crystal or can be of a porous material as for example, acrylamide, agarose or another polymer. In another embodiment, it is possible to use cells producing the specific antibodies for detecting the proteins of the invention by means of their culture in array filters. After the induction of the expression of the antibodies, the latter are immobilized in the filter in the position of the array where the producing cell was located. An array of antibodies can be put into contact with a labeled target and the binding level of the target to the immobilized antibodies can be determined. If the target is not labeled, a sandwich type assay can be used in which a second labeled antibody specific for the polypeptide which binds to the polypeptide which is immobilized in the support is used. The quantification of the amount of polypeptide present in the sample in each point of the array can be stored in a database as an expression profile. The array of antibodies can be produced in duplicate and can be used to compare the binding profiles of two different samples.

In another aspect, the invention relates to the use of a kit of the invention for predicting the outcome of a patient suffering colorectal cancer, or for determining whether a patient suffering colorectal cancer is candidate to therapy after surgery, or for selecting a patient which is likely to benefit from adjuvant therapy after surgical resection of colorectal cancer. In a preferred embodiment, the use of the kits according to the invention is carried out in patients suffering stage II or stage III CRC.

### Methods for the treatment of colorectal cancer metastasis

The authors of the present invention have observed that pharmacological inhibition of TGF-beta signalling blocks metastasis initiation from colorectal cancer (see example 4). Thus, the document also discloses a TGF-beta inhibitor for use in the treatment or prevention of colorectal cancer metastasis. In another disclosure, the document teaches a method for the treatment or prevention of colorectal cancer metastasis in a subject in need thereof comprising the administration to said patient of a TGF-beta inhibitor.

The term "metastasis" as used herein refers to the growth of a cancerous tumor in an organ or body part, which is not directly connected to the organ of the original cancerous tumor. Metastasis will be understood to include micrometastasis, which is the presence of an undetectable amount of cancerous cells in an organ or body part which is not directly connected to the organ of the original cancerous tumor. In a preferred embodiment, the metastasis is liver metastasis.

In a particular disclosure, the invention relates to a TGF-beta inhibitor for use in the treatment or prevention of colorectal cancer metastasis derived from a colorectal tumor that has a high content in colon cancer stem cells (CoCSCs). The terms "colon cancer stem cell" or "CoCSC", as used herein, refer to a cell that has the ability to extensively proliferate and form new colon tumours, i.e., cells with indefinite proliferative potential that drive the formation and growth of colon tumours. A cancer stem cell has the ability to re-grow a tumor as demonstrated by its ability to form tumors in immunocompromised mice, and typically to form tumors upon subsequent serial transplantation in immunocompromised mice. The term "high content" means that the colorectal cancer has a percentage of CoCSC of at least 10%, at least 20%, at least 30%, at least 40%, at least 50%, at least 60%, at least 70%, at least 80%, at least 90% or up to 100% of the cells of the colorectal tumor. The skilled person knows how to determine if a cell is a CoCSC as well as how to quantify the content of CoCSC of a colorectal tumor using, for example, the methods described by Merlos-Suarez et al., (2011, Cell Stem Cell 8, 511-524).

The term "TGF-beta inhibitor" has been described above in the context of the personalized method according to the invention and includes specific inhibitors of TGF-β1, TGF-β2 or TGF-β3 as well as broad spectrum inhibitors which inhibit two or more of the above isofoms.

In a preferred disclosure, the TGFβ inhibitor is LY2157299 ((2-(6-methyl-pyridin-2-yl)-3-[6-amido-quinolin-4-yl)-5,6-dihydro-4H-pyrrolo[1,2-b]pyrazole) inhibitor of TGF-beta receptor type I; Lilly Research) in a crystalline form or any polymorph, solvate or hydrate thereof.

In a preferred disclosure, the TGF-beta inhibitor is selected from the group consisting of any compound identified in Table 1 or any combination thereof. In a more preferred disclosure, the TFG-beta inhibitor is a compound having the structure 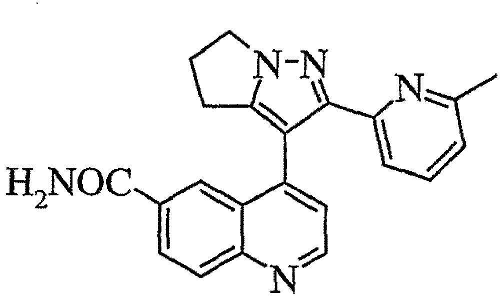 or any polymorph, solvate or hydrate thereof.

Methods of the present disclosure include administration of a TGF-beta inhibitor by a route of administration including, but not limited to, oral, rectal, nasal, pulmonary, epidural, ocular, otic, intraarterial, intracardiac, intracerebroventricular, intradermal, intravenous, intramuscular, intraperitoneal, intraosseous, intrathecal, intravesical, subcutaneous, topical, transdermal, and transmucosal, such as by sublingual, buccal, vaginal, and inhalational, routes of administration.

The TGF-beta inhibitors for use according to the disclosure may be formulated into a pharmaceutical composition. This pharmaceutical composition may be in any dosage form suitable for administration to a subject, illustratively including solid, semisolid and liquid dosage forms such as tablets, capsules, powders, granules, suppositories, pills, solutions, suspensions, ointments, lotions, creams, gels, pastes, sprays and aerosols. Liposomes and emulsions are well-known types of pharmaceutical formulations that can be used to deliver a pharmaceutical agent, particularly a hydrophobic pharmaceutical agent. The pharmaceutical compositions generally include a pharmaceutically acceptable carrier such as an excipient, diluent and/or vehicle. Delayed release formulations of compositions and delayed release systems, such as semipermeable matrices of solid hydrophobic polymers can be used.

The term "pharmaceutically acceptable carrier" refers to a carrier which is suitable for use in a subject without undue toxicity or irritation to the subject and which is compatible with other ingredients included in a pharmaceutical composition. Pharmaceutically acceptable carriers, methods for making pharmaceutical compositions and various dosage forms, as well as modes of administration are well-known in the art, for example as detailed in Pharmaceutical Dosage Forms: Tablets, eds. H. A. Lieberman et al., New York: Marcel Dekker, Inc., 1989; and in L. V. Allen, Jr. et al., Ansel's Pharmaceutical Dosage Forms and Drug Delivery Systems, 8th Ed., Philadelphia, Pa.: Lippincott, Williams and Wilkins, 2004; A. R. Gennaro, Remington: The Science and Practice of Pharmacy, Lippincott Williams and Wilkins, 21st ed., 2005, particularly chapter 89; and J. G. Hardman et al., Goodman and Gilman's The Pharmacological Basis of Therapeutics, McGraw-Hill Professional, 10th ed., 2001.

The dosage of the TGF-beta for use according to the method of the disclosure will vary based on factors such as, but not limited to, the route of administration; the age, health, sex, and weight of the subject to whom the composition is to be administered; the nature and extent of the subject's symptoms, if any, and the effect desired. Dosage may be adjusted depending on whether treatment is to be acute or continuing. One of skill in the art can determine a pharmaceutically effective amount in view of these and other considerations typical in medical practice. Detailed information concerning customary ingredients, equipment and processes for preparing dosage forms is found in Pharmaceutical Dosage Forms: Tablets, eds. H. A. Lieberman et al., New York: Marcel Dekker, Inc., 1989; and in L. V. Allen, Jr. et al., Ansel's Pharmaceutical Dosage Forms and Drug Delivery Systems, 8th Ed., Philadelphia, Pa.: Lippincott, Williams and Wilkins, 2004; A. R. Gennaro, Remington: The Science and Practice of Pharmacy, Lippincott Williams and Wilkins, 21st ed., 2005, particularly chapter 89; and J. G. Hardman et al., Goodman and Gilman's The Pharmacological Basis of Therapeutics, McGraw-Hill Professional, 10th ed., 2001.

In a particular disclosure, the patient has been selected as likely to respond to the therapy with a TGF-beta inhibitor by the prognostic method of the invention or the personalized therapeutic methods of the invention.

The invention is detailed below by means of the following examples which are merely illustrative and by no means limiting for the scope of the invention.

### EXAMPLES

### Materials and methods

### Clinical material

Human tissue samples were obtained from the Pathology Department of Hospital del Mar or Hospital Clinic (Barcelona, Spain) with the approval of the Bank Tumor Committee according to Spanish Ethical regulations. The study followed the guidelines of the Declaration of Helsinki and patient's identity of pathological specimens remained anonymous in the context of this study. Human fibroblasts were grown from fresh explants obtained from either a colorectal adenoma (adenoma associated fibroblasts or AAFs) or a colorectal carcinoma sample (cancer associated fibroblasts or CAFs) by standard protocols (Freshney; Culture of animal cells: a manual of basic techniques, Fourth edition, published by Wiley-Liss). Human colon normal fibroblasts (CCD-18Co) were obtained from ATCC. All fibroblasts were cultured for less than 10 passages prior to experiments.

### Classification of tumors samples according to p-Smad3 staining

Adenomas (n = 25) and CRC (n = 30) samples routinely collected at Hospital del Mar were stained with anti-p-SMAD3 antibody. Qualitative categorization of the samples according overall p-SMAD3 intensity or staining in tumor-associated stroma and epithelial cancer cells was performed by an expert pathologist (M.I). Average staining levels were summarized into three categories; high, medium and low. For tumor-associated stroma, all cell types were taken into consideration.

### Tumor disaggregation and staining

Freshly obtained tumours from CRC patients treated at Hospital del Mar or Hospital Clinic (Barcelona, Spain) were minced with sterile razor blade and incubated for 20-30 minutes at 37°C in DMEM/F12 (Gibco), containing 100X penicillin/streptomycin (Gibco); and Collagenase IV (Sigma; 100 U mL⁻¹). Pieces were then homogenized by pipetting and passed through consecutive 18G and 21G needles. Enzymatic reaction was stopped by adding 10% FBS and single cells were collected by sequential filtering through cell strainers of 100 µm → 70 µm → 40 µm (BD Falcon). Cells were centrifuged, resuspended in 5 ml ammonium chloride (0.15M; Sigma Aldrich) and incubated 3 minutes at room temperature to lyse erythrocytes. After two washes with HBSS (Lonza), cells were stained in staining buffer (SB: DMEM/F12 + 5% FBS) with FAP unconjugated antibody (30 min; 1/50). After two washes with HBSS (Lonza), cells were stained in SB with an APC conjugated donkey anti-rabbit antibody (30 min; 1/400; Jackson); anti-hEPCAM/TROPl-FITC conjugated antibody (30 min; 1/50; R&D), anti-CD31-PE conjugated antibody (10 min; 1/10; Miltenyi Biotec), anti-CD45-PE-Cy7 conjugated antibody (30 min; 1/50; R&D). Dead cells were labelled with Propidium Iodide (Sigma Aldrich). Fluorescence Activated Cell Sorting (FACS) was used to separate 2000 cells from each tumour cell population; cells of Hematopoietic origin [CD45+EPCAM-CD31-FAP-], tumour epithelial cells [CD45-EPCAM+CD31-FAP-], endothelial cells [CD45-EPCAM-CD31+FAP-] and cancer associated fibroblasts [CD45-EPCAM-CD31-FAP+] from six primary CRC samples. RNA was processed and amplified as previously described (Gonzalez-Roca et al., 2010, PLoS One 5, e14418). Each sample (4 tumour cell populations per 6 CRC samples) was hybridized on HT HG-U133+ PM arrays (GEO dataset GSE39396). We also isolated by FACS CD45+EPCAM-, CD45-EPCAM+ and CD45-EPCAM- populations from eight additional CRC samples. This second set (3 tumour cell populations per 8 samples) was hybridized on Affymetrix Human Genome U133 Plus 2.0 Array (GEO dataset GSE39395). Labelling and hybridization of samples on Affymetrix gene expression chips were performed by the IRB Transcriptomic Core Facility using standard methodology. For analysis, we pooled 14 CD45+ and EPCAM+ arrays from the two sets of hybridizations. We used limma to adjust for batch effects at the probeset level. We performed the three contrasts FAP+ *vs* CD31+, FAP+ vs CD45+ and FAP+ vs EPCAM+, and defined FAP-specific genes as those up-regulated with limma p-value < 0.05 (Smyth et al., 2004, Linear Models for Microarray, User's Guide) in all three comparisons. We defined CD31, CD45 and EPCAM-specific genes analogously.

### Generation of individual TBRS gene expression signatures

AAFs, CAFs, and normal colon mucosa-derived fibroblasts (CCD_co_18) were seeded at 60% confluence and cultured in low serum conditions (0.2%) before treatment with TGF-beta 1 (Peprotech; 5 ng mL⁻¹) for 8 hours. Gene expression profiles were measured in duplicate using HG-U133 plus 2.0. We used RMA background correction, quantile normalization and RMA summarization (Gautier et al., 2004, Bioinformatics 20, 307-315). A TGF-beta response signature (TBRS) was obtained for each fibroblast population by selecting genes with limma p-value < 0.05 and at least two fold up-regulation in TGF-beta treated fibroblasts.

From each individual TGF-beta response signature (TBRS) derived from the above experiment (AAF-TBRS; CRC-TBRS and CCD-TBRS) we selected those genes specifically expressed in cancer associated fibroblasts (FAP+) and/or endothelial cells (CD31+) from 16 colorectal cancer patients (GEO datasets GSE39396 and GSE39395) defined as described above and in Calon et al., Cancer Cell 2012, Nov 13;22(5):571-84. In addition, we substracted all those genes appearing in the publication Calon et al., Cancer Cell 2012, Nov 13;22(5):571-84. This analysis yielded a signature of 73 annotated genes (134 probes; TBRS of this invention) from which we derived specific predictors.

TGF-beta response signatures in T-Cells (Stockis et al., 2009, Eur J Immunology, 2009, 39: 869-82), macrophages (Gratchev et al., 2008, J. Immunology 2008, 180: 6553-65) and endothelial cells (Wu et al., 2006, Microvascular. Res. 2006, 71: 12-9) had been previously described. For the T-TBRS we used the GEO dataset GSE14330 (Stockis et al., 2009, *supra*.) and for macrophages (Ma-TBRS) we used GSE7568 (Gratchev et al., 2008, *supra*.)*.* TBRS signatures were defined as genes with limma p-value < 0.05 and at least two fold up-regulated in TGF-beta treated samples. In the case of T-Cells we pooled together gene expression datasets of independent Th clones cultured plus or minus addition of TGF-beta as described in GSE14330 (Stockis et al., 2009, *supra*.). For the case of macrophages, we compared microarray data from mature macrophages (treated with IL4 in combination with dexamethasone for 5 days), stimulated or not with TGF-beta during 24h as detailed in GSE7568 (n = 5 samples in each group). For the endothelial TGF-beta response signature (End-TBRS), we used the list of genes upregulated in Human Microvessel Endothelial Cells upon TGF-beta exposure as previously described (Wu et al., 2006, *supra*.)*,* again selecting genes with p-value < 0.05 and at least two fold up-regulated. The Str-TBRS was derived as describe above from CCD-co-18 normal intestinal fibroblasts cultured in 0.5% serum conditions before treatment with TGF-beta 1 (Peprotech; 5 ng mL⁻¹) for 8 hours.

### Datasets

Datasets corresponding to human colon adenomas and carcinomas have been previously described (Sabates-Bellver et al., 2007; Mol Cancer Res 5, 1263-1275; van der Flier et al., 2007; Gastroenterology 132, 628-632). To correlate gene expression with clinical disease progression, we pooled two sets of Affymetrix transcriptomic profiles (GSE17537 and GSE14333), available at Gene Expression Omnibus, www.ncbi.nlm.nih.gov/geo, corresponding to primary CRCs for which clinical follow-up was available. GSE17537is composed of 55 colon cancer patients treated at Vanderbilt University Medical Center (Vanderbilt, USA). GSE14333contains a pool of 290 CRC patients treated at two different hospitals; Peter MacCallum Cancer Center (Australia) and H. Lee Moffitt Cancer Center (USA). Available annotated clinical data for GSE17537 and GSE14333 datasets included AJCC staging, age, gender and disease free survival intervals. The representation of tumor samples at different AJCC stages in these cohorts follows the natural distribution of CRC patients receiving standard treatment in the aforementioned hospitals. In order to remove systematic biases between datasets, expression levels for all genes were transformed to z-scores prior to pooling. For in silico validation studies two additional cohorts were used, datasets GSE33113 and GSE26906. GSE33113 contained a set of 90 AJCC stage II CRC patient material collected in the Academic Medical Center (AMC) in Amsterdam, The Netherlands. Extensive medical records were kept from these patients and long-term clinical follow-up was available for the large majority. The 90 stage II CRC samples in cohort described in GSE26906 (Birnbaum et al. 2012; Transl Oncol 5:72-6) did not contain clinical follow up, only data on whether patients experienced Metastasis or not.

### Association of gene expression signatures with clinical parameters.

GSEA (Subramanian et al., 2005, Proc. Natl. Acad. Sci. USA 102, 15545-15550) was based on ranking genes according to their fold change for the indicated variables. The output of GSEA is an enrichment score (ES), a normalized enrichment score (NES) which accounts for the size of the gene set being tested, a p-value and an estimated False Discovery rate (FDR). We used the GSEA implementation provided in the Bioconductor package phenoTest. We computed p-values using 10,000 permutations for each signature and adjusted them with the Benjamini-Yekutieli method (Benjamini et al., 2001, Behav Brain Res 125, 279-284).

A SCAD-based logistic regression model was fitted (Fan & Li, 1999, Journal of the American Statistical Association, 1999, 96: 1348-1360) to predict recurrence events based on patient age, gender, staging and gene expression, and selected the variables with non-zero coefficient estimates. The SCAD penalization parameter was set via 10-fold cross-validation, as implemented in the R package ncvreg. We performed this analysis for stage II, III and also for all patients, which provided several short and highly predictive gene signatures.

In order to further assess the association of each gene signature and disease-free survival, the average signature expression was computed (i.e. across all genes in the signature) and fit multivariate Cox proportional-hazards models that included staging as an adjustment variable. To visualize the results we stratified the patients according to their average signature expression and obtained Kaplan-Meier plots, and estimated the effect on the hazard ratio as a smooth function using quartic penalized splines (Eilers et al, 1996, Statistical Science, 11, 89-121) as implemented in the R package pspline.

### Immunohistochemistry

Immunohistochemistry was performed on paraffin sections using primary antibodies raised against phospho-SMAD3 (Rockland). Briefly, sections were autoclaved 10 minutes in citrate buffer pH 6 previous to incubation with anti phospho-smad 3 (1/100). Secondary biotinylated antibodies (Vector Laboratories Inc) were used at a 1:200 dilution and detected using the Vectastain ABC kit (Vector Laboratories Inc), as recommended by the supplier.

### Orthotopic mouse studies

All experiments with mouse models were approved by the animal care and use committee of the Barcelona Science Park (CEEA-PCB) and the Catalan Government. Cells were injected subcutaneously in 5 to 6 weeks old Swiss nude or NSG mice, (Jackson Labs), which were followed for the periods described. Tumour appearance was assessed by palpation. Five to six weeks old Balb/c nude or NSG mice (Jackson Labs) were used to perform metastasis experiments by intra-splenic injection (Warren et al., 1995, J. Clin.Invest. 95, 1789-1797)

### LY2157299 treatment.

The TGF-beta receptor I kinase antagonist, LY2157299, developed by Eli Lilly (Beight, 2004, Eli Lilly and Co. PCT Int. Appl. In PCT Int. Appl. WO2004048382A1; Bueno et al., 2008;Eur J Cancer 44, 142-150) was synthesized following the procedure described (Mundla, 2007; Eli Lilly and Company. PCT Int. Appl. WO2007018818A1. In, (USA)). TGF-beta receptor type I inhibitor LY2157299 was suspended in a formulation (vehicle) composed of 1% sodium carboxy methylcellulose (NaCMC), 0.4% sodium lauryl sulfate (SLS), 0.05% anti-foam and 0.085% polyvinylpyrrolidone (PVP) in a concentration of 6.67 mg mL⁻¹. Mice were dosed twice a day with 2mg in 300 µL (2 mg) *per os.* starting 3 days before CRC cell lines inoculation. A tenfold higher dosed treatment for 3 days was used to show TGF-beta signalling (p-SMAD2) and target gene mRNA downregulation in pre-established xenografts.

### Bioluminescent imaging and analysis.

Mice were anaesthetized, injected retro-orbitally with D-luciferin (Promega) and imaged for luciferase activity as previously described (Minn et al., 2005; J Clin Invest 115,44-55). Luciferase activity measured immediately after cell injection was used to exclude any mice that were not successfully xenografted and to normalize bioluminescence. For bioluminescent tracking, cell lines were lentivirally infected with a fusion protein reporter construct encoding red fluorescent protein (Cherry) and firefly luciferase.

### EXAMPLE 1

### TGF-beta signaling during CRC progression

TGF-beta signaling during CRC progression was explored. Gene expression profiling of colon tumor samples confirmed elevated levels of *TGFB1, TGFB2* and *TGFB3* mRNAs in a subset of CRCs whereas all adenomas displayed low levels of the three TGF-beta isoforms (Fig. 1). Characteristic features of the adenoma-CRC transition include increased desmoplastic reaction, inflammation and neovascularization, all of which involve several non-cancerous cell types that reside within the tumor stroma.

To identify cell populations targeted by TGF-beta in adenomas and CRCs, phosphorylation of SMAD3 (p-SMAD3) was used as a marker of TGF-beta pathway activation. Immunohistochemistry analysis on clinical material revealed prominent nuclear p-SMAD3 accumulation in epithelial cancer cells in 40% of the adenomas but only in 7% of the CRCs (Fig. 2). This finding may reflect the frequent acquisition of inactivating mutations in TGF-beta signaling pathway components during CRC progression. Additionally, epithelial p-SMAD3+ CRC cells may have impaired TGF-beta transcriptional response due to genetic alterations in SMAD4 (Liu et al., 1997, Genes Dev., 11: 3157-3167 and Alarcon, C., 2009, Cell, 139: 757-769). Concurrent with the loss of epithelial TGF-beta signaling in CRCs, tumor stromal cells displayed high levels of p-SMAD3 (Fig. 2). While the stroma of most adenomas contained few p-SMAD3 highly positive cells and stained weakly overall, a large proportion of CRCs (63%) were characterized by an abundance of stromal cells with strong nuclear p-SMAD3 staining.

Altogether, these observations suggest that a subset of colon tumors undergoes an increase in the expression of TGF-beta at the adenoma-carcinoma transition, and tumor stroma components are the main contributors to this increase. Elevated levels of TGF-beta in CRCs preferentially target tumor-associated stromal cells rather than the cancer cells.

### EXAMPLE 2

### Identification of TGF-beta induced genes that associate with negative outcome in CRC.

As shown in example 1, p-SMAD3 accumulated in the nucleus of different tumor stromal-associated cells. We characterized the stromal cell types stained by p-SMAD3 in CRCs but could not discriminate any obvious cell type-specificity. Rather, p-SMAD3 indiscriminately labelled all major types of stromal cells in CRCs including T-cells, macrophages, endothelial cells and fibroblasts (data not shown).

We determined that TGF-beta-activated stromal genes associated with clinical disease progression. To this end, we used as surrogates the gene expression programmes induced by addition of TGF-beta (TGF-beta response signatures or TBRS) in cultures of normal-tissue-derived T-cells (T-), macrophages (Ma-), endothelial cells (End-) or fibroblasts (Fib-). We used the full set of genes upregulated by TGF-beta signalling in these cell cultures (>2 fold, p<0.05). A representative pooled cohort of 340 CRC cases treated at three different hospitals for which transcriptomic profiles of primary tumors and clinical follow-up were publicly available (see Methods) was interrogated. By Gene Set Enrichment Analysis (GSEA) (Subramanian et al., 2005, *supra*.)*,* we determined that all stromal TBRSs were highly enriched in CRCs compared to adenomas (Fig. 3). These signatures were good predictors of disease relapse in stage-I, -II and -III CRC patients in our cohort of patients and segregated a low-expression patient group with virtually no risk of developing recurrent cancer after therapy (not shown).

To further analyse the cell-type specific expression of each stromal TBRS *in vivo,* we purified by FACS various cell populations from fresh CRC samples and assessed their gene expression profiles (see Materials and Methods). Relative levels of cell type-specific marker genes confirmed the purification of epithelial tumour cells (EPCAM+), Leukocytes (CD45+), endothelial cells (CD31+) and fibroblasts (FAP+) (data not shown).

Remarkably, a comparative analysis revealed a very significant trend towards high expression levels of those genes within the TBRSs that associated positively with cancer relapse (HR>1, p<0.05) in CAFs and in endothelial cells (Fig. 4). In other words, from all the genes in the T-TBRS, Ma-TBRS, End-TBRS and Str-TBRS there is a elevated expression of recurrence-associated TGF-beta induced genes in FAP+ cancer associated fibroblasts and endothelial cells purified from CRC tumours (Fig. 4). Altogether, these data highlight that the response of stromal cells to TGF-beta is an accurate predictor of disease relapse in CRC patients. Whereas high *TGFB* levels activate gene programmes in a wide range of tumour-associated stromal cell types, our data from cell populations purified from CRC patients indicate that CAFs, and to a lower extent endothelial cells, are the main contributors to the association of stromal TGF-beta-driven programmes with poor outcome after therapy.

We next identified the set of genes regulated by TGF-beta in intestinal fibroblasts. Cancer associated Fibroblasts (CAFs), adenoma associated fibroblasts (AAFs) and normal colon mucosa-derived fibroblasts (CCD_co_18) were cultured in the presence or absence of TGF-beta and global gene expression profiles were measured in duplicate using HG-U133 plus 2.0. A TGF-beta response signature (TBRS) was obtained for each fibroblast population by selecting genes with limma p-value < 0.05 and at least two fold up-regulation in TGF-beta treated fibroblasts.

From each individual TGF-beta response signature derived from the above experiment (AAF-TBRS; CRC-TBRS and CCD-TBRS) we selected those genes specifically expressed in cancer associated fibroblasts (FAP+) and/or endothelial cells (CD31+) purified from colorectal cancer patients (Fig. 3; GEO datasets GSE39396 and GSE39395). The gene expression profiles described in GSE39396 and GSE39395 were obtained as described in Materials and Methods and in Calon et al., Cancer Cell 2012, Nov 13;22(5):571-84. FAP + specific genes were defined as those up-regulated with limma p-value < 0.05 (Smyth, 2004, et al., Linear Models for Microarray, User's Guide 2004) in the three comparisons FAP+ *vs* CD31+ (endothelial), FAP+ vs CD45+ (Leukocytes) and FAP+ *vs* EPCAM+ (epithelial) cell populations (Figure 5 and Table I). We defined CD31-specific genes analogously (Figure 5 and Table I). In addition, we substracted all those genes appearing in the publication Calon et al., Cancer Cell 2012, Nov 13;22(5):571-84. This analysis yielded a signature of 73 annotated genes (Figure 5 and Table I, 134 probes; TBRS of this invention) from which we derived specific predictors.

**Table 1: List of genes that are regulated at least two fold by TGF-beta in either stimulated adenoma associated fibroblasts (AAFs), carcinoma associated fibroblasts (CAFs) or normal colon fibroblasts (CCDs) and are specifically expressed in CRC tumours in vivo in either cancer associated fibroblasts (FAP+) or endothelial cells (CD31+) vs the other populations (epithelial EpCAM+ and Leukocytes CD45+) wherein "FC plus TGFbeta in:" refers to the fold change in gene expression level in the indicated TGF-beta stimulated cells and wherein "CAFs vs rest" refers to the specific gene expression in the cancer associated fibroblasts fraction (FAP+) and "End- vs rest" refers to the specific gene expression in endothelial cells (CD31+) from a number of dissagregated human tumours. NA: Not annotated.**

| | | **FC plus TGFbeta in:** | | | **Increased expression in:** | |
|---|---|---|---|---|---|---|
| **Affy ID** | **Gene** | **AAFs** | **CAFs** | **CCDs** | **CAFs vs rest** | **End. vs rest** |
| 205132_at | ACTC1 | 6.8 | 2.9 | 17.1 | ✔ | ✔ |
| 220866_at | ADAMTS6 | 2.0 | 2.3 | 7.0 | | ✔ |
| 206176_at | BMP6 | 3.2 | -1.5 | 6.1 | ✔ | |
| 225990_at | BOC | 2.2 | 1.9 | 1.7 | ✔ | |
| 227883_at | CCDC71L | 1.6 | -1.1 | 2.2 | ✔ | |
| 206932_at | CH25H | 2.1 | 2.3 | 4.6 | ✔ | |
| 203951_at | CNN1 | 2.1 | 1.1 | 2.1 | ✔ | |
| 206818_s_at | CNNM2 | 2.2 | 1.5 | 2.3 | ✔ | |
| 209874_x_at | CNNM2 | 2.0 | 1.3 | 2.0 | ✔ | |
| 1554523_a_at | CNNM2 | 2.0 | 1.3 | 2.2 | ✔ | |
| 211980_at | COL4A1 | 1.50 | 2.07 | 1.62 | ✔ | |
| 205713_s_at | COMP | 3.4 | 2.2 | 2.8 | ✔ | |
| 225129_at | CPNE2 | 1.3 | 1.6 | 2.7 | | ✔ |
| 228759_at | CREB3L2 | 2.3 | 1.6 | 2.9 | ✔ | |
| 1555809_at | CRISPLD2 | 1.9 | -1.3 | 2.3 | ✔ | |
| 221541_at | CRISPLD2 | 1.5 | 1.4 | 2.6 | ✔ | |
| 209283_at | CRYAB | 2.1 | 1.2 | 1.1 | ✔ | |
| 228228_at | DACT3 | 2.2 | 1.2 | 1.2 | ✔ | |
| 1553768_a_at | DCBLD1 | 2.6 | 2.8 | 3.3 | ✔ | |
| 226609_at | DCBLD1 | 3.2 | 4.4 | 4.0 | ✔ | |
| 215328_at | EFR3B | 4.5 | 1.7 | 4.8 | | ✔ |
| 201693_s_at | EGR1 | -1.1 | 1.2 | 3.6 | ✔ | |
| 201694_s_at | EGR1 | -1.4 | 1.4 | 2.2 | ✔ | |
| 227404_s_at | EGR1 | -1.2 | -1.0 | 2.3 | ✔ | |
| 212670_at | ELN | 4.7 | 3.1 | 10.5 | ✔ | |
| 216269_s_at | ELN | 3.1 | 3.3 | 10.2 | ✔ | |
| 212570_at | ENDOD1 | 1.2 | -1.1 | 2.0 | | ✔ |
| 226876_at | FAM101B | 2.0 | 1.5 | 2.3 | | ✔ |
| 226905_at | FAM101B | 1.6 | 1.6 | 2.1 | | ✔ |
| 221766_s_at | FAM46A | 1.6 | 1.3 | 3.3 | ✔ | |
| 224973_at | FAM46A | 1.5 | 1.5 | 2.8 | ✔ | |
| 229737_at | FAM46A | 1.4 | 1.0 | 2.1 | ✔ | |
| 202949_s_at | FHL2 | 1.7 | 2.1 | 2.0 | ✔ | |
| 218980_at | FHOD3 | 1.7 | 1.2 | 2.1 | ✔ | |
| 1554966_a_at | FILIP 1 L | 4.8 | -1.3 | 2.7 | ✔ | |
| 204135_at | FILIP 1 L | 4.3 | -1.1 | 2.4 | ✔ | |
| 213260_at | FOXC1 | 2.0 | -1.1 | -1.6 | | ✔ |
| 239058_at | FOXC2 | 2.1 | 2.3 | 2.5 | ✔ | |
| 225464_at | FRMD6 | 2.3 | 1.2 | 1.5 | ✔ | |
| 225481_at | FRMD6 | 2.3 | 1.4 | 1.5 | ✔ | |
| 224325_at | FZD8 | 22.6 | 7.1 | 18.9 | ✔ | |
| 227405_s_at | FZD8 | 10.4 | 3.3 | 11.2 | ✔ | |
| 202191_s_at | GAS7 | 4.2 | 1.3 | 2.1 | ✔ | |
| 202192_s_at | GAS7 | 5.1 | 1.2 | 2.4 | ✔ | |
| 207704_s_at | GAS7 | 3.3 | 1.4 | 2.6 | ✔ | |
| 210872_x_at | GAS7 | 4.8 | 1.5 | 2.2 | ✔ | |
| 211067_s_at | GAS7 | 5.7 | 1.4 | 2.5 | ✔ | |
| 202269_x_at | GBP1 | 1.2 | 1.4 | 2.1 | ✔ | |
| 231577_s_at | GBP1 | 1.3 | 1.4 | 2.1 | ✔ | |
| 205100_at | GFPT2 | 2.8 | 1.5 | 4.0 | ✔ | |
| 229435_at | GLIS3 | 3.8 | 1.6 | 2.1 | ✔ | |
| 235371_at | GXYLT2 | 2.2 | -1.1 | -1.0 | ✔ | |
| 235733_at | GXYLT2 | 2.0 | 1.1 | -1.3 | ✔ | |
| 219985_at | HS3ST3A1 | 2.0 | 1.5 | 1.4 | ✔ | |
| 209542_x_at | IGF1 | 2.6 | 1.1 | 1.5 | ✔ | |
| 211577_s_at | IGF1 | 2.9 | 1.1 | 1.3 | ✔ | |
| 203233_at | IL4R | 2.3 | 1.1 | 1.2 | | ✔ |
| 1554819_a_at | ITGA11 | 1.7 | 1.0 | 2.2 | ✔ | |
| 229578_at | JPH2 | 2.3 | 2.1 | 3.0 | ✔ | |
| 227230_s_at | KIAA1211 | 1.9 | 1.6 | 2.1 | ✔ | |
| 220002_at | KIF26B | 2.9 | 1.8 | 3.6 | ✔ | |
| 220825_s_at | KIRREL | 2.4 | 1.0 | 2.5 | ✔ | |
| 225303_at | KIRREL | 2.6 | -1.0 | 2.1 | ✔ | |
| 232467_at | KIRREL | 2.2 | 1.6 | 2.1 | ✔ | |
| 204356_at | LIMK1 | 1.9 | 1.2 | 2.1 | ✔ | |
| 229280_s_at | LINC00340 | 2.9 | 1.7 | 2.2 | | ✔ |
| 227889_at | LPCAT2 | 1.5 | 1.3 | 2.3 | | ✔ |
| 229791_at | LPCAT2 | 1.3 | -1.1 | 2.2 | | ✔ |
| 239598_s_at | LPCAT2 | 1.6 | -1.0 | 2.5 | | ✔ |
| 213909_at | LRRC15 | 3.5 | 1.6 | -1.1 | ✔ | |
| 224624_at | LRRC8A | 2.1 | 1.3 | 1.7 | | ✔ |
| 233487_s_at | LRRC8A | 2.0 | 1.4 | 2.1 | | ✔ |
| 204682_at | LTBP2 | 1.3 | 1.2 | 2.0 | ✔ | |
| 207761_s_at | METTL7A | 1.3 | 2.1 | 1.1 | ✔ | |
| 203417_at | MFAP2 | 4.9 | 1.7 | 2.2 | ✔ | |
| 211620_x_at | NA | 2.9 | 1.3 | 5.0 | ✔ | |
| 220990_s_at | NA | 2.0 | 1.4 | 2.6 | ✔ | |
| 222288_at | NA | 2.6 | 1.1 | -1.1 | ✔ | |
| 224917_at | NA | 3.1 | 1.8 | 3.6 | ✔ | |
| 226885_at | NA | 3.1 | 1.3 | 2.4 | ✔ | |
| 229024_at | NA | 2.3 | 1.1 | 1.9 | ✔ | |
| 229130 at | NA | 3.4 | 1.5 | 3.9 | ✔ | |
| 229296_at | NA | 2.0 | -1.0 | 2.3 | ✔ | |
| 231559_at | NA | 2.7 | 1.3 | 2.6 | ✔ | |
| 235629_at | NA | 2.9 | 2.2 | 3.8 | ✔ | |
| 237978_at | NA | 2.1 | -1.1 | 1.5 | ✔ | |
| 238617_at | NA | 2.9 | 2.7 | 3.4 | ✔ | |
| 240135_x_at | NA | 3.3 | -1.0 | 2.5 | ✔ | |
| 241272_at | NA | 3.3 | 1.6 | 4.3 | ✔ | |
| 244788_at | NA | 2.2 | -1.1 | 1.1 | ✔ | |
| 238623_at | NA | 2.0 | 2.2 | 2.1 | ✔ | |
| 1557241_a_at | NA | 1.5 | 1.2 | 2.2 | ✔ | |
| 227107_at | NA | 1.8 | 1.2 | 2.1 | ✔ | |
| 228629_s_at | NA | 1.6 | -1.1 | 2.3 | | ✔ |
| 228756_at | NA | 1.1 | -1.1 | 2.5 | ✔ | |
| 233223_at | NA | 1.9 | 1.1 | 2.8 | | ✔ |
| 233335_at | NA | 2.0 | 1.1 | 2.5 | ✔ | |
| 236251_at | NA | 1.8 | 1.5 | 2.1 | | ✔ |
| 238933_at | NA | -1.1 | 1.3 | 2.7 | ✔ | |
| 239809_at | NA | 1.5 | -1.0 | 2.3 | | ✔ |
| 240960_at | NA | 1.7 | -1.1 | 2.4 | | ✔ |
| 202237_at | NNMT | 2.4 | -1.0 | 1.9 | ✔ | |
| 202238_s_at | NNMT | 3.0 | -1.1 | 2.3 | ✔ | |
| 223601_at | OLFM2 | 2.3 | 1.8 | 3.9 | ✔ | |
| 228703_at | P4HA3 | 1.7 | 1.4 | 2.3 | ✔ | |
| 219093_at | PID1 | 1.3 | 2.2 | 2.3 | ✔ | |
| 236044_at | PPAPDC1A | 1.8 | 1.5 | 2.9 | ✔ | |
| 201958_s_at | PPP1R12B | 2.1 | -1.1 | 4.5 | ✔ | |
| 201957_at | PPP1R12B | 1.9 | 1.1 | 3.7 | ✔ | |
| 204284_at | PPP1R3C | 1.7 | 2.8 | 2.4 | ✔ | |
| 229441_at | PRSS23 | 2.0 | 1.1 | 2.3 | | ✔ |
| 223634_at | RASD2 | 3.4 | 2.0 | 2.6 | ✔ | |
| 1553721_at | RNF152 | 1.5 | 2.3 | 5.4 | ✔ | |
| 204642_at | S1PR1 | 2.3 | 1.1 | -1.2 | | ✔ |
| 228407_at | SCUBE3 | 2.2 | 1.1 | 1.4 | ✔ | |
| 232894_at | SEC14L2 | 2.1 | 1.1 | 2.4 | ✔ | |
| 212190_at | SERPINE2 | 2.3 | 1.4 | 2.6 | ✔ | |
| 227487_s_at | SERPINE2 | 2.0 | 1.2 | 2.6 | ✔ | |
| 201739_at | SGK1 | 3.7 | 2.9 | 3.1 | | ✔ |
| 207661_s_at | SH3PXD2A | 2.8 | 1.8 | 2.3 | ✔ | |
| 213252_at | SH3PXD2A | 3.3 | 1.4 | 3.2 | ✔ | |
| 224817_at | SH3PXD2A | 2.5 | 1.7 | 2.2 | ✔ | |
| 230493_at | SHISA2 | 1.7 | 6.1 | 1.2 | ✔ | |
| 207390_s_at | SMTN | 1.4 | -1.0 | 2.1 | ✔ | |
| 209427 at | SMTN | 1.5 | 1.1 | 2.1 | ✔ | |
| 221489_s_at | SPRY4 | -1.1 | 1.2 | 2.6 | | ✔ |
| 1555875_at | SRGAP1 | 2.2 | 1.6 | 2.3 | ✔ | |
| 1569269_s_at | SRGAP1 | 2.5 | 1.3 | 2.9 | ✔ | |
| 225720_at | SYNPO2 | 1.2 | 1.3 | 4.8 | ✔ | |
| 225721_at | SYNPO2 | 1.1 | 1.3 | 3.3 | ✔ | |
| 225894 at | SYNPO2 | 1.4 | 1.3 | 4.3 | ✔ | |
| 225895_at | SYNPO2 | 1.1 | 1.3 | 3.4 | ✔ | |
| 244108_at | SYNPO2 | 1.1 | 1.2 | 3.3 | ✔ | |
| 217287_s_at | TRPC6 | 1.0 | 1.2 | 2.8 | ✔ | |
| 209825_s_at | UCK2 | 2.0 | 1.7 | 2.5 | | ✔ |

### EXAMPLE 3

### Identification of mini-signatures capable of predicting outcome of CRC

The TBRS of this invention was further analyzed as described in Materials and Methods in order to identify the minimal subset of genes that provides good prediction of disease-free survival. A subset of four genes (minisignature) formed by the FAM46A, FHL2, FOXC2 and COL4A1 genes was identified which associated with time to recurrence in a statistically significant manner in all patients analysed as well as in patients from stage II and stage III subgroups. Cox proportional hazards multivariate analysis (Table 2) demonstrated that the 4 genes minisignature expression is an independent predictor of cancer recurrence from the AJCC staging system for the identification of CRC patients that remain disease-free upon therapy.

**Table 2. Multivariate analysis using Cox Proportional Hazards Model to assess dependency of the expression signature comprised by FAM46A, FHL2, FOXC2, and COL4A1 and AJCC staging in the prediction of cancer relapse. This analysis demonstrated that the expression of the abovementioned four genes is an independent predictor of cancer recurrence from the AJCC staging system in the identification of CRC patients at high or low risk to develop recurrent disease.**

| **Multivariate Cox Analysis** | **HR** | **95% CI** | **P value** |
|---|---|---|---|
| **4 genes predictor (+1 SD) FAM46A, FHL2, FOXC2, COL4A1** | **1.81** | **1.5 - 2.2** | **<0.0001** |

| **Average Signature Expression** | | | |
|---|---|---|---|
| **Medium vs. Low** | **3.13** | **-2.6 - 26.1** | **0.2308** |
| **High vs. Low** | **18.73** | **2.6 - 136.6** | **<0.0001** |
| **High vs. Medium** | **5.98** | **2.5 - 14.0** | **<0.0001** |

| **AJCC Stage** | | | |
|---|---|---|---|
| **Stage 2 vs. 1** | **2.84** | **-1.5 - 12.5** | **0.1189** |
| **Stage 3 vs. 1** | **7.90** | **1.9 - 33.0** | **<0.0001** |
| **Stage 3 vs. 2** | **2.78** | **1.5 - 5.2** | **0.0006** |

In addition, as shown in Figure 6, the slope of the average expression of the four gene signature shows an approximate incremental linear relationship with the risk of recurrence. For every increase in overall expression (+1 Standard Deviation) of the four genes the risk of cancer recurrence augmented by 93% (p<0.0001, HR per +1 Standard Deviation = 1.93). Figure 7 shows Kaplan Meier curves wherein survival of patients is plotted depending on the average expression of the FAM46A, FHL2, FOXC2 and COL4A1 genes in all patients (panel A), in stage II patients (panel B) or in stage III patients (panel C).

When the patients were stratified according to the stage of the tumor, two additional signatures were identified that provided statistically significant prediction of time to recurrence. In stage II patients, the expression signature formed by FAM46A, FHL2, FOXC2, COL4A1 and FRMD6 allowed prediction of time to recurrence with a p<0.05 (Fig. 8A). In stage III patients, the expression signature formed by FAM46A, FHL2, FOXC2, COL4A, SPRY4 and DACT3 allowed prediction of time to recurrence with a p<0.0001 (Fig. 10A). In both cases, the signature expression displayed an incremental and approximately linear effect on the risk of recurrence (Fig. 9B and 10B).

Since tumour stage is an information available to the oncologist at the time of diagnosis, the prognostic value of 4 genes predictor (FAM46A, FHL2, FOXC2, and COL4A1) or the 5 predictor genes specific for stage II patients (FAM46A, FHL2, FOXC2, COL4A1 and FRMD6) or the 6 predictor genes specific for stage III patients (FAM46A, FHL2, FOXC2, COL4A, SPRY4 and DACT3) in combination with staging was evaluated. This allows for the identification of a group of patients at very low risk of disease recurrence (local or distant) in both groups. For example, using the 6 gene predictor, a SCAD coefficient below or equal to -1 identifies a total of 38 patients that will not recur whereas only 1 patient with a SCAD coefficient below -1 experience recurrence.

| **CORE (4 genes)** | **[≤-1]** | **[-1,0]** | **[>0]** |
|---|---|---|---|
| **Stage I** | | | |
| No recurrent | 10 | 17 | 16 |
| Recurrent | 0 | 0 | 2 |

| **Stage II** | | | |
|---|---|---|---|
| No recurrent | 9 | 40 | 46 |
| Recurrent | 0 | 1 | 13 |

| **Stage III** | | | |
|---|---|---|---|
| No recurrent | 18 | 32 | 23 |
| Recurrent | 1 | 5 | 31 |

| **Stage II (5 genes)** | **[≤-1]** | **[-1,0]** | **[>0]** |
|---|---|---|---|
| **Stage I** | | | |
| No recurrent | 7 | 23 | 13 |
| Recurrent | 0 | 0 | 2 |

| **Stage II** | | | |
|---|---|---|---|
| No recurrent | 13 | 35 | 47 |
| Recurrent | 0 | 3 | 11 |

| **Stage III** | | | |
|---|---|---|---|
| No recurrent | 16 | 32 | 25 |
| Recurrent | 1 | 6 | 30 |

| **Stage III (6 genes)** | **[≤-1]** | **[-1,0]** | **[>0]** |
|---|---|---|---|
| **Stage I** | | | |
| No recurrent | 12 | 18 | 13 |
| Recurrent | 0 | 0 | 2 |

| **Stage II** | | | |
|---|---|---|---|
| No recurrent | 10 | 39 | 46 |
| Recurrent | 0 | 4 | 10 |

| **Stage III** | | | |
|---|---|---|---|
| No recurrent | 16 | 33 | 24 |
| Recurrent | 1 | 4 | 32 |

In addition, we studied the association of the 5 genes stage II predictor in two independent cohorts of stage II patients GSE 33113 (Fig. 10) and GSE 26906 (Fig. 11). Figure 10 shows Kaplan Meier curves wherein survival of patients is plotted depending on the average expression of the FAM46A, FHL2, FOXC2, COL4A1 and FRMD6 genes in all patients (Fig. 10A). The slope of the average expression of the five gene signature in this patient cohort shows an approximate incremental linear relationship with the risk of recurrence. For every increase in overall expression (+1 Standard Deviation) of the four genes the risk of cancer recurrence augmented by 104% (p=0.0008, HR per +1 Standard Deviation = 2.04). Figure 11 shows Box Plots illustrating the average signature expression in disease free patients and in patients experiencing metastatic disease from the GSE 26906 patient cohort. This cohort does not contain data on clinical follow up.

### EXAMPLE 4

### Pharmacological inhibition of stromal TGF-beta signalling blocks metastasis initiation

We used tumours generated in mice derived from human colon cancer stem cells (CoCSCs) to demonstrate that pharmacological inhibition of stromal TGF-beta signalling blocks metastasis initiation. The purification of Colon Cancer CoCSCs from CRC biopsies is described elsewhere (Merlos-Suarez et al., 2011, Cell Stem Cell 8, 511-524.). We isolated CoCSCs from the primary tumour of a Stage IV CRC patient and cultured them as epithelial tumour organoids as described in (Jung et al., 2011; Nat Med 17, 1225-1227; Sato et al., 2011; Gastroenterology 141, 1762-1772). Genomic analysis of the tumour organoids revealed that the two *TGFBR2* alleles were inactivated by mutations in this patient (data not shown). Indeed, treatment with TGFBR1-specific inhibitor LY2157299 (Bueno et al., 2008, Eur. J. Cancer 44, 142-150) or addition of active TGF-beta did not modify *in vitro* growth rates, morphology or organoid forming activity of this CoCSC-derived culture (data not shown). When injected in immunodeficient mice, they generated tumours with abundant p-SMAD2+ stromal cells, implying that this primary CoCSCs elicited a TGF-beta response in the tumour microenvironment (data not shown). Feeding mice bearing macroscopic tumours from CoCSCs-derived cultures with the TGF-beta inhibitor LY2157299 conferred resistance to the formation of subcutaneous tumours by primary CoCSC-derived cells (Fig. 12A). Remarkably, this TGF-beta inhibitor regime also reduced formation of liver metastasis by CoCSCs inoculated via the spleen (Fig. 12B). Kinetics of metastatic colonization showed that LY2157299 reduced the number of cells that engrafted the liver immediately after inoculation (Fig. 12B, - inset).

## Claims

1. A method for predicting the outcome of a patient suffering colorectal cancer, for selecting a suitable treatment in a patient suffering colorectal cancer or for selecting a patient which is likely to benefit from adjuvant therapy after surgical resection of colorectal cancer comprising the determination of the expression levels of the FAM46A, FHL2, FOXC2 and COL4A1 genes in a sample from said patient,
wherein an increased expression level of said genes with respect to a reference value for said genes is indicative of an increased likelihood of a negative outcome of the patient, that the patient is candidate for receiving therapy after surgical treatment or that the patient is likely to benefit from therapy after surgical treatment or
wherein a decreased expression level of said genes with respect to reference values for said genes is indicative of an increased likelihood of a positive outcome of the patient, that the patient is not candidate for receiving therapy after surgical treatment or that the patient is unlikely to benefit from therapy after surgical treatment.

2. Method according to claim 1, additionally comprising the determination of the expression levels of the FRMD6 gene, wherein the patient is a patient suffering from stage II colorectal cancer, and
wherein increased expression levels of FRMD6 with respect to a reference value for said gene is indicative of an increased likelihood of negative outcome, that the patient is candidate for receiving therapy after surgical treatment or that the patient is likely to benefit from therapy after surgical treatment or
wherein decreased expression levels of FRMD6 with respect to a reference value for said gene is indicative of an increased likelihood of positive outcome, that the patient is not a candidate for receiving therapy after surgical treatment or that the patient is unlikely to benefit from therapy after surgical treatment.

3. Method according to claim 1, additionally comprising the determination of the expression levels of the SPRY4 and DACT3 genes and wherein the patient is a patient suffering from stage III colorectal cancer and
wherein increased expression levels of SPRY4 and DACT3 with respect to a reference value for said genes is indicative of an increased likelihood of negative outcome, that the patient is candidate for receiving therapy after surgical treatment or that the patient is likely to benefit from therapy after surgical treatment or
wherein decreased expression levels of SPRY4 and DACT3 with respect to a reference value for said genes is indicative of an increased likelihood of positive outcome, that the patient is not a candidate for receiving therapy after surgical treatment or that the patient is unlikely to benefit from therapy after surgical treatment.

4. Method according to any of claims 1 to 3, additionally comprising the determination of the expression levels of one or more genes selected from the group consisting of ACTC1, BOC, CNN1, COMP, CRISPLD2, CRYAB, FAM101B, FILIP1L, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2 genes
wherein increased expression levels of said one or more genes with respect to a reference value for said one or more genes is indicative of an increased likelihood of negative outcome, that the patient is candidate for receiving therapy after surgical treatment or that the patient is likely to benefit from therapy after surgical treatment or
wherein decreased expression levels of one or more genes with respect to a reference value for one or more genes is indicative of an increased likelihood of positive outcome, that the patient is not a candidate for receiving therapy after surgical treatment or that the patient is unlikely to benefit from therapy after surgical treatment.

5. Method according to claim 4 further comprising the determination of the expression levels of one or more genes selected from the group consisting of ADAMTS6, BMP6, CCDC71L, CH25H, CNNM2, CPNE2, CREB3L2, DCBLD1, EFR3B, EGR1, ELN, ENDOD1, FHOD3, FOXC1, FZD8, GBP1, GXYLT2, IGF1, IL4R, ITGA11, JPH2, KIAA1211, KIF26B, LIMK1, LINC00340, LPCAT2, LRRC8A, METTL7A, OLFM2, PID1, PPP1R12B, PRSS23, RASD2, RNF152, SCUBE3, SEC14L2, SERPINE2, SGK1, SH3PXD2A, SHISA2, SMTN, SRGAP1, TRPC6 and UCK2 and of the genes which hybridize specifically with the probes having the sequences SEQ ID NO:1 to 26
wherein increased expression levels of said one or more genes with respect to reference values for said one or more genes is indicative of an increased likelihood of a negative outcome, that the patient is candidate for receiving therapy after surgical treatment or that the patient is likely to benefit from therapy after surgical treatment or
wherein decreased expression levels of one or more of said genes with respect to reference values for one or more of said genes is indicative of an increased likelihood of a positive outcome, that the patient is not a candidate for receiving therapy after surgical treatment or that the patient is unlikely to benefit from therapy after surgical treatment

6. Method according to any of claims 1 to 5 wherein the tumor stage in the patient is additionally determined and
wherein a high tumor stage is indicative of an increased likelihood of a negative outcome, that the patient is candidate for receiving therapy after surgical treatment or that the patient is likely to benefit from therapy after surgical treatment or
wherein a low tumor stage is indicative of an increased likelihood of a positive outcome, that the patient is not a candidate for receiving therapy after surgical treatment or that the patient is unlikely to benefit from therapy after surgical treatment.

7. Method according to any of claims 1 to 6 wherein the therapy is selected from the group consisting of chemotherapy, radiotherapy and/or a therapy comprising a TGF-beta inhibitor.

8. The method according to claim 7, wherein the TGF-beta inhibitor is a compound as defined in Table 1 or wherein the TGF-beta inhibitor is a compound having the structure 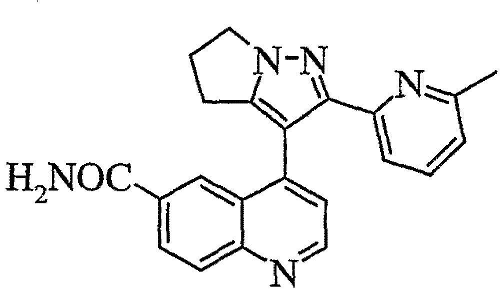 or any polymorph, solvate or hydrate thereof.

9. Method according to any of claims 1 to 8 wherein the outcome to be predicted is either recurrence or development of metastasis, preferably liver metastasis.

10. Method according to any of claims 1 to 9 wherein the sample is selected from the group consisting of a tumor biopsy or a biofluid.

11. A chemotherapeutic compound and/or a TGF-beta inhibitor, for use in the treatment of colorectal cancer in a patient after surgical treatment of the cancer, wherein the patient has been selected by a method according to any of claims 1 to 10.

12. The chemotherapeutic compound and/or the TGF-beta inhibitor for use according to claim 11, wherein the TGF-beta inhibitor is a compound as defined in Table 1 or wherein the TGF-beta inhibitor is a compound having the structure 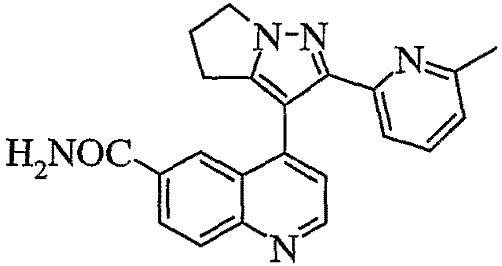 or any polymorph, solvate or hydrate thereof.

13. A kit comprising reagents adequate for determining the expression levels of the FAM46A, FHL2, FOXC2 and COL4A1 genes and, optionally, reagents for the determination of the expression levels of one or more housekeeping genes, wherein the reagents adequate for the determination of the expression levels of the FAM46A, FHL2, FOXC2 and COL4A1 genes comprise at least 10% of the total amount of reagents adequate for the determination of the expression levels of genes forming the kit, wherein the reagents are selected from the group consisting of primers, probes, aptamers and antibodies.

14. A kit according to claim 13, further comprising reagents adequate for the determination of the expression levels of the FRMD6 gene or reagents adequate for the determination of the expression levels of the SPRY4 and DACT3 genes, wherein the reagents are selected from the group consisting of primers, probes, aptamers and antibodies.

15. A kit according to any of claims 13 or 14, further comprising reagents adequate for the determination of the expression levels of one or more genes selected from the group consisting of ACTC1, BOC, CNN1, COMP, CRISPLD2, CRYAB, FAM101B, FILIP1L, GAS7, GFPT2, GLIS3, HS3ST3A1, KIRREL, LRRC15, LTBP2, MFAP2, NNMT, P4HA3, PPAPDC1A, PPP1R3C, S1PR1 and SYNPO2, wherein the reagents are selected from the group consisting of primers, probes, aptamers and antibodies.

16. A kit according to any of claims 13 to 15, further comprising reagents adequate for the determination of the expression levels of one or more genes selected from the group consisting of ADAMTS6, BMP6, CCDC71L, CH25H, CNNM2, CPNE2, CREB3L2, DCBLD1, EFR3B, EGR1, ELN, ENDOD1, FHOD3, FOXC1, FZD8, GBP1, GXYLT2, IGF1, IL4R, ITGA11, JPH2, KIAA1211, KIF26B, LIMK1, LINC00340, LPCAT2, LRRC8A, METTL7A, OLFM2, PID1, PPP1R12B, PRSS23, RASD2, RNF152, SCUBE3, SEC14L2, SERPINE2, SGK1, SH3PXD2A, SHISA2, SMTN, SRGAP1, TRPC6 and UCK2 and of the genes which hybridize specifically with the probes having the sequences SEQ ID NO:1 to 26, wherein the reagents are selected from the group consisting of primers, probes, aptamers and antibodies.

17. Use of a kit according to any of claims 13 to 16 for predicting the outcome of a patient suffering colorectal cancer, for selecting a suitable treatment for patient suffering colorectal cancer or for selecting a patient which is likely to benefit from adjuvant therapy after surgical resection of colorectal cancer.

## Patentansprüche

1. Verfahren zur Vorhersage des Ergebnisses von einem Patienten, der an Darmkrebs leidet, für die Auswahl einer geeigneten Behandlung eines Patienten, der an Darmkrebs leidet, oder für die Auswahl von einem Patienten der nach einer chirurgischen Darmkrebs Resektion wahrscheinlich von einer adjuvanten Therapie profitieren würde, umfassend die Bestimmung der Expressionsniveaus der Gene FAM46A, FHL2, FOXC2 und COL4A1 in einer Probe eines Patienten,
wobei ein erhöhtes Expressionsniveau von diesem Gene in Bezug auf einen Referenzwert für diese Gene indikativ für eine erhöhte Wahrscheinlichkeit eines negativen Ergebnisses des Patienten ist, dass der Patient ein Kandidat für den Erhalt einer Therapie nach einer chirurgischen Behandlung ist oder, dass der Patient wahrscheinlich von einer Therapie nach einer chirurgischen Behandlung profitiert oder
wobei ein vermindertes Expressionsniveau von diesem Gene in Bezug auf Referenzwerte für diese Gene indikativ für eine erhöhte Wahrscheinlichkeit eines positiven Ergebnisses des Patienten ist, dass der Patient kein Kandidat für den Erhalt einer Therapie nach einer chirurgischen Behandlung ist oder, dass der Patient unwahrscheinlich von einer Therapie nach einer chirurgischen Behandlung profitiert.

2. Verfahren nach Anspruch 1, welches zusätzlich die Bestimmung des Expressionsniveaus des FRMD6-Gens umfasst, wobei der Patient ein Patient ist der an Stadium II Darmkrebs leidet, und
wobei erhöhte Expressionsniveaus von FRMD6 in Bezug auf einen Referenzwert für dieses Gen indikativ für eine erhöhte Wahrscheinlichkeit eines negativen Ergebnisses ist, dass der Patient ein Kandidat für den Erhalt einer Therapie nach einer chirurgischen Behandlung ist oder, dass der Patient wahrscheinlich von einer Therapie nach einer chirurgischen Behandlung profitiert oder
wobei verminderte Expressionsniveaus von FRMD6 in Bezug auf einen Referenzwert für dieses Gen indikativ für eine erhöhte Wahrscheinlichkeit eines positiven Ergebnisses ist, dass der Patient kein Kandidat für den Erhalt einer Therapie einer chirurgischen Behandlung ist oder, dass der Patient unwahrscheinlich von einer Therapie nach einer chirurgischen Behandlung profitiert.

3. Verfahren nach Anspruch 1, welches zusätzlich die Bestimmung der Expressionsniveaus der SPRY4 und DACT3 Gene umfasst, und wobei der Patient ein Patient ist der an Stadium III Darmkrebs leidet und
wobei erhöhte Expressionsniveaus von SPRY4 und DACT3 in Bezug auf einen Referenzwert für diese Gene indikativ für eine erhöhte Wahrscheinlichkeit eines negativen Ergebnisses ist, dass der Patient ein Kandidat für den Erhalt einer Therapie nach einer chirurgischen Behandlung ist oder, dass der Patient wahrscheinlich von einer Therapie nach einer chirurgischen Behandlung profitiert oder
wobei verminderte Expressionsniveaus von SPRY4 und DACT3 in Bezug auf einen Referenzwert für diese Gene indikativ für eine erhöhte Wahrscheinlichkeit eines positiven Ergebnisses ist, dass der Patient kein Kandidat für den Erhalt einer Therapie nach einer chirurgischen Behandlung ist oder, dass der Patient unwahrscheinlich von einer Therapie nach einer chirurgischen Behandlung profitiert.

4. Verfahren nach einem der Ansprüche 1 bis 3, welches zusätzlich die Bestimmung des Expressionsniveaus von einem oder mehreren Gen(en) umfasst, ausgewählt aus der Gruppe bestehend aus ACTC1, BOC, CNN1, COMP, CRISPLD2, CRYAB, FAM101B, FILIP1L, GAS7, GFPT2, GLIS3, HS3ST3A1, KIRREL, LRRC15, LTBP2, MFAP2, NNMT, P4HA3, PPAPDC1A, PPP1R3C, S1PR1 und SYNP02 Genen
wobei erhöhte Expressionsniveaus von dem einen oder mehreren Gen(en) in Bezug auf einen Referenzwert von dem einen oder mehreren Gen(en) indikativ für eine erhöhte Wahrscheinlichkeit eines negativen Ergebnisses ist, dass der Patient ein Kandidat für den Erhalt einer Therapie nach einer chirurgischen Behandlung ist oder, dass der Patient wahrscheinlich von einer Therapie nach einer chirurgischen Behandlung profitiert oder
wobei verminderte Expressionsniveaus von dem einen oder mehreren Gen(en) in Bezug auf einen Referenzwert von dem einen oder mehreren Gen(en) indikativ für eine erhöhte Wahrscheinlichkeit eines positiven Ergebnisses ist, dass der Patient kein Kandidat für den Erhalt einer Therapie nach einer chirurgischen Behandlung ist oder, dass der Patient unwahrscheinlich von einer Therapie nach einer chirurgischen Behandlung profitiert.

5. Verfahren nach Anspruch 4, ferner umfasst die Bestimmung des Expressionsniveaus von einem oder mehreren Gen(en), ausgewählt aus der Gruppe bestehend aus ADAMTS6, BMP6, CCDC71L, CH25H, CNNM2, CPNE2, CREB3L2, DCBLD1, EFR3B, EGR1, ELN, ENDOD1, FHOD3, FOXC1, FZD8, GBP1, GXYLT2, IGF1, IL4R, ITGA11, JPH2, KIAA1211, KIF26B, LIMK1, LINC00340, LPCAT2, LRRC8A, METTL7A, OLFM2, PID1, PPP1R12B, PRSS23, RASD2, RNF152, SCUBE3, SEC14L2, SERPINE2, SGK1, SH3PXD2A, SHISA2, SMTN, SRGAP1, TRPC6 und UCK2 und von den Genen die spezifisch mit den Sonden der Sequenzen SEQ ID NO: 1 bis 26 hybridisieren
wobei erhöhte Expressionsniveaus von dem einen oder mehreren Gen(en) in Bezug auf Referenzwerte von dem einen oder mehreren Gen(en) indikativ für eine erhöhte Wahrscheinlichkeit eines negativen Ergebnisses ist, dass der Patient ein Kandidat für den Erhalt einer Therapie nach einer chirurgischen Behandlung ist oder, dass der Patient wahrscheinlich von einer Therapie nach einer chirurgischen Behandlung profitiert oder
wobei verminderte Expressionsniveaus von dem einen oder mehreren Gen(en) in Bezug auf Referenzwerte von dem einen oder mehreren Gen(en) indikativ für eine erhöhte Wahrscheinlichkeit eines positiven Ergebnisses ist, dass der Patient kein Kandidat für den Erhalt einer Therapie nach einer chirurgischen Behandlung ist oder, dass der Patient unwahrscheinlich von einer Therapie nach einer chirurgischen Behandlung profitiert.

6. Verfahren nach einem der Ansprüche 1 bis 5, wobei das Tumorstadium im Patienten zusätzlich ermittelt wird und
wobei ein hohes Tumorstadium indikativ für eine erhöhte Wahrscheinlichkeit eines negativen Ergebnisses ist, dass der Patient ein Kandidat für den Erhalt einer Therapie nach einer chirurgischen Behandlung ist oder, dass der Patient wahrscheinlich von einer Therapie nach einer chirurgischen Behandlung profitiert oder
wobei ein niedriges Tumorstadium indikativ für eine erhöhte Wahrscheinlichkeit eines positiven Ergebnisses ist, dass der Patient kein Kandidat für den Erhalt einer Therapie nach einer chirurgischen Behandlung ist oder, dass der Patient unwahrscheinlich von einer Therapie nach einer chirurgischen Behandlung profitiert.

7. Verfahren nach einem der Ansprüche 1 bis 6, wobei die Therapie ausgewählt ist aus der Gruppe bestehend aus Chemotherapie, Strahlentherapie und/oder einer Therapie die eine TGF-beta-Hemmer umfasst.

8. Verfahren nach Anspruch 7, wobei der TGF-beta-Inhibitor eine Verbindung ist wie in Tabelle 1 definiert oder wobei der TGF-beta-Inhibitor eine Verbindung mit der Strukturformel 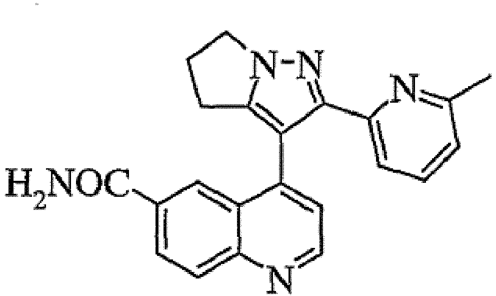 oder ein Polymorph, Solvat oder Hydrat davon ist.

9. Verfahren nach einem der Ansprüche 1 bis 8, wobei das vorherzusagende Ergebnis entweder das Wiederauftreten oder die Entwicklung von Metastasen ist, vorzugsweise Lebermetastasen.

10. Verfahren nach einem der Ansprüche 1 bis 9, wobei die Probe ausgewählt ist aus der Gruppe bestehend aus einer Tumorbiopsie oder einem Biofluid.

11. Chemotherapeutische Verbindung und/oder ein TGF-beta-Inhibitor, zur Verwendung in der Behandlung von Darmkrebs in einem Patienten nach einer chirurgischen Behandlung des Krebses, wobei der Patient durch ein Verfahren nach einem der Ansprüche 1 bis 10 ausgewählt wurde.

12. Chemotherapeutikum und/oder der TGF-beta-Inhibitor zur Verwendung nach Anspruch 11, wobei der TGF-beta-Inhibitor eine Verbindung ist wie in Tabelle 1 definiert oder wobei der TGF-beta-Inhibitor eine Verbindung mit der 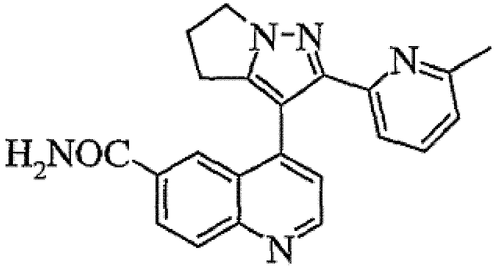 Strukturformel
oder ein Polymorph, Solvat oder Hydrat davon ist.

13. Kit umfassend die Reagenzien, die für die Bestimmung der Expressionsniveaus der FAM46A, FHL2, FOXC2 und COL4A1 Gene geeignet sind, und optional Reagenzien die für die Bestimmung der Expressionsniveaus von einem oder mehrerer Haushaltsgen(en) geeignet ist/sind, wobei die Reagenzien die für die Bestimmung der Expressionsniveaus der FAM46A, FHL2, FOXC2 und COL4A1 Gene geeignet sind, mindestens 10% der Gesamtmenge an Reagenzien umfasst, die für die Bestimmung der Expressionsniveaus der Gene geeignet sind die das Kit bilden, wobei die Reagenzien ausgewählt sind aus der Gruppe bestehend aus Primern, Sonden, Aptameren und Antikörpern.

14. Kit nach Anspruch 13, ferner umfassend Reagenzien, die für die Bestimmung des Expressionsniveaus von dem FRMD6-Gen geeignet sind, oder Reagenzien die für die Bestimmung der Expressionsniveaus der Gene SPRY4 und DACT3 geeignet sind, wobei die Reagenzien ausgewählt sind aus der Gruppe bestehend aus Primern, Sonden, Aptamere und Antikörper.

15. Kit nach einem der Ansprüche 13 oder 14, ferner umfassend Reagenzien, die für die Bestimmung des Expressionsniveaus von einem oder mehreren Gen(en), ausgewählt aus der Gruppe bestehend aus ACTC1, BOC, CNN1, COMP, CRISPLD2, CRYAB, FAM101B, FILIP1L, GAS7, GFPT2, GLIS3, HS3ST3A1, KIRREL, LRRC15, LTBP2, MFAP2, NNMT, P4HA3, PPAPDC1A, PPP1R3C, S1PR1 und SYNP02 geeignet ist/sind, wobei die Reagenzien ausgewählt sind aus der Gruppe bestehend aus Primern, Sonden, Aptamere und Antikörper.

16. Kit nach einem der Ansprüche 13 bis 15, ferner umfassend Reagenzien, die für die Bestimmung des Expressionsniveaus von einem oder mehreren Gen(en), ausgewählt aus der Gruppe bestehend aus ADAMTS6, BMP6, CCDC71L, CH25H, CNNM2, CPNE2, CREB3L2, DCBLD1, EFR3B, EGR1, ELN, ENDOD1, FHOD3, FOXC1, FZD8, GBP1, GXYLT2, IGF1, IL4R, ITGA11, JPH2, KIAA1211, KIF26B, LIMK1, LINC00340, LPCAT2, LRRC8A, METTL7A, OLFM2, PID1, PPP1R12B, PRSS23, RASD2, RNF152, SCUBE3, SEC14L2, SERPINE2, SGK1, SH3PXD2A, SHISA2, SMTN, SRGAP1, TRPC6 und UCK2 geeignet ist/sind, und von den Genen die spezifisch mit den Sonden mit den Sequenzen SEQ ID NO: 1 bis 26 hybridisieren, wobei die Reagenzien ausgewählt sind aus der Gruppe bestehend aus Primern, Sonden, Aptamere und Antikörper.

17. Verwendung eines Kits nach einem der Ansprüche 13 bis 16, zur Vorhersage des Ergebnisses eines Patienten der unter Darmkrebs leidet, für die Auswahl einer geeignete Behandlung für einen Patient der unter Darmkrebs leidet, oder für die Auswahl eines Patienten der wahrscheinlich von einer adjuvanten Therapie nach einer chirurgischen Darmkrebs Resektion profitiert.

## Revendications

1. Procédé pour prédire l'issue d'un patient souffrant de cancer colorectal, permettant de choisir un traitement approprié chez un patient souffrant de cancer colorectal ou permettant de choisir un patient qui est susceptible de bénéficier d'un traitement adjuvant après résection chirurgicale d'un cancer colorectal comprenant la détermination des niveaux d'expression des gènes FAM46A, FHL2, FOXC2 et COL4A1 dans un échantillon provenant dudit patient,
où un niveau d'expression accru desdits gènes par rapport à une valeur de référence pour lesdits gènes est indicatif d'une probabilité accrue d'une issue négative pour le patient, que le patient est candidat pour recevoir un traitement après traitement chirurgical ou que le patient est susceptible de bénéficier d'un traitement après traitement chirurgical ou
où un niveau d'expression réduit desdits gènes par rapport à des valeurs de référence pour lesdits gènes est indicatif d'une probabilité accrue d'une issue positive pour le patient, que le patient n'est pas candidat pour recevoir un traitement après traitement chirurgical ou que le patient est peu susceptible de bénéficier d'un traitement après traitement chirurgical.

2. Procédé selon la revendication 1, comprenant en outre la détermination des niveaux d'expression du gène FRMD6, où le patient est un patient souffrant de cancer colorectal de stade II, et
où des niveaux d'expression accrus de FRMD6 par rapport à une valeur de référence pour ledit gène est indicatif d'une probabilité accrue d'issue négative, que le patient est candidat pour recevoir un traitement après traitement chirurgical ou que le patient est susceptible de bénéficier d'un traitement après traitement chirurgical ou
où des niveaux d'expression réduits de FRMD6 par rapport à une valeur de référence pour ledit gène est indicatif d'une probabilité accrue d'issue positive, que le patient n'est pas un candidat pour recevoir un traitement après traitement chirurgical ou que le patient est peu susceptible de bénéficier d'un traitement après traitement chirurgical.

3. Procédé selon la revendication 1, comprenant en outre la détermination des niveaux d'expression des gènes SPRY4 et DACT3 et où le patient est un patient souffrant de cancer colorectal de stade III et
où des niveaux d'expression accrus de SPRY4 et de DACT3 par rapport à une valeur de référence pour lesdits gènes est indicatif d'une probabilité accrue d'issue négative, que le patient est candidat pour recevoir un traitement après traitement chirurgical ou que le patient est susceptible de bénéficier d'un traitement après traitement chirurgical ou
où des niveaux d'expression réduits de SPRY4 et de DACT3 par rapport à une valeur de référence pour lesdits gènes est indicatif d'une probabilité accrue d'issue positive, que le patient n'est pas un candidat pour recevoir un traitement après traitement chirurgical ou que le patient est peu susceptible de bénéficier d'un traitement après traitement chirurgical.

4. Procédé selon l'une quelconque des revendications 1 à 3, comprenant en outre la détermination des niveaux d'expression d'un ou plusieurs gènes choisis dans le groupe constitué par les gènes ACTC1, BOC, CNN1, COMP, CRISPLD2, CRYAB, FAM101B, FILIP1L, GAS7, GFPT2, GLIS3, HS3ST3A1, KIRREL, LRRC15, LTBP2, MFAP2, NNMT, P4HA3, PPAPDC1A, PPP1R3C, S1PR1 et SYNPO2
où des niveaux d'expression accrus desdits un ou plusieurs gènes par rapport à une valeur de référence pour lesdits un ou plusieurs gènes est indicatif d'une probabilité accrue d'issue négative, que le patient est candidat pour recevoir un traitement après traitement chirurgical ou que le patient est susceptible de bénéficier d'un traitement après traitement chirurgical ou
où des niveaux d'expression réduits des un ou plusieurs gènes par rapport à une valeur de référence pour les un ou plusieurs gènes est indicatif d'une probabilité accrue d'issue positive, que le patient n'est pas un candidat pour recevoir un traitement après traitement chirurgical ou que le patient est peu susceptible de bénéficier d'un traitement après traitement chirurgical.

5. Procédé selon la revendication 4 comprenant en outre la détermination des niveaux d'expression d'un ou plusieurs gènes choisis dans le groupe constitué par ADAMTS6, BMP6, CCDC71L, CH25H, CNNM2, CPNE2, CREB3L2, DCBLD1, EFR3B, EGR1, ELN, ENDOD1, FHOD3, FOXC1, FZD8, GBP1, GXYLT2, IGF1, IL4R, ITGA11, JPH2, KIAA1211, KIF26B, LIMK1, LINC00340, LPCAT2, LRRC8A, METTL7A, OLFM2, PID1, PPP1R12B, PRSS23, RASD2, RNF152, SCUBE3, SEC14L2, SERPINE2, SGK1, SH3PXD2A, SHISA2, SMTN, SRGAP1, TRPC6 et UCK2 et les gènes qui s'hybrident spécifiquement avec les sondes ayant les séquences SEQ ID NO:1 à 26
où des niveaux d'expression accrus desdits un ou plusieurs gènes par rapport à des valeurs de référence pour lesdits un ou plusieurs gènes est indicatif d'une probabilité accrue d'une issue négative, que le patient est candidat pour recevoir un traitement après traitement chirurgical ou que le patient est susceptible de bénéficier d'un traitement après traitement chirurgical ou
où des niveaux d'expression réduits d'un ou de plusieurs desdits gènes par rapport à des valeurs de référence pour les un ou plusieurs desdits gènes est indicatif d'une probabilité accrue d'une issue positive, que le patient n'est pas un candidat pour recevoir un traitement après traitement chirurgical ou que le patient est peu susceptible de bénéficier d'un traitement après traitement chirurgical.

6. Procédé selon l'une quelconque des revendications 1 à 5 où le stade de la tumeur chez le patient est en outre déterminé et
où un stade de tumeur élevé est indicatif d'une probabilité accrue d'une issue négative, que le patient est candidat pour recevoir un traitement après traitement chirurgical ou que le patient est susceptible de bénéficier d'un traitement après traitement chirurgical ou
où un stade de tumeur faible est indicatif d'une probabilité accrue d'une issue positive, que le patient n'est pas un candidat pour recevoir un traitement après traitement chirurgical ou que le patient est peu susceptible de bénéficier d'un traitement après traitement chirurgical.

7. Procédé selon l'une quelconque des revendications 1 à 6 où le traitement est choisi dans le groupe constitué par une chimiothérapie, une radiothérapie et/ou un traitement comprenant un inhibiteur TGF-bêta.

8. Procédé selon la revendication 7, où l'inhibiteur de TGF-bêta est un composé tel que défini dans le Tableau 1 ou où l'inhibiteur de TGF-bêta est un composé ayant la structure 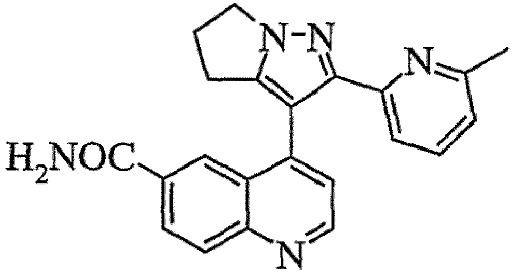 ou tout polymorphe, solvate ou hydrate de celui-ci.

9. Procédé selon l'une quelconque des revendications 1 à 8, où l'issue à prédire est soit une récurrence, soit un développement de métastases, de préférence des métastases hépatiques.

10. Procédé selon l'une quelconque des revendications 1 à 9, où l'échantillon est choisi dans le groupe constitué par une biopsie tumorale ou un fluide biologique.

11. Composé chimiothérapeutique et/ou inhibiteur de TGF-bêta, destiné à être utilisé dans le traitement de cancer colorectal chez un patient après traitement chirurgical du cancer, où le patient a été choisi par un procédé selon l'une quelconque des revendications 1 à 10.

12. Composé chimiothérapeutique et/ou inhibiteur de TGF-bêta, destiné à être utilisé selon la revendication 11, où l'inhibiteur de TGF-bêta est un composé tel que défini dans le Tableau 1 ou où l'inhibiteur de TGF-bêta est un composé ayant la structure 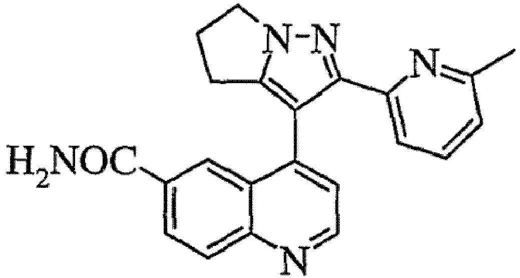 ou tout polymorphe, solvate ou hydrate de celui-ci.

13. Kit comprenant des réactifs adéquats pour déterminer les niveaux d'expression des gènes FAM46A, FHL2, FOXC2 et COL4A1 et, facultativement, des réactifs pour la détermination des niveaux d'expression d'un ou de plusieurs gènes domestiques, où les réactifs adéquats pour la détermination des niveaux d'expression des gènes FAM46A, FHL2, FOXC2 et COL4A1 représentent au moins 10 % de la quantité totale de réactifs adéquats pour la détermination des niveaux d'expression des gènes formant le kit, où les réactifs sont choisis dans le groupe constitué par des amorces, des sondes, des aptamères et des anticorps.

14. Kit selon la revendication 13, comprenant en outre des réactifs adéquats pour la détermination des niveaux d'expression du gène FRMD6 ou des réactifs adéquats pour la détermination des niveaux d'expression des gènes SPRY4 et DACT3, où les réactifs sont choisis dans le groupe constitué par des amorces, des sondes, des aptamères et des anticorps.

15. Kit selon l'une quelconque des revendications 13 ou 14, comprenant en outre des réactifs adéquats pour la détermination des niveaux d'expression d'un ou de plusieurs gènes choisis dans le groupe constitué par ACTC1, BOC, CNN1, COMP, CRISPLD2, CRYAB, FAM101B, FILIP1L, GAS7, GFPT2, GLIS3, HS3ST3A1, KIRREL, LRRC15, LTBP2, MFAP2, NNMT, P4HA3, PPAPDC1A, PPP1R3C, S1PR1 et SYNPO2, où les réactifs sont choisis dans le groupe constitué par des amorces, des sondes, des aptamères et des anticorps.

16. Kit selon l'une quelconque des revendications 13 à 15, comprenant en outre des réactifs adéquats pour la détermination des niveaux d'expression d'un ou de plusieurs gènes choisis dans le groupe constitué par ADAMTS6, BMP6, CCDC71L, CH25H, CNNM2, CPNE2, CREB3L2, DCBLD1, EFR3B, EGR1, ELN, ENDOD1, FHOD3, FOXC1, FZD8, GBP1, GXYLT2, IGF1, IL4R, ITGA11, JPH2, KIAA1211, KIF26B, LIMK1, LINC00340, LPCAT2, LRRC8A, METTL7A, OLFM2, PID1, PPP1R12B, PRSS23, RASD2, RNF152, SCUBE3, SEC14L2, SERPINE2, SGK1, SH3PXD2A, SHISA2, SMTN, SRGAP1, TRPC6 et UCK2 et les gènes qui s'hybrident spécifiquement avec les sondes ayant les séquences SEQ ID NO:1 à 26, où les réactifs sont choisis dans le groupe constitué par des amorces, des sondes, des aptamères et des anticorps.

17. Utilisation d'un kit selon l'une quelconque des revendications 13 à 16, pour prédire l'issue d'un patient souffrant de cancer colorectal, permettant de choisir un traitement approprié pour un patient souffrant de cancer colorectal ou permettant de choisir un patient qui est susceptible de bénéficier d'un traitement adjuvant après résection chirurgicale de cancer colorectal.


### SEQUENCE LISTING

<110> FUNDACIÓ PRIVADA INSTITUCIÓ CATALANA DE RECERCA I ESTUDIS AVANÇATS FUNDACIÓ PRIVADA INSTITUT DE RECERCA BIOMÈDICA
<120> METHODS AND KITS FOR THE PROGNOSIS OF COLORECTAL CANCER
<130> P8897PC00
<150> EP12192291
   <151> 2012-11-12
<150> EP12382530
   <151> 2012-12-24
<160> 26
<170> PatentIn version 3.5
<210> 1
   <211> 521
   <212> DNA
   <213> Artificial Sequence
<220>
   <223> Affymetrix 211620_x_at probe
<400> 1
   gagggaaaag cttcactctg accatcactg tcttcacaaa cccaccgcaa gtcgccacct      60
   accacagagc catcaaaatc acagtggatg ggccccgaga acctcgaaga catcggcaga     120
   aactagatga tcagaccaag cccgggagct tgtccttttc cgagcggctc agtgaactgg     180
   agcagctgcg gcgcacagcc atgagggtca gcccacacca cccagccccc acgcccaacc     240
   ctcgtgcctc cctgaaccac tccactgcct ttaaccctca gcctcagagt cagatgcagg     300
   aatcatggat gctgccaatt ttgagcagtt tttgcaagaa aggatcaaag tgaacggaaa     360
   agctgggaac cttggtggag gggtggtgac catcgaaagg agcaagagca agatcaccgt      420
   gacatccgag gtgcctttct ccaaaaggta tttgaaatat ctcaccaaaa aatatttgaa     480
   gaagaataat ctacgtgact ggttgcgcgt agttgctaac a                         521
<210> 2
   <211> 499
   <212> DNA
   <213> Artificial Sequence
<220>
   <223> Affymetrix 220990_s_at probe
<400> 2
   agcagtttag tccatacttt gcactgacat actttttcct tctgtgctaa ggtaaggtat      60
   ccaccctcga tgcaatccac cttgtgtttt cttagggtgg aatgtgatgt tcagcagcaa     120
   acttgcaaca gactggcctt ctgtttgtta ctttcaaaag gcccacatga tacaattaga     180
   gaattcccac cgcacaaaaa aagttcctaa gtatgttaaa tatgtcaagc tttttaggct     240
   tgtcacaaat gattgctttg ttttcctaag tcatcaaaat gtatataaat tatctagatt     300
   ggataacagt cttgcatgtt tatcatgtta caatttaata ttccatcctg cccaaccctt     360
   cctctcccat cctcaaaaaa gggccatttt atgatgcatt gcacaccctc tggggaaatt     420
   gatctttaaa ttttgagaca gtataaggaa aatctggttg gtgtcttaca agtgagctga     480
   caccattttt tattctgtg                                                  499
<210> 3
   <211> 120
   <212> DNA
   <213> Artificial Sequence
<220>
   <223> Affymetrix 222288_at probe
<220>
   <221> misc_feature
   <222> (43)..(43)
   <223> n is a, c, g, or t
<400> 3
   cctttccgtt tctgtctatg atgtaggctt ctgaggagaa ccnagaagct tggctttagt      60
   ggtagaatga cagaacttag ggatcccttg caggctagaa caaagttctg acccttagac     120
<210> 4
   <211> 407
   <212> DNA
   <213> Artificial Sequence
<220>
   <223> Affymetrix 224917_at probe
<220>
   <221> misc_feature
   <222> (200)..(200)
   <223> n is a, c, g, or t
<220>
   <221> misc_feature
   <222> (323)..(323)
   <223> n is a, c, g, or t
<400> 4
   tttcatctga ccatccatat ccaatgttct catttaaaca ttacccagca tcattgttta        60
   taatcagaaa ctctggtcct tctgtctggt ggcacttaga gtcttttgtg ccataatgca       120
   gcagtatgga gggaggattt tatggagaaa tggggatagt cttcatgacc acaaataaat       180
   aaaggaaaac taagctgcan tgtgggtttt gaaaaggtta ttatacttct taacaattct       240
   ttttttcagg gacttttcta gctgtatgac tgttacttga ccttctttga aaagcattcc       300
   caaaatgctc tattttagat agnttaacat taaccaacat aatttttttt agatcgagtc       360
   agcataaatt tctaagtcag cctctagtcg tggttcatct ctttcac                     407
<210> 5
   <211> 489
   <212> DNA
   <213> Artificial Sequence
<220>
   <223> Affymetrix 226885_at probe
<220>
   <221> misc_feature
   <222> (65)..(65)
   <223> n is a, c, g, or t
<400> 5
   gaatcttcct gccaacaggt aggtcctgct ggacaggacc tgaaaattaa gagatttttt        60
   ttttnaagca gtgaaattta aaaatctatt acctcaagaa ttttcagtcg atatgtcaat       120
   gtactgttcc ttctcagtaa gtacagtgtc acatgctgct ggcggtaaaa gcaacccaag       180
   tgtcagatca cacaaatttt agggacaagt ctttcaaaag acggacacct tgtgatattt       240
   tggagtctaa tttccgactt ttcgtaatgt cccaatgacc ttttgttgtg gttgggcttt       300
   tataaaaaat tattttcggt tggttgttgt agaatggaag agtaatctta attatgaaat       360
   cagattgcat ggaagaatat aaagacactc actcggatga aaactataac ggttttcaaa       420
   ctattttgat ggtcattgta cgagctattg tatggattac tgtggagtgc tgtttaccac       480
   atgatgtgt                                                               489
<210> 6
   <211> 344
   <212> DNA
   <213> Artificial Sequence
<220>
   <223> Affymetrix 229024_at probe
<400> 6
   ggtctgaatt tgtgttggca tccatggtac ttactgttca gtaggatgtt attgcaagga      60
   gcagagtgcc ctctgctgga gtaatcgcaa ttattcttgc agcagattaa tttgacttgg     120
   gtcatgaatt caacaaccag ttacttgcct ttcatcatac aatttcttcg gtagttgaga     180
   atttggtcta catttatcaa atgaggaaag agtgtcacaa actctaaaaa gctgaaggag     240
   accccacaga tcttctcact gtcagcagcc cttacttctg caaaatgttg aaggataatg     300
   ttgctctgtt tgcaaagaag atgcctctgg ctagaatgtt tgtg                      344
<210> 7
   <211> 583
   <212> DNA
   <213> Artificial Sequence
<220>
   <223> Affymetrix 229130_at probe
<220>
   <221> misc_feature
   <222> (28)..(28)
   <223> n is a, c, g, or t
<220>
   <221> misc_feature
   <222> (31)..(31)
   <223> n is a, c, g, or t
<220>
   <221> misc_feature
   <222> (68)..(68)
   <223> n is a, c, g, or t
<220>
   <221> misc_feature
   <222> (71)..(71)
   <223> n is a, c, g, or t
<220>
   <221> misc_feature
   <222> (87)..(87)
   <223> n is a, c, g, or t
<220>
   <221> misc_feature
   <222> (101)..(101)
   <223> n is a, c, g, or t
<220>
   <221> misc_feature
   <222> (398)..(398)
   <223> n is a, c, g, or t
<400> 7
   tgttctgctg tgataactgc ccattctngt nctctatcta agaaggcatt tagttttgaa      60
   aatatttngt nggcttgtaa gatattngta catattaagc ncagcaaacc tgtaacatat     120
   tcactgtaaa tcattattcc tcatgcatca tttgccattt aactgtattt ttttcctttt     180
   tgatgtaacc tgctatagtt tacattttta aatcagaaat gcctgcacat tttaaaacga     240
   agactcttat gctctagcct ttgaggtcat gcttggaaag tcctttctca cacaaagatt     300
   atttacactt tcatctcttt ttctctgagt acgtttcagt gtttaattga ccaggaatgt     360
   atttcgaggt gtgatatgag acaaggatct gaaatgcntc gcccactgaa tcatcatgca     420
   ctgaccgaaa tgccacctgt atgagtgcta aaatcattgt cgctggacct gtgtctgcac     480
   tgtttaattt tagtgttgtt tctcacatca gcttctgttg ttttcatggt tgtggttctc     540
   atacttttaa tatctgttgg agcaaataaa gcctcgtttc tca                       583
<210> 8
   <211> 548
   <212> DNA
   <213> Artificial Sequence
<220>
   <223> Affymetrix 229296_at probe
<220>
   <221> misc_feature
   <222> (118)..(118)
   <223> n is a, c, g, or t
<220>
   <221> misc_feature
   <222> (224)..(224)
   <223> n is a, c, g, or t
<220>
   <221> misc_feature
   <222> (256)..(256)
   <223> n is a, c, g, or t
<400> 8
   aagtgtgtac ctacgtgtca ggcccatcct tcctgcaggc ctttgtcttg gtgtcaccct 60
   gcctgagctg gccccaacca cagtagcaac agtgatgctg atgtgtgact gctgccangg 120
   agcctgctca catgaccgtg tggagaaagt tctttccctg aggaccatct ggagtggacg 180
   cgtgcactac ccctctctga atacaccctc cccacgaggc cctntggagc atcctgtaga 240
   gccagcccct acccanagac cctgagccca gagaatgcgc tgggtggtga gcctggggac 300
   gaatgcagag ccctaggtct cctctccaga aagaccggat tttgccatgt tggccaggct 360
   ggtcttgaac tcctgacctc aagtgatcca cctgcctcgg cctcccaaag tgcagggatt 420
   acaggtgtga gccactgcac ccggcctcag gcaggtattt ttaccagccg aatactcttg 480
   cactgtgcac tcttcgtgtt catcatcagt cccccggatg ggtgtttaag gcatttccaa 540
   agctctgt 548
<210> 9
   <211> 413
   <212> DNA
   <213> Artificial Sequence
<220>
   <223> Affymetrix 231559_at probe
<400> 9
   ctgagtcaca tcacacttca gcacctgctt gaccgcctgt ctcaacttct cctccttctc 60
   tggacccttg actctgttcc cttcaagatc acacacatag gtcaccactg gggaccagtc 120
   aaaggcctct ggctctttct tcagccactt ctccagctcc tgcaggttct ggtctgagta 180
   gtcagtgacg acgatctcct taaaggattc acaagcagag aggagctgat agatagtggg 240
   gccagagccg atgttcagaa ggtgcttaag aatctggctt tctgcagagt gcctagaacc 300
   aaacttgtaa tatttttcta ggtaatcccg agggttaaaa tggcttagat aggtgtcctt 360
   ggaggtgaag cctgattcca ttatgtctca cttctgtacc actggagcac tgc 413
<210> 10
   <211> 442
   <212> DNA
   <213> Artificial Sequence
<220>
   <223> Affymetrix 235629_at probe
<400> 10
   tgacttttag atattccaca gaaccagact tatttgatgt ggataataac caatgattta 60
   gcattttgtt tgcttttgtt ttattttatc cgggttcatt ttttactctt cccatgtaca 120
   tgaaacaggt ggtggcgtgt agagatcagc tgatccttgt tttatggtta attgaactac 180
   tttgtatcca gggtttctgc aaatccaaaa gtgatttttc atctaggatc tattcctaac 240
   agtctactcc aatcccactt tagttttcca caattttaaa tcttaatagt gagaattcaa 300
   atgaaagtca tttcatttga ctattctgat gacatgattg tggcagaata aattgggtct 360
   taaaatgccc tagaaaatgg taaatgataa aaaataatat tttaaaattc aaccaaagaa 420
   atggcccatt ggccaggtgt gg 442
<210> 11
   <211> 308
   <212> DNA
   <213> Artificial Sequence
<220>
   <223> Affymetrix 237978_at probe
<220>
   <221> misc_feature
   <222> (29)..(29)
   <223> n is a, c, g, or t
<220>
   <221> misc_feature
   <222> (251)..(251)
   <223> n is a, c, g, or t
<400> 11
   cttttcacga catcctaaaa tgagaattnc gatggaacta agaaagtgta taaaagaact 60
   tcatgaattt ctgtacacac acctaaacat aaagtgtttc aatatgtttc acagtaagag 120
   aataatacag gccagattat acaatacttc tgtgcacacc agatggttgt aaagaaagat 180
   cagcgagaaa ggctttggaa gttttaatcc ttctttccac ctttttcccc ccgcatttag 240
   taaatcacaa ncctacctga ctggcatcca attatcagag atattcagtg tttaagctac 300
   cctcttta 308
<210> 12
   <211> 443
   <212> DNA
   <213> Artificial Sequence
<220>
   <223> Affymetrix 238617_at probe
<220>
   <221> misc_feature
   <222> (30)..(30)
   <223> n is a, c, g, or t
<220>
   <221> misc_feature
   <222> (182)..(182)
   <223> n is a, c, g, or t
<220>
   <221> misc_feature
   <222> (203)..(203)
   <223> n is a, c, g, or t
<400> 12
   cccaggtgca tttattgtgc cagaaacagn gaattcctaa ataagttcat aaaatcttgt      60
   tttacaattc aatggtagtg cttaatgtta ctgtctgaaa tccactgttt aaccagatgg     120
   aagactgggg ccattcaaaa cattattagt tcacaagtgt ttgctgttaa aatgcttcaa     180
   tnaaactcat ttgttaaagt canaaattca actcttctgt acttgggcag gtcccagcag     240
   ccccttggca ggctagtttt gtgcatgggc ctttgcaagc tatataaacg ttaggtgttg     300
   gcttgattat gataaatatg agcaggctcc ctggagaaca gtccagaaaa aaagagttat     360
   agttctcaca aactggtgac atgaatccat taagctgtgt ggcattttcc agtgtaaaga     420
   cattgtttgc tggctacgat ctc                                             443
<210> 13
   <211> 528
   <212> DNA
   <213> Artificial Sequence
<220>
   <223> Affymetrix 240135_x_at probe
<220>
   <221> misc_feature
   <222> (116)..(116)
   <223> n is a, c, g, or t
<220>
   <221> misc_feature
   <222> (159)..(159)
   <223> n is a, c, g, or t
<220>
   <221> misc_feature
   <222> (163)..(163)
   <223> n is a, c, g, or t
<220>
   <221> misc_feature
   <222> (168)..(168)
   <223> n is a, c, g, or t
<220>
   <221> misc_feature
   <222> (205)..(205)
   <223> n is a, c, g, or t
<220>
   <221> misc_feature
   <222> (226)..(226)
   <223> n is a, c, g, or t
<220>
   <221> misc_feature
   <222> (264)..(264)
   <223> n is a, c, g, or t
<220>
   <221> misc_feature
   <222> (341)..(341)
   <223> n is a, c, g, or t
<220>
   <221> misc_feature
   <222> (344)..(344)
   <223> n is a, c, g, or t
<220>
   <221> misc_feature
   <222> (355)..(355)
   <223> n is a, c, g, or t
<220>
   <221> misc_feature
   <222> (395)..(395)
   <223> n is a, c, g, or t
<220>
   <221> misc_feature
   <222> (434)..(434)
   <223> n is a, c, g, or t
<400> 13
   agatcctttt gggcagcctg aagactgggg agggttttcc tccctcctcc acctccaccc 60
   acattccact taccctttcc ctcctcatcc cagttggtaa cattcccacc tgattnagtg 120
   tggggagttg ctgctgcagc tgagttctgg ttttgcccna gcnaggantc acacacgtgc 180
   ggccaggagg ctggcaggtg gaatncaggg tgtgcagggg tgcagnccca agtccttttc 240
   tgctggaaaa tgggctgggt gacnttcact tgcgtttttt tggtatgtag tgggagtagg 300
   tccccattct tcccccttgc cctttgcttt ggggtttcta nccntaaaaa ttganccacg 360
   gaaaagtgaa aacaagcatg agtagatcct gcttnccaat gggggccttc ccgtctgctt 420
   actgcttagg tttnctgcgg tgggtctgag caatgcagaa agtttcttac aatttctctg 480
   gattgaaaca agaaatagct ccctcgctcc tcttcataaa tttctcag 528
<210> 14
   <211> 427
   <212> DNA
   <213> Artificial Sequence
<220>
   <223> Affymetrix 241272_at probe
<220>
   <221> misc_feature
   <222> (204)..(205)
   <223> n is a, c, g, or t
<220>
   <221> misc_feature
   <222> (216)..(216)
   <223> n is a, c, g, or t
<220>
   <221> misc_feature
   <222> (224)..(224)
   <223> n is a, c, g, or t
<220>
   <221> misc_feature
   <222> (227)..(228)
   <223> n is a, c, g, or t
<220>
   <221> misc_feature
   <222> (260)..(260)
   <223> n is a, c, g, or t
<220>
   <221> misc_feature
   <222> (264)..(264)
   <223> n is a, c, g, or t
<220>
   <221> misc_feature
   <222> (272)..(272)
   <223> n is a, c, g, or t
<220>
   <221> misc_feature
   <222> (275)..(276)
   <223> n is a, c, g, or t
<220>
   <221> misc_feature
   <222> (281)..(281)
   <223> n is a, c, g, or t
<220>
   <221> misc_feature
   <222> (288)..(288)
   <223> n is a, c, g, or t
<220>
   <221> misc_feature
   <222> (298)..(298)
   <223> n is a, c, g, or t
<220>
   <221> misc_feature
   <222> (327)..(327)
   <223> n is a, c, g, or t
<220>
   <221> misc_feature
   <222> (401)..(401)
   <223> n is a, c, g, or t
<400> 14
   taactgggca ccagctaagc cactgtgctg cctaaatgga caaccacttc tcagttctgg     60
   aattcttcaa acacctaggg ttaggagggt cttcgctttg gcagttagag ttttctcaat    120
   tcttttatga cacaggttgc accggactca ctcaactatg gagatcagat gtcctcagga    180
   aaaaaaaaaa aaaaaaaaaa aaanntacac acttanggct gttnccnngg ctcctctctc    240
   ttcctgacac attccaaaan ggantttatg gntgnnggga nggacaangg gtgaggantt    300
   tctaggacat ttaagaacgc ccatgtnagc aactctatgc ttgacaggcc atgcacaata    360
   aaactctttg gcctgtaagc gagaagaaaa atttggtgta ngctgcctca tgggttgcca    420
   aaatgat                                                              427
<210> 15
   <211> 402
   <212> DNA
   <213> Artificial Sequence
<220>
   <223> Affymetrix 244788_at probe
<220>
   <221> misc_feature
   <222> (302)..(302)
   <223> n is a, c, g, or t
<400> 15
   ctggactgct ggccggtagc aaaagaccag ctggaaattt cataaaaggt tagacaatcc 60
   acagttactc agtgttacgc aacaggtctc tgcagcacag agcacgaact ggggagtcag 120
   gatgctggag tccagccctg cctctctgcg gattccctgc ctgaccttgg gaaagcctct 180
   tggggtctcg gggcataggg caggactgag acgttgagac cccaagtgca aagaagacac 240
   atttctccta ccacgagtaa tcaaaaacac tatgaaactg taatatgggc cgggtgcggt 300
   gnctcacgcc tgtaatcaca ctttcggagg ccgaggagat tgagcccagg agttgaagac 360
   cagcctgggc aacataggca gatcccatct cattttgtta at 402
<210> 16
   <211> 388
   <212> DNA
   <213> Artificial Sequence
<220>
   <223> Affymetrix 238623_at probe
<220>
   <221> misc_feature
   <222> (172)..(172)
   <223> n is a, c, g, or t
<400> 16
   gcctgatagt gctgaatttc tgaattgacc ttcatcttat ttactcaata atattcattt 60
   gacaaatact tattaagtgc atatatgtgt caggaactgt actagatgct gaggatatag 120
   cagtaagcgc aacagaccaa gctaacagct tagtaaaggg tgaggtaaaa cnaaaacaaa 180
   aaagtattca aaaaaataaa ctaattttta tcttaattta aaaaattaca aatttttaaa 240
   aatcacaaat tggtatccat gtataattca tttccgtgca ttttcttgtg tgaagaaagc 300
   tcagtaaaag tatttcttag gtttctgtaa ttctagttct ctactcgatt ttcttctgca 360
   attttctgag ccagaaccct tcttagaa 388
<210> 17
   <211> 499
   <212> DNA
   <213> Artificial Sequence
<220>
   <223> Affymetrix 1557241_a_at probe
<220>
   <221> misc_feature
   <222> (459)..(460)
   <223> n is a, c, g, or t
<220>
   <221> misc_feature
   <222> (462)..(463)
   <223> n is a, c, g, or t
<400> 17
   tagcccgacc cctgtatata ttgtacctct ctctctgccc acctctcccc cacttctccc      60
   cactgcccca ttctctctta tcctgcctat tcctctcccc tatttctctg tctctctctc     120
   tctcacatac acacacactc actcacaagc agtacagtca ctaagttggc taggtaatat     180
   ccaaagcatt ctgattgtcc tactttttgt aaattgaact tatttaagat attttagaag     240
   agatgagtca cgtcttcctt tgacccttca cccataacac acataccatt ctcacaggag     300
   gccacatggt gcagtagagt cagactttga ttcaaagccc aactctgtca cgtgacagta     360
   ggcttgagtt tcctcatctg taaaaaggga agatttccta cttcataaag tggttggaga     420
   aattaaagaa atagtatatg aagccttgca cataaaaann anntgatata tattaatgcc     480
   cttttgtaca agaggtcaa                                                  499
<210> 18
   <211> 539
   <212> DNA
   <213> Artificial Sequence
<220>
   <223> Affymetrix 227107_at probe
<400> 18
   ccctgcccac cagaggagaa agcccttgta gaattctgca gtgttacaag tgttccctac      60
   aaaaactgaa accatcagct cctctttaac aagttggctt tttaaaagca cgtaattaca     120
   atttaatggt attctgtaaa gtggtgctct aggcataatt taaattcttt ttaatgacta     180
   tatttcttca aaactttgaa agaaaaatgt gttctttttg ctgcatcctt tgtaagaaga     240
   ctgccaacag aggaaaaagg actttacaaa ttaagaccat cttggtttca tttccacaaa     300
   gatgagaaca aatcatggtg ttaggaaagg atccttagaa gaacacaaga atttgaaagc     360
   ccttggtggt tatcactact atatttcata tttccacaga agtgacttag ccaagctctg     420
   cattttgagc ctgctgactt tcatttaaaa ggaatgaaag gctgaaaatc caggctgctg     480
   tgtctgtaga taaaggtcaa accatgtttg agttcttcac tgttgtgtcc acctaaata      539
<210> 19
   <211> 419
   <212> DNA
   <213> Artificial Sequence
<220>
   <223> Affymetrix 228629_s_at probe
<400> 19
   agggtcttgc catgtggtga ttttttctat gtgattgagg aagtacctct ggccagtggc    60
   cgtgaaggtc atctcccagc ccgggggcag tggcagctcg tcggtcacgt cgtaggactg  120
   ctggcggagg tgcgcgtgct gctgcgcggg gctacccgca gcacccgcgc cggtgcccag  180
   ctgcagggac gcgggcgacg agtgcgagcg gacatgctgg gcacccccag ccagtcgagg  240
   ccccgggtgg ccgcccgacg agtcggtgct ggactggcgc gagtgcgagc ccgaatcagg  300
   ctccttaaag aaagactccg gcaggatctt cttccgccac gagctaggct tcggattcat  360
   gacagagttg aagagggctt cgaggtctgt gtctaggtcc tgcgtgacgt ggatcactt   419
<210> 20
   <211> 503
   <212> DNA
   <213> Artificial Sequence
<220>
   <223> Affymetrix 228756_at probe
<400> 20
   gccgcacctt ctctgaaatg ctggccagga gcttggtggc ctcaagcagc gagcagtgtt   60
   ccatgctggt gccatcgatg gacaggatgt ggtctccagg gtgcagggct ccgctcctgt  120
   ccaccacgct ggctggcttg atgcggtcga tggtaatgac tgacttgttc cggagggagg  180
   tggtggtgag cgagatcccc agggcagacc ctggcgtctt gactatttcc accatcaagg  240
   gtcccgaagc attagccacc gtgtcagggg tggccacatc atactccacc tgaaagagtg  300
   cctcgtggct gcactgccgc agggtggcca gggcggtggc atggctggcc ccgtgcagcg  360
   ggattccatc gacgctgagc agcctgtcgc ccaccttcag ggagccctcc ctgtcggcag  420
   ggccaccggg ccgcacgtag gtcaggacaa gcgggcggga cttgtgccca tcttcatggg  480
   cacctcctct aaggacaaag cca                                          503
<210> 21
   <211> 461
   <212> DNA
   <213> Artificial Sequence
<220>
   <223> Affymetrix 233223_at probe
<220>
   <221> misc_feature
   <222> (128)..(128)
   <223> n is a, c, g, or t
<400> 21
   gcgaacacta gtttcagggg ggtcaggctc ccgatctgcg tcattatcct gtttaaatag      60
   ggaggggcag gccctttcct gctgtctgtc tcccatagtc tctggctatg aggcttgagt     120
   gacttggnac atgacatttc tctttctggt tcattcattt tccccacttc catctgaaaa     180
   ttctaaccaa atgtcttgct gaattctcta agtacaacaa tttcaccctc cctgcagaca     240
   atgaggaaat cagagtccag aatccatttc aacttagcaa acatttactg agtctatatt     300
   ttgtttcgga tactggggta aagttctgga aatgcaaaag gaatttgcat ctctgaactt     360
   aaggagttca cagtcttgaa cagggcaatc caagttgtac tgtaaagaaa tagtatgatc     420
   gagaactggg aaaggaaaca gcctttgaat agatacccat t                         461
<210> 22
   <211> 288
   <212> DNA
   <213> Artificial Sequence
<220>
   <223> Affymetrix 233335_at probe
<400> 22
   tttgtgtcac tgtctattct attagtttca acatcatgct aaaataggac agcatgttcc      60
   aaaactgaaa ctgaaacagg aacggagcct tgctgattta gcatgaggag ggaattgtaa     120
   gccttgcacc tggacactaa aacttttcaa taagatgtat gctaagccgt acctagcaaa     180
   cttaccaggt ccaaagtaga gtacaagcag aaagttattt ttaaaggatg ggcactatcc     240
   ttgagggact taccctttct aatcagcttt gcatggtttg ttttgaga                  288
<210> 23
   <211> 372
   <212> DNA
   <213> Artificial Sequence
<220>
   <223> Affymetrix 236251_at probe
<400> 23
   gaattaatcc ctttctatga gaacaactaa aaagggaaca actacaaaag agtacaacta 60
   ttgagcctac tatggttgga aaatacttga aagtatcatt taaagatggt tactttaatt 120
   agatctccgt actaaacgtg ggaggatagc tttatatttt actttttgaa gagcattaag 180
   ttctggtctg tccatttcct gaaattctga gctatgttat tcatctctgt tgttggctct 240
   ataatgtatg tatctgtttg ctgctattca taataacaaa ggtcttagcg aagacctagc 300
   aatcttaggg taataggaag acagaggaat ttcatattgg aatttttaac ttgcagatca 360
   caacttgtta gt 372
<210> 24
   <211> 522
   <212> DNA
   <213> Artificial Sequence
<220>
   <223> Affymetrix 238933_at probe
<400> 24
   gctcaggagc tctacttgtt ggaagcctgt ctgctttacc tgtagtccag tgaccttttc 60
   tgcctgccct ttctcttgca tagcctctta gttctggctt gctcgttttc tagtactatc 120
   ccctgccatc cctttgtttc cacctcttat ttcatgttcc tgacattatc ctttttttct 180
   tttgctcctt ttcagaattt cgcagtactt gtggcccact ctctgcagag agtaaattga 240
   gagcccaaga agcagggcaa aaagagcctc tcagactcgg tcatagcagc cctgccacct 300
   ttacctggca tcaaatttat cttttagttt tgaaactgca gctgacaaag acacagaccc 360
   cagccatgcc ttttgggttc agtttttttg tttttgtcat tctttgttat gccctccccc 420
   tttaaaagaa aaaagaaaaa gaaaaaaaac agaaaaagac tctttccatt tctttttcat 480
   tcagtttcct tttttgtgat gtcaaggatt ccgtacctcc aa 522
<210> 25
   <211> 326
   <212> DNA
   <213> Artificial Sequence
<220>
   <223> Affymetrix 239809_at probe
<400> 25
   gaaatcaagt ccaagtatct caagtaacag tacaacaatc catatcagga aaaacagctt 60
   agggcttgac ttcattatgg ttttggctgc cattttatgc attctgagta gaagaaacat 120
   tcctcctagc gcccccacct tgtctttaac attccgcagt ggaagttcct caaagctctc 180
   cgttttgaaa gcatcctcta tgattaccag ccactaaatt gcatctgcca ccgagggccc 240
   aatgggcttt gaacctgtta ttcttgggaa attcaagctg cgagggagag agagaacgtt 300
   gtacacgtgt gcagctgtgc tttgga 326
<210> 26
   <211> 279
   <212> DNA
   <213> Artificial Sequence
<220>
   <223> Affymetrix 240960_at probe
<400> 26
   tcctgactcc tcttaaggtg cggctttgcc tgaacagttg ggtggatctc tgggaataga 60
   cattgctcca cggtctttct tctgtagtgg catatctcat cctcagttct gagatagccc 120
   actttgaaac acagcttcat gggcctggaa gttgcttcaa acttgcatta accgtctcat 180
   ctgagtcaaa gtggcaaaga agacaagaca gcgtgtttat tgttttcctg ggaatggaga 240
   gagaatggag tataagctgt ttgtagtcag gcctggagt 279